# EUROPÄISCHE PATENTSCHRIFT

(11) **EP 2 242 740 B1**
(45) Veröffentlichungstag und Bekanntmachung des Hinweises auf die Patenterteilung: **12.12.2012**
(21) Anmeldenummer: 09709089.8
(22) Anmeldetag: 23.01.2009
(51) Int. Cl.: C07D 209/44, C07D 403/10, A61K 31/4035, A61P 7/02

(54) **SF5-DERIVATE ALS PAR1-INHIBITOREN, IHRE HERSTELLUNG UND VERWENDUNG ALS ARZNEIMITTEL**
SF5 DERIVATIVES AS PAR1 INHIBITORS, PRODUCTION THEREOF, AND USE AS MEDICAMENTS
DÉRIVÉS DE SF5 EN TANT QU'INHIBITEURS DE PAR1, LEUR PRODUCTION ET LEUR UTILISATION EN TANT QUE MÉDICAMENTS

(30) Priorität: 05.02.2008 EP 08290115
(43) Veröffentlichungstag der Anmeldung: 27.10.2010
(73) Patentinhaber: SANOFI, 75008 Paris (FR)
(72) Erfinder: HEINELT, Uwe, 65926 Frankfurt am main (DE); WEHNER, Volkmar, 65926 Frankfurt am Main (DE); HERMANN, Matthias, 65926 Frankfurt am main (DE); SCHOENAFINGER, Karl, 63755 Alzenau (DE); STEINHAGEN, Henning, 52099 Aachen (DE)
(74) Vertreter: Then, Johann
(86) Internationale Anmeldenummer: PCT/EP2009/000408
(87) Internationale Veröffentlichungsnummer: WO 2009/097972

(56) Entgegenhaltungen:
- EP-A- 1 391 451
- US-A1- 2003 216 476
- US-A1- 2004 242 659
- US-A1- 2005 197 370
- WELCH, JOHN T.: LIM, DONG SUNG: "The synthesis and biological activity of pentafluorosulfanyl analogs of fluoxetine, fenfluramine, and norfenfluramine" BIOORGANIC & MEDICINAL CHEMISTRY, Bd. 15, 2007, Seiten 6659-6666, XP002478937

## Beschreibung

Die Erfindung betrifft neue Verbindungen der Formel I 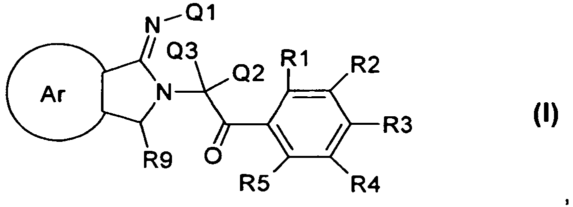 wobei R1, R2, R3, R4, R5, R9, Ar, Q1, Q2 und Q3 die unten bezeichnete Bedeutung haben. Die Verbindungen der Formel I haben antithrombotische Aktivität und inhibieren insbesondere den Protease-aktivierten Rezeptor 1 (PAR1). Die Erfindung betrifft ferner ein Verfahren zur Herstellung der Verbindung der Formel I und deren Verwendung als Arzneimittel.

Der Protease-aktivierte Rezeptor 1 (PAR1) ist ein Thrombin Rezeptor, der zur Klasse der G Protein-gekoppelten Rezeptoren (GPCR) gehört. Das Gen für PAR1 liegt auf Chromosom 5q13, besteht aus zwei Exonen und deckt eine Region von etwa 27 kb ab.
PAR1 wird unter anderem in Endothelzellen, glatten Muskelzellen, Fibroblasten, Neuronen und humanen Blutplättchen exprimiert. Auf Blutplättchen ist PAR1 ein wichtiger Rezeptor der Signalübertragung welcher an der Initiation der Aggregation von Blutplättchen beteiligt ist.
Die Aktivierung der PARs erfolgt über die proteolytische Abspaltung eines Teils des N-Terminus der PARs, wodurch eine neue N-terminale Sequenz freigelegt wird, die dann den Rezeptor aktiviert (Pharmacol Rev 54:203-217, 2002).

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der vaskulären Konstriktion: Hierdurch wird der Blutverlust in das geschädigte Areal vermindert.
2. Die nächste Phase ist die der Plättchenadhäsion an das freigelegte Kollagen im Subendothel. Diese primäre Adhäsion an die Matrix aktiviert die Plättchen, die daraufhin verschiedene Aktivatoren sekretieren, die zur Verstärkung der Aktivierung führen. Diese Aktivatoren stimulieren zudem die weitere Rekrutierung neuer Plättchen zum Ort der Gefäßschädigung und fördern die Plättchenaggregation. Die Plättchen aggregieren an der Stelle des Gefäßwandschadens und bilden ein noch lockeres Plättchengerinnsel. Weiterhin führt die Aktivierung der Plättchen zur Präsentation von Phosphatidylserin und Phosphatidylinositol entlang der Zellmembranoberflächen. Die Exposition dieser Phospholipide ist zur Bindung und Aktiverung von Multienzymkomplexen der Blutgerinnungskaskade essentiell.
3. Das anfänglich noch lockere Plättchenaggregat wird durch Fibrin vernetzt. Wenn der Thrombus lediglich Plättchen und Fibrin enthält, handelt es sich um einen weißen Thrombus. Sind zusätzlich rote Blutkörperchen vorhanden, handelt es sich um einen roten Thrombus.
4. Nach Wundheilung wird der Thrombus durch die Einwirkung des Proteins Plasmin aufgelöst.

Zwei alternative Wege führen zur Bildung eines Fibringerinnsels, der intrinsische und der extrinsische Weg. Diese Wege werden durch unterschiedliche Mechanismen eingeleitet, in späterer Phase konvergieren sie jedoch zu einer gemeinsamen Wegstrecke der Gerinnungskaskade. Die Bildung eines roten Thrombus oder eines Gerinnsels auf dem Boden einer Gefäßwandabnormität ohne Wunde ist das Resultat des intrinsischen Weges. Die Fibringerinnselbildung als Antwort auf einen Gewebsschaden oder eine Verletzung ist das Resultat des extrinsischen Weges. Beide Wege beinhalten eine größere Anzahl von Proteinen, die als Gerinnungsfaktoren bekannt sind.

Der intrinsische Weg erfordert die Gerinnungsfaktoren VIII, IX, X, XI und XII sowie Präkallekrein, hochmolekulares Kininogen, Calciumionen und Phospholipide aus Plättchen. Jedes dieser Proteine führt zur Aktivierung des Faktors X.

Der intrinsische Weg wird eingeleitet, wenn Präkallekrein, hochmolekulares Kininogen Faktor XI und XII an eine negativ geladene Oberfläche binden. Dieser Moment wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallekrein in Kallekrein, das wiederum den Faktor XII aktiviert. Faktor XIIa hydrolysiert weiteres Präkallekrein zu Kallekrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwsenheit von Ca²⁺-Ionen aktiviert der Faktor XIa den Faktor IX. Faktor IX ist ein Proenzym, das Vitamin-K abhängige, c-Karboxiglutamat (GLA)-Reste enthält. Die Serinproteaseaktivität kommt nach Bindung von Ca²⁺-Ionen an diese GLA-Reste zum Tragen. Mehrere der Serinproteasen der Blutgerinnungskaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Proteasekomplexes aus von Ca²⁺-Ionen und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Proteasekomplexes möglich. Faktor VIII hat in diesem Vorgang die Funktion eines Rezeptors für die Faktoren IXa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor VIIIa schließlich durch Thrombin weiter gespalten und inaktiviert. Diese duale Aktivität des Thrombins in Bezug zum Faktor VIII führt zu einer Selbstbegrenzung der Proteasekomplexbildung und damit zu einer Eingrenzung der Blutgerinnung.

Bei der Aktivierung von humanen Blutplättchen durch Thrombin spielen PAR1 und PAR4 eine zentrale Rolle; die Aktivierung dieser Rezeptoren führt in Blutplättchen zu morphologischen Veränderungen, Freisetzung von ADP und Aggregation der Blutplättchen (Nature 413:26-27, 2001).

Inhibitoren von PAR 1 werden beispielsweise in den Europäischen Patentanmeldungen EP1391451 oder EP1391452, den amerikanischen Patentanmeldungen US 6,063,847 und US 2004/0152736 sowie der Internationalen Anmeldung WO 03/089428 beschrieben.

Die Verbindungen der Formel I zeichnen sich im Vergleich mit Verbindungen aus WO2006 051623 oder EP1391451 durch eine erhöhte metabolische Stabilität aus, wie sie beispielsweise durch in-vitro Testungen in Leber-Mikrosomen gezeigt werden kann. Die metabolische Stabilität lässt sich beispielsweise durch Inkubation der Verbindung der Formel I in Leber-Mikrosomen in Anwesenheit oder Abwesenheit von NADPH ermitteln.

Die Verbindungen der Formel I eignen sich daher für die prophylaktische als auch für die therapeutische Anwendung am Menschen, die an Erkrankungen leiden, die mit Thrombosen, Embolien, Hyperkoagulabilität oder fibrotischen Veränderungen einhergehen. Beispiele solcher Erkrankungen sind Thrombose, tiefe Venenthrombose, Lungenembolien, Gehirninfarkt, Herzinfarkt, Bluthochdruck, Entzündliche Erkrankungen, Rheuma, Asthma, Glomerulonephritis oder Osteoporose. Die Verbindungen der Formel I können zur Sekundär-Prävention eingesetzt werden und eignen sich sowohl für eine akute als auch für eine Langzeittherapie.
Die Verbindungen der Formel I sind auch in Kombination mit Wirkstoffen einsetzbar, die über andere antithrombotische Prinzipien wie PAR 1 wirken.
1) Die Erfindung betrifft daher eine Verbindung der Formel I 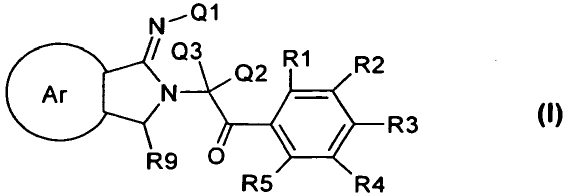 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
   - Ar: für einen annelierten Benzol-, Pyridin-, Pyrimidin-, Pyridazin- oder Pyrazin-Ring steht, wobei der annilierte Ring unsubstituiert oder ein-, zwei-, drei- oder vierfach unabhängig voneinander durch -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R1)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R1)-C(O)-R12, Halogen, OH, -CN, -NO₂, -SO₂CH₃, -SO₂CF₃, -SF₅, -Si[-(C₁-C₄)-Alkyl]₃, -(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₁-C₆)-Alkylen-O-(C₁-C₆)-Alkyl, substituiert ist,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl substituiert ist, wobei in Alkyl, Alkylen oder Cycloalkyl die Wasserstoffatome ganz oder teilweise durch Fluor ersetzt sein können,
   - Q1: für Wasserstoffatom, -(C₁-C₆)-Alkyl, -(C₃-C₆)-Cycloalkyl, -OH, -O-(C₁-C₆)-Alkyl oder -O-(C₃-C₆)-Cycloalkyl steht, wobei Alkyl und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
   - Q2 und Q3: gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
   - R11 und R12: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen, wobei in Alkyl, Alkylen oder Cycloalkyl die Wasserstoffatome ganz oder teilweise durch Fluor ersetzt sein können, oder
   - R11 und R12: in den Fragmenten "N(R11)-R12" und "N(R11)-C(O)-R12" für einen 5-bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder-O-(C₃-C₆)-Cycloalkyl, substituiert ist, wobei in Alkyl oder Cycloalkyl die Wasserstoffatome ganz oder teilweise durch Fluor ersetzt sein können,
   - R1, R2, R3, R4 und R5: gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₆)-Alkyl, -O-(C₃-C₆)-Cycloalkyl, -(C₀-C₄)-Alkylen-(CO)-N(R21)-R22, - SO₂CH₃, -SO₂CF₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -O-(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -Si[-(C₁-C₄)-Alkyl]₃ oder -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, stehen,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-Cg)-Alkyl oder -O-(C₃-C₆)-Cycloalk substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₃-C₆)-Cycloalkyl, substituiert ist,
   wobei in Alkyl, Alkylen oder Cycloalkyl die Wasserstoffatome ganz oder teilweise durch Fluor ersetzt sein können,
   mit der Maßgabe, das mindestens ein R1, R2, R3, R4 oder R5 für -SF₅ steht,
   - R9: für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
   -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, steht, und wobei in Alkyl, Alkylen oder Cycloalkyl die Wasserstoffatome ganz oder teilweise durch Fluor ersetzt sein können,
   - R21 und R22: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen, wobei in Alkyl, Alkylen oder Cycloalkyl die Wasserstoffatome ganz oder teilweise durch Fluor ersetzt sein können, oder
   - R21 und R22: in den Fragmenten "N(R21)-R22" und "N(R21)-C(O)-R22" für einen 5-bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können.
2) Bevorzugt ist eine Verbindung der Formel I, wobei
   - Ar: für einen annelierten Benzol- oder Pyridin-Ring steht, wobei der annilierte Ring unsubstituiert oder ein-, zwei-, drei- oder vierfach unabhängig voneinander durch -(C₁-C₆)-Alkyl, -(C₃-C₆)-Cycloalkyl, OH, -CN, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, SO₂CH_{3,} -(C₀-C₄)-Alkylen-N(R11)-R12, -(C₀-C₄)-Alkylen-N(R11)-C(O)-R12, Halogen, -SO₂CF₃ -Si[-(C₁-C₄)-Alkyl]₃, -(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₄-C₁₅)-Het, wobei Het unsubstituiert oder ein-, zwei- oder dreifach unabhängig voneinander durch - (C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₁-C₆)-Alkylen-O-(C₁-C₆)-Alkyl substituiert ist,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
   -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
   -O-(C₃-C₆)-Cycloalkyl substituiert ist,
   wobei in Alkyl, Alkylen oder Cycloalkyl die Wasserstoffatome ganz oder teilweise durch Fluor ersetzt sein können,
   - Q1, Q2 und Q3: gleich oder verschieden sind und unabhängig voneinander für Wasserstoffatom, -(C₁-C₆)-A)ky) oder -(C₃-C₆)-Cycloalkyl stehen, wobei in Alkyl oder Cycloalkyl die Wasserstoffatome ganz oder teilweise durch Fluor ersetzt sein können,
   - R11 und R12: unabhängig voneinander für Wasserstoffatom, -(C₁-C₆)-Alkyl, -(C₃-C₆)-Cycloalkyl, -SO₂CH₃ oder -SO₂CF₃ stehen, wobei in Alkyl, Alkylen oder Cycloalkyl die Wasserstoffatome ganz oder teilweise durch Fluor ersetzt sein können, oder
   - R11 und R12: in den Fragmenten "N(R11)-R12" und "N(R11)-C(O)-R12" für einen 5-bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
   - R1, R2, R3, R4 und R5: gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₆)-Alkyl, -O-(C₃-C₆)-Cycloalkyl, -(C₀-C₄)-Alkylen-(CO)-N(R21)-R22, - SO₂CH₃, -SO₂CF₃, -(C₀-C4)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -O-(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -Si[-(C₁-C₄)-Alkyl]₃ oder -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, stehen,
   wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl, substituiert ist, wobei in Alkyl, Alkylen oder Cycloalkyl die Wasserstoffatome ganz oder teilweise durch Fluor ersetzt sein können,
   mit der Maßgabe, das mindestens ein R1, R2, R3, R4 oder R5 für -SF₅ steht,
   - R9: für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist,
   -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, steht, und wobei in Alkyl, Alkylen oder Cycloalkyl die Wasserstoffatome ganz oder teilweise durch Fluor ersetzt sein können,
   - R21 und R22: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen, wobei in Alkyl, Alkylen oder Cycloalkyl die Wasserstoffatome ganz oder teilweise durch Fluor ersetzt sein können, oder
   - R21 und R22: in den Fragmenten "N(R21)-R22" und "N(R21)-C(O)-R22" für einen 5-bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können.
3) Besonders bevorzugt ist eine Verbindung der Formel I, wobei
   - Ar: für einen annelierten Benzol- oder Pyridin-Ring steht, wobei der annilierte Ring unsubstituiert oder ein-, zwei-, drei- oder vierfach unabhängig voneinander durch -(C₁-C₆)-Alkyl, -(C₃-C₆)-Cycloalkyl, OH, -CN, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, SO₂CH₃, -(C₀-C₄)-Alkylen-N(R11)-R12, -(C₀-C₄)-Alkylen-N(R11)-C(O)-R12, Halogen, -Si[-(C₁-C₄)-Alkyl]₃, -(C₁-C₆)-Alkylen-O-(C₁-C₆)-Alkyl oder -O-(C₁-C₆)-Alkylen-O-(C₁-C₆)-Alkyl substituiert ist, wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, -(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl substituiert ist, wobei in Alkyl, Alkylen oder Cycloalkyl die Wasserstoffatome ganz oder teilweise durch Fluor ersetzt sein können,
   - Q1, Q2 und Q3: gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
   - R11 und R12: unabhängig voneinander für Wasserstoffatom, -(C₁-C₆)-Alkyl, -(C₃-C₆)-Cycloalkyl oder -SO₂CH₃ stehen, wobei in Alkyl, Alkylen oder Cycloalkyl die Wasserstoffatome ganz oder teilweise durch Fluor ersetzt sein können, oder
   - R11 und R12: in den Fragmenten "N(R11)-R12" und "N(R11)-C(O)-R12" für einen 5-bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
   - R1, R2, R3, R4 und R5: gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₆)-Alkyl, -O-(C₃-C₆)-Cycloalkyl, -(C₀-C₄)-Alkylen-(CO)-N(R21)-R22, - SO₂CH₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -O-(C₁-C₆)-Alkylen-O-(C₁-C₆)-Alkyl, -Si[-(C₁-C₄)-Alkyl]₃ oder -(C₄-C₁₅)₋Het stehen,
   wobei Alkyl, Alkylen und Cycloalkyl jeweils unsubstituiert oder ein-, zwei- oder dreifach unabhängig voneinander durch durch -(C₁-C₄)-Alkyl, OH, -(C₃-C₆)-Cycloalkyl, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
   mit der Maßgabe, das ein R1, R2, R3, R4 oder R5 für -SF₅ steht,
   - R9: für Wasserstoffatom, -(C₁-C₆)-Alkyl oder -(C₃-C₆)-Cycloalkyl steht, wobei in Alkyl oder Cycloalkyl die Wasserstoffatome ganz oder teilweise durch Fluor ersetzt sein können,
   - R21 und R22: unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen, wobei in Alkyl, Alkylen oder Cycloalkyl die Wasserstoffatome ganz oder teilweise durch Fluor ersetzt sein können, oder
   - R21 und R22: in den Fragmenten "N(R21)-R22" und "N(R21)-C(O)-R22" für einen 5-bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können.
4) Die Erfindung betrifft ferner eine Verbindung der Formel I, wobei
   - Ar: für einen annelierten Benzol-Ring steht,
   wobei der annilierte Ring unsubstituiert oder ein- oder zweifach unabhängig voneinander durch -O-(C₁-C₆)-Alkyl, -C(O)-N(R11)-R12 oder Halogen substituiert ist,
   - Q1, Q2 und Q3: gleich sind und für jeweils ein Wasserstoffatom stehen,
   - R11 und R12: unabhängig voneinander für Wasserstoffatom oder -(C₁-C₆)-Alkyl stehen,
   - R1, R2, R3, R4 und R5: gleich oder verschieden sind und unabhängig voneinander für Wasserstoffatom, -(C₁-C₆)-Alkyl, -O-(C₁-C₆)-Alkyl, Halogen, -SF₅, -(C₀-C₄)-Alkylen-N(R21)-R22, -O-(C₁-C₆)-Alkylen-O-(C₁-C₆)-Alkyl, oder -(C₀-C₄)-Alkylen-N(R21)-C(O)-R22 stehen,
   mit der Maßgabe, das ein R1, R2, R3, R4 oder R5 für -SF₅ steht,
   - R9: für Wasserstoffatom steht,
   - R21 und R22: unabhängig voneinander für Wasserstoffatom, -(C₁-C₆)-Alkyl, -(C₃-C₆)-Cycloalkyl, CF₃ oder -SO₂CH₃ stehen,
   wobei in Alkyl, Alkylen oder Cycloalkyl die Wasserstoffatome ganz oder teilweise durch Fluor ersetzt sein können, oder
   - R21 und R22: in dem Fragment "N(R21)-R22" für einen Ring steht, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl.
5) Außerordentlich bevorzugt sind Verbindungen der Formel I, wobei folgende Verbindungen umfasst sind,
2-(1-Imino-1,3-dihydro-isoindol-2-yl)-1-(3-pentafluorsulfanyl-phenyl)-ethanon als Hydrobromid, 2-[2-(3-Dimethylamino-5- pentafluorsulfanyl-phenyl)-2-oxo-ethyl]-6-ethoxy-3-imino-2,3-dihydro-1H-isoindol-5-carbonsäuremethylamid,
6-Ethoxy-3-imino-2-[2-(3-methylamino-5-pentafluorsulfanyl-phenyl)-2-oxo-ethyl]-2,3-dihydro-1H-isoindol-5-carbonsäuremethylamid,
6-Ethoxy-3-imino-2-{2-[3-pentafluorsulfanyl-5-(2,2,2-trifluor-acetylamino)-phenyl]-2-oxo-ethyl}-2,3-dihydro-1H-isoindol-5-carbonsäuremethylamid,
6-Ethoxy-2-(2-{3-[ethyl-(2,2,2-trifluor-acetyl)-amino]-5-pentafluorsulfanyl-phenyl}-2-oxo-ethyl)-3-imino-2,3-dihydro-1H-isoindol-5-carbonsäuremethylamid,6-Ethoxy-2-[2-(3-ethylamino-5-pentafluorsulfanyl-phenyl)-2-oxo-ethyl]-3-imino-2,3-dihydro-1H-isoindol-5-carbonsäuremethylamid als Hydrochlorid,
2-[2-(3-Amino-5-pentafluorsulfanyl-phenyl)-2-oxo-ethyl]-6-ethoxy-3-imino-2,3-dihydro-1H-isoindol-5-carbonsäuremethylamid,2-[2-(3-Acetylamino-5-pentafluorsulfanylphenyl)-2-oxo-ethyl]-6-ethoxy-3-imino-2,3-dihydro-1H-isoindol-5-carbonsäuremethylamid,
2-(5,6-Diethoxy-7-fluor-1-imino-1,3-dihydro-isoindol-2-yl)-1-[3-methylamino-5-(pentamethyl-sulfanyl)-phenyl]-ethanon,
6-Ethoxy-3-imino-2-{2-[3-dimethansulfonylamino-5-(pentafluorsulfanyl)-phenyl]-2-oxo-ethyl}-2,3-dihydro-1H-isoindol-5-carbonsäuremethylamid,
2-{2-[3-(Acetyl-methyl-amino)-5-(pentafluorsulfanyl)-phenyl]-2-oxo-ethyl}-6-ethoxy-3-imino-2,3-dihydro-1H-isoindol-5-carbonsäuremethylamid,
N-[3-[2-(5,6-Diethoxy-7-fluor-1-imino-1,3-dihydro-isoindol-2-yl)-acetyl]-5-(pentafluorsulfanyl)-phenyl]-N-methyl-acetamid,
N-[3-[2-(5,6-Diethoxy-7-fluor-1-imino-1,3-dihydro-isoindol-2-yl)-acetyl]-5-(pentafluorsulfanyl)-phenyl]-isobutyramid,
N-[3-[2-(5,6-Diethoxy-7-fluor-1-imino-1,3-dihydro-isoindol-2-yl)-acetyl]-5-pentafluorsulfanyl)-phenyl]-acetamid als Hydrobromid,
N-[5-[2-(5,6-Diethoxy-7-fluor-1-imino-1,3-dihydro-isoindol-2-yl)-acetyl]-4-methyl-2-(pentafluor-sulfanyl)-phenyl]-acetamid,
1-[5-Amino-2-methyl-4-(pentafluorsulfanyl)-phenyl]-2-(5,6-diethoxy-7-fluor-1-imino-1,3-dihydro-isoindol-2-yl)-ethanon,
N-{3-[2-(5,6-Diethoxy-7-fluor-1-imino-1,3-dihydro-isoindol-2-yl)-acetyl]-5-pentafluorsulfanyl-phenyl}-3,3-dimethyl-butyramid,
1-{3-[2-(5,6-Diethoxy-7-fluor-1-imino-1,3-dihydro-isoindol-2-yl)-acetyl]-5-pentafluorsulfanyl-phenyl}-pyrrolidin-2,5-dion,
1-[2-Amino-4-(pentafluorsulfanyl)-phenyl]-2-(5,6-diethoxy-7-fluor-1-imino-1,3-dihydro-isoindol-2-yl)-ethanon,
1-[2-Amino-5-brom-4-(pentafluorsulfanyl)-phenyl]-2-(5,6-diethoxy-7-fluor-1-imino-1,3-dihydro-isoindol-2-yl)-ethanon,
2-(5,6-Diethoxy-7-fluor-1-imino-1,3-dihydro-isoindol-2-yl)-1-[3-methoxy-5-(pentafluorsulfanyl)-phenyl]-ethanon,
Cyclobutancarbonsäure [3-[2-(5,6-diethoxy-7-fluor-1-imino-1,3-dihydro-isoindol-2-yl)-acetyl]-5-(pentafluorsulfanyl)-phenyl]-amid als Hydrochlorid, Cyclopropancarbonsäure-[3-[2-(5,6-diethoxy-7-fluor-1-imino-1,3-dihydro-isoindol-2-yl)-acetyl]-5-(pentafluorsulfanyl)-phenyl]-amid als Hydrochlorid,
2-(5,6-Diethoxy-7-fluor-1-imino-1,3-dihydro-isoindol-2-yl)-1-[3-ethoxy-5-(pentafluorsulfanyl)-phenyl]-ethanon,
6-Ethoxy-3-imino-2-{2-[3-methoxy-5-(pentafluorsulfanyl)-phenyl]-2-oxo-ethyl}-2,3-dihydro-1H-isoindol-5-carbonsäure-methylamid, 1-[3-Brom-5-(pentafluor-sulfanyl)-phenyl]-2-(5,6-diethoxy-7-fluor-1-imino-1,3-dihydro-isoindol-2-yl)-ethanon,
1-[3-Chlor-5-(pentafluorsulfanyl)-phenyl]-2-(5,6-diethoxy-7-fluor-1-imino-1,3-dihydro-isoindol-2-yl)-ethanon,
1-[2-Brom-4-(pentafluorsulfanyl)-phenyl]-2-(5,6-diethoxy-7-fluor-1-imino-1,3-dihydro-isoindol-2-yl)-ethanon,
1-[2-Chlor-4-(pentafluorsulfanyl)-phenyl]-2-(5,6-diethoxy-7-fluor-1-imino-1,3-dihydro-isoindol-2-yl)-ethanon,
2-(5,6-Diethoxy-7-fluor-1-imino-1,3-dihydro-isoindol-2-yl)-1-[3-morpholin-4-yl-5-(pentafluorsulfanyl)-phenyl]-ethanon oder
2-(5,6-Diethoxy-7-fluor-1-imino-1, 3-dihydro-isoindol-2-yl)-1-[3-(2-methoxy-ethoxy)-5-(pentafluorsulfanyl)-phenyl]-ethanon als Hydrochlorid.

Unter dem Begriff "(C₁-C₄)-Alkyl" oder "(C₁-C₆)-Alkyl" werden Kohlenwasserstoffreste verstanden, deren Kohlenstoffkette geradkettig oder verzweigt ist und 1 bis 4 oder 1 bis 6 Kohlenstoffatome enthält, beispielsweise Methyl, Ethyl, Propyl, Isopropyl, Butyl, Isobutyl, sec-Butyl, tertiär-Butyl, Pentyl, Isopentyl, Neopentyl, 1-Ethylpropyl, Hexyl, 2,3-Dimethylbutyl oder Neohexyl.
Unter dem Begriff "(C₀-C₄)-Alkylen" werden Kohlenwasserstoffreste verstanden, deren Kohlenstoffkette geradkettig oder verzweigt ist und 1 bis 4 Kohlenstoffatome enthält, beispielsweise Methylen, Ethylen, 1-Methylmethylen, Propylen, 1-Methylethylen, Butylen, 1-Propylmethylen, 1-Ethyl-1-methylmethylen, 1,2-Dimethylethylen, 1,1-Dimethylmethylen, 1-Ethylethylen, 1-Methylpropylen, 2-Methylpropylen. "-C₀-Alkylen" ist eine kovalente Bindung.
Unter dem Begriff "-O-(C₁-C₆)-Alkyl" werden Alkoxyreste verstanden, deren Kohlenstoffkette geradkettig oder verzweigt ist und 1 bis 6 Kohlenstoffatome enthält, beispielsweise Methoxy, Ethoxy, Propoxy, Iso-Propoxy, Butoxy, Iso-Butoxy, tertiär-Butoxy, 1-Pentoxy, 2-Pentoxy, 3-Pentoxy, 1-Hexoxy, 2-Hexoxy oder 3-Hexoxy. Unter dem Begriff "(C₃-C₆)-Cycloalkyl" werden Reste verstanden wie Verbindungen, die sich von 3- bis 6-gliedrige Monocyclen wie Cyclopropan, Cyclobutan, Cyclopentan oder Cyclohexan herleiten.

Unter dem Begriff "-O-(C₃-C₆)-Cycloalkyl" werden Cycloalkoxy-Reste verstanden wie Verbindungen, die sich von 3- bis 6-gliedrige Monocyclen wie Cyclopropoxy, Cyclobutoxy, Cyclopentoxy oder Cyclohexoxy herleiten.
Unter dem Begriff "-(C₆-C₁₄)-Aryl" werden aromatische Kohlenstoffreste verstanden mit 6 bis 14 Ring-Kohlenstoffatomen verstanden. -(C₆-C₁₄)-Arylreste sind beispielsweise Phenyl, 1-Naphthyl, 2-Naphthyl, Anthryl oder Fluorenyl. Naphthylreste und insbesondere Phenylreste sind bevorzugte Arylreste.
Unter dem Begriff "Ar für einen annelierten Benzol-Ring steht" werden bizyklische Ringsysteme verstanden, die zusammen mit dem Dihydropyrrolring in Formel I einen 2,3-Dihydro-1H-isoindol-ring bilden, der die folgende Struktur aufweist: 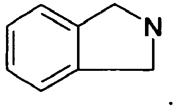 Für den Fall das "Ar für einen annelierten Pyridin-Ring steht" werden bizyklische Ringsysteme verstanden, die zusammen mit dem Dihydropyrrolring in Formel I beispielsweise einen 2,3-Dihydro-1H-pyrrolo[3,4-c]pyridin-ring bilden, der die folgende Struktur aufweist: 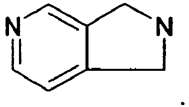 Für den Fall das "Ar für einen annelierten Pyrimidin-Ring steht" werden bizyklische Ringsysteme verstanden, die zusammen mit dem Dihydropyrrolring in Formel I beispielsweise einen 6,7-Dihydro-5H-pyrrolo[3,4-d]pyrimidin-ring bilden, der die folgende Struktur aufweist: 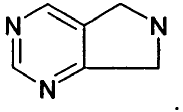 Für den Fall das "Ar für einen annelierten Pyridazin-Ring steht" werden bizyklische Ringsysteme verstanden, die zusammen mit dem Dihydropyrrolring in Formel I beispielsweise einen 6,7-Dihydro-5H-pyrrolo[3,4-d]pyridazin-ring bilden, der die folgende Struktur aufweist: 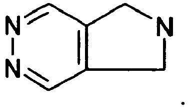 Für den Fall das "Ar für einen annelierten Pyrazin-Ring steht" werden bizyklische Ringsysteme verstanden, die zusammen mit dem Dihydropyrrolring in Formel I beispielsweise einen 6,7-Dihydro-5H-pyrrolo[3,4-b]pyrazin-ring bilden, der die folgende Struktur aufweist:

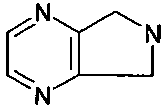

Unter dem Begriff "-(C₄-C₁₅)-Het" werden Ringsysteme mit 4 bis 15 Kohlenstoffatomen verstanden, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrol, Thienopyridin, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl oder Xanthenyl.

Unter den Begriffen "R11 und R12 in den Fragmenten "N(R11)-R12" und "N(R11)-C(O)-R12" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten" oder "R11 und R12 in den Fragmenten "N(R21)-R22" und "N(R21)-C(O)-R22" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten" werden beispielsweise Ringsysteme wie cyclische Amine verstanden wie Azetidinyl, Pyrrolidinyl, Piperidinyl, Piperazinyl, Azepinyl, Morpholinyl oder Thiomorpholinyl, bei den Imiden Reste wie Pyrrolidin-2,5-dionyl, Piperidin-2,6-dionyl, Piperazin-2,6-dionyl, Morpholin-3,5-dionyl, und bei den Lactamen Reste wie Pyrrolidin-2-onyl, Piperidin-2-onyl, Piperazin-2-onyl, Morpholin-3-onyl. Unter der Umschreibung "Alkyl, Alkylen oder Cycloalkyl die Wasserstoffatome ganz oder teilweise durch Fluor ersetzt sein" wird ein partiell oder vollständig fluorierter Alkyl-, Alkylen- oder Cycloalkylrest verstanden, der sich beispielsweise für Alkyl von folgenden Resten -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂, -CF₂-CF₂-CH₂F, -CH(CF₃)₂, -CH(CHF₂)₂, -CH(CFH₂)₂, -CH(CFH₂)(CHF₂), -CH(CFH₂)(CF₃), -CH(CFH₂)(CH₃), -CH(CHF₂)(CH₃), -CH(CF₃)(CH₃), -CF(CF₃)₂, -CF(CHF₂)₂, -CF(CFH₂)₂, -CF(CFH₂)(CHF₂), -CF(CFH₂)(CF₃), -CF(CFH₂)(CH₃), -CF(CHF₂)(CH₃), -CF(CF₃)(CH₃), sowie die weiteren möglichen Kombinationen für Butyl, Pentyl und Hexyl, die wie Propyl auch verzweigt sein können, für Alkylen beispielsweise von folgenden Resten -CF₂-, -CHF-, -CHF-CF₂-, -CHF-CHF-, -CHF-CH₂-, -CF₂-CF₂-, -CF₂-CH₂F, sowie die weiteren möglichen Kombinationen für Propylen, Butylen, Pentylen und Hexylen, die auch verzweigt sein können,
und für Cycloalkyl beispielsweise von den Resten 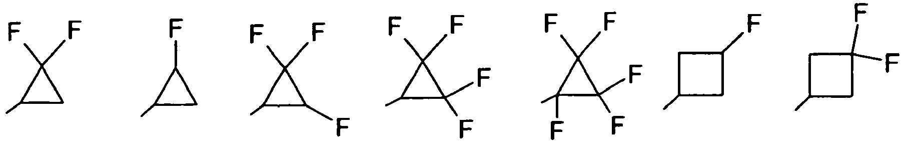 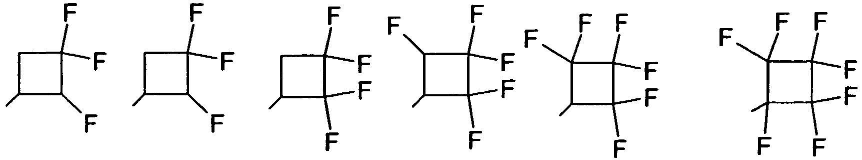 sowie den analogen größeren Ringen Cyclopentyl und Cyclohexyl ableitet.
Unter dem Begriff "Halogen" wird Fluor, Chlor, Brom oder Jod verstanden, bevorzugt sind Fluor, Chlor oder Brom, insbesondere Fluor oder Chlor.
Oben beschriebene Begriffe sind auch beliebig kombinierbar wie beispielsweise in "-(C₁-C₆)-Alkylen-O-(C₁-C₆)-Alkyl" geschehen.

Funktionelle Gruppen der verwendeten Intermediate, beispielsweise Amino- oder Carboxylgruppen in der Verbindung der Formel I, können dabei durch geeignete Schutzgruppen maskiert werden. Geeignete Schutzgruppen für Aminofunktionen sind beispielweise die t-Butoxycarbonyl-, die Benzyloxycarbonyl- oder die Phtalolylgruppe sowie die Trityl- oder Tosylschutzgruppe. Geeignete Schutzgruppen für die Carboxylfunktion sind beispielweise Alkyl-, Aryl- oder Arylalkylester. Schutzgruppen können durch wohlbekannte oder hier beschriebene Techniken eingeführt und entfernt werden (siehe Greene, T.W., Wuts, P.G.M., Protective Groups in Organic Synthesis (1999), 3rd Ed., Wiley-Interscience, oder Kocienski, P. J., Protecting Groups (2004), 3rd Ed., Thieme). Der Begriff Schutzgruppe kann auch entsprechende polymergebundene Schutzgruppen umfassen.

Die erfindungsgemäßen Verbindungen können nach wohlbekannten Verfahren oder nach hier beschriebenen Verfahren hergestellt werden.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel II, 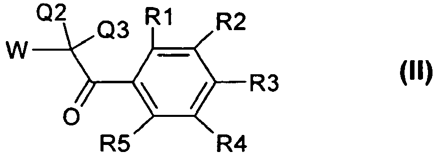 wobei R1, R2, R3, R4, R5, Q2 und Q3 wie in Formel I definiert sind und W für Chlorid, Bromid, Mesylat oder Tosylat steht, mit einer Verbindung der Formel III, 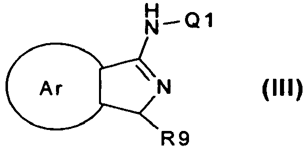 wobei Ar, R9 und Q1 wie in Formel I definiert sind, mit oder ohne Basenzu-gabe in einem Lösungsmittel zu einer Verbindung der Formel I umsetzt, oder
b) eine Verbindung der Formel VII, 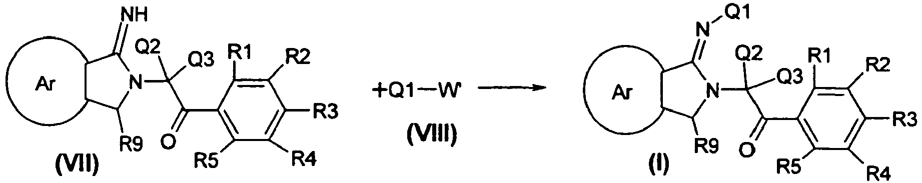 wobei R1, R2, R3, R4, R5, R9, Ar, Q1, Q2 und Q3 wie in Formel I definiert sind, mit der Verbindung Q1-W', wobei W' Chlorid, Bromid, Mesylat, Tosylat, Methylsulfat oder eine ähnliche gute Fluchtgruppe bedeutet, mit oder ohne Basenzugabe zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

Die Erfindung betrifft ferner ein Verfahren zur Herstellung der Verbindung der Formel I nach Schema 1, wobei ein R1, R2, R3, R4 oder R5 für SF₅ steht.

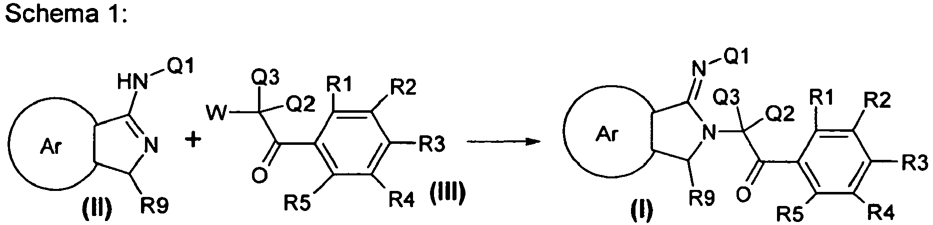

Die Edukte II und III, wobei II gegebenenfalls (ggf.) in Form eines Salzes vorliegt, werden dabei bei RT oder leicht erhöhter Temperatur von 40 °C bis 60 °C vorteilhafterweise, wenn II als Salz vorliegt, in Gegenwart einer Base, bevorzugt Hünig-Base, in einem Lösungsmittel, bevorzugt Dimethylformamid (DMF) oder Dioxan, zu der Verbindung der Formel I umgesetzt. Die Reste R1, R2, R3, R4, R5, Ar und Q sind wie in Formel I definiert, W entspricht einer guten Fluchtgruppe wie Chlorid, Bromid, Mesylat oder Tosylat, bevorzugt Bromid oder Mesylat.

Die Verbindungen der Formel I können teilweise auch in isomeren Formen auftreten, wobei Q1 in der folgenden Teilfomel von Formel I entweder (E) oder (Z) konfiguriert sein kann:

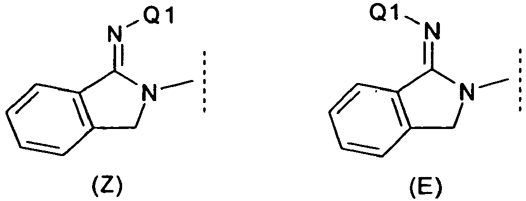

Verbindungen der Formel II sind teilweise käuflich oder lassen sich nach literaturbekannten Verfahren gewinnen (W02006 018954/5, WO2006 018954, EP 1 391 451). Ein Zugang zu den Pentafluorsulfanyl-Derivaten der Formel III - Verbindung der Foremel III, worin ein R1, R2, R3, R4 oder R5 für SF₅ steht - ist nachfolgend wiedergegeben.

Allgemein lassen sich Acetylbromide (W=Br) der Pentafluorsulfanyl-Derivate vom Typ IIIa, worin ein R1, R2, R3, R4 oder R5 für SF₅ steht, wie in Schema 2) beschrieben darstellen.

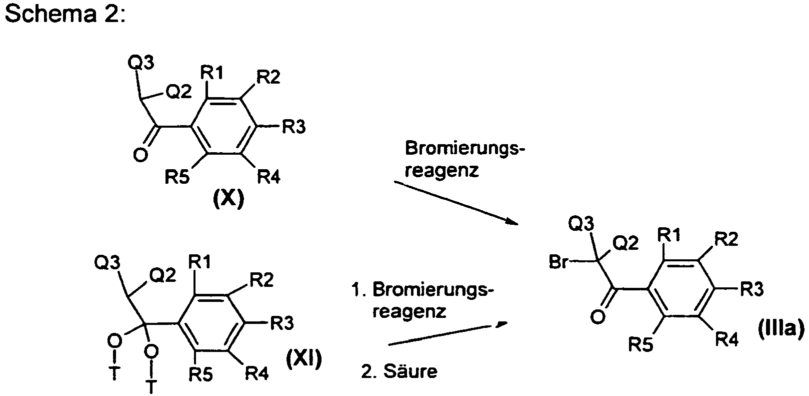

Dabei können die Acetophenon-Derivate der Formel X, worin ein R1, R2, R3, R4 oder R5 für SF₅ steht, entweder direkt beispielsweise mit Br₂, N-Brom-succinimid (NBS) oder Phenyl-trimethyl-tribromid, bevorzugt in Eissessig, Methanol oder Methanol/THF-Gemischen, zu Verbindungen der Formel IIIa bromiert werden oder aber es werden die entsprechenden Ketale XI, worin ein R1, R2, R3, R4 oder R5 für SF₅ steht, der Acetophenon-Derivate X mit beispielsweise obigen Bromierungs-reagenzien, vorzugsweise Phenyl-trimethyl-tribromid, bromiert. Anschließend werden zur Gewinnung der Verbindungen der Formel IIIa die Ketale in Gegenwart von Säure, beispielsweise Salzsäure, Bromwasserstoffsäure, Schwefelsäure, Trifluoressigsäure, bevorzugt Schwefelsäure, gespalten.

Die Ketale der Formel XI und XI' lassen sich ausgehend von den Ketonen der Formel X durch dem Fachmann bekannte Ketalisierungsreaktionen gewinnen.
Bevorzugt wird die Umsetzung zu den Verbindungen der Formel XI in Methanol mit Methyl-ortho-formiat in Gegenwart von DL-10-Camphersulfonsäure (Schema 3) durchgeführt.

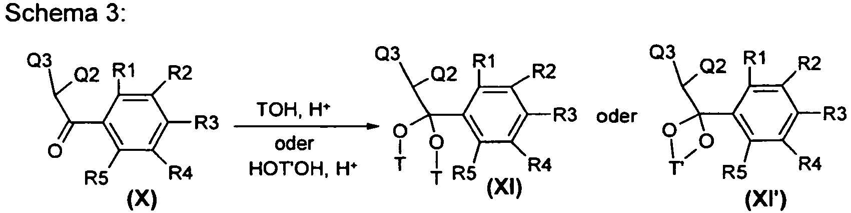

Die Reste R1, R2, R3, R4 oder R5, wobei ein R1, R2, R3, R4 oder R5 für SF₅ steht, sind wie in Formel I definiert. Der Rest T entspricht einer -(C₁-C₄)-Alkylgruppe. Der Rest T' entspricht Ethylen, Propylen oder Butylen oder bildet zusammen mit der Gruppe -O-C-O- einen 1,3- Dioxo-Ring der Ringgröße 5, 6 oder 7. Ketale dieses Typs werden durch Umsetzung mit Alkylenglykolen wie Ethylenglykol in Gegenwart von Säuren wie Schwefelsäure oder para-Toluolsulfonsäure und/oder wasserentziehenden Mitteln erhalten. Im einfachsten Fall arbeitet man in Toluol in Gegenwart katalytischer Mengen para-Toluolsulfonsäure am Wasserabscheider.

Komplexer substituierte Verbindungen der Formel X, worin ein R1, R2, R3, R4 oder R5 für Pentafluorsulfanyl (SF₅) steht, und wenigstens ein weiterer Rest R1, R2, R3, R4 oder R5 die Bedeutung in Formel I aufweist, lassen sich ausgehend von käuflichen Pentafluorsulfanyl-Derivaten gewinnen. Nicht käufliche Derivate können in Analogie zu bekannten Herstellungsverfahren gewonnen werden (Tetrahedron 56 (2000) 3399; Organic Letters 4 (17) (2002) 3013; WO 2005/047240). Für das 1-(3-Dimethyl-amino-5-pentafluorsulfanyl-phenyl)-ethanon ist ein Syntheseweg in Schema 4 gezeigt.

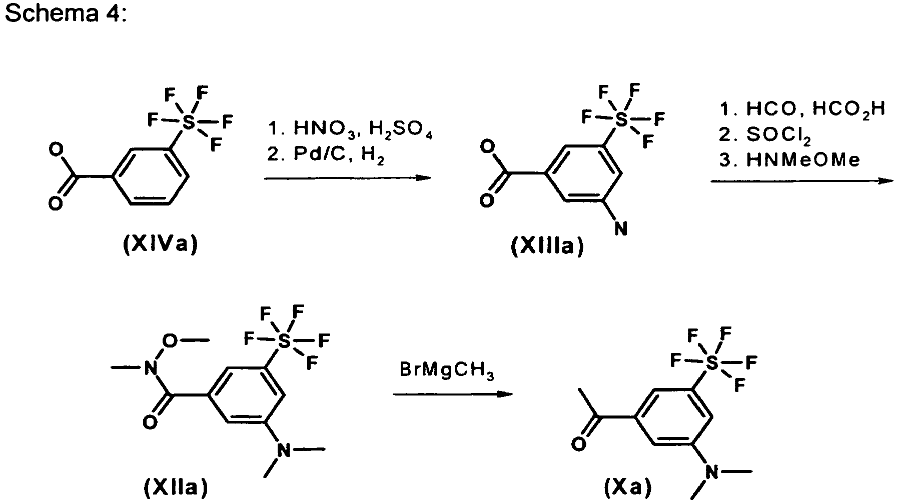

Ausgehend von käuflicher 3-(Pentafluorsulfanyl)-benzoesäure XIVa wurde mit dem Fachmann bekannten Umsetzungen zunächst nitriert und anschließend mit Palladium auf Kohle in Gegenwart von Wasserstoff zum Amin reduziert. Die erhaltene 3-Amino-5-pentafluorsulfanyl-benzoesäure XIIIa wurde dann unter Eschweiler-Clark-Bedingungen am Aminstickstoff dimethyliert, die Carbonsäure mit Thionylchlorid ins Säurechlorid übergeführt und anschließend mit O,N-Dimethyl-hydroxylamin umgesetzt. Das so gewonnene 3-Dimethylamino-N-methoxy-N-methyl--5-pentafluorsulfanyl-benzamid XIIa wurde mit Methylmagnesiumbromid in die entsprechenden Pentafluorsulfanyl-Derivate der Formel Xa übergeführt.

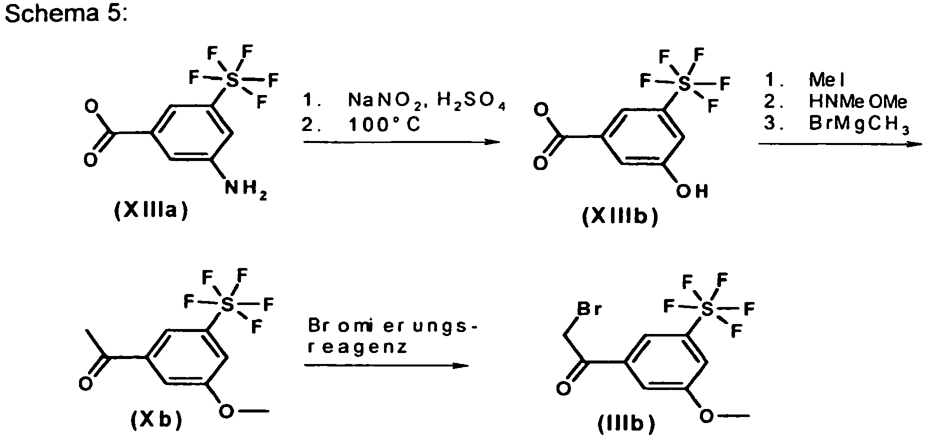

Phenolische 3-(Pentafluorsulfanyl)-benzoesäure-Derivate der Formel Xlllb lassen sich, wie in Schema 5 gezeigt, ausgehend von der 3-Amino-5-pentafluorsulfanyl-benzoesäure XIIIa durch Diazotierung und Verkochung gewinnen. Nachfolgende Methylierung, Bildung des Weinreb-Amids und Umsetzung mit Methylmagnesiumbromid liefert den Phenolether Xb, der entsprechend Schema 2 zum Acylbromid IIIb bromiert werden kann.

Eine nach Schema 1 hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren und Abspaltung der chiralen Hilfsgruppen in die reinen Enantiomeren aufgetrennt werden (Verfahren c), oder die nach Schema 1 hergestellte Verbindung der Formel I kann entweder in freier Form isoliert oder im Falle des Vorliegens von sauren oder basischen Gruppen in physiologisch verträgliche Salze umwandelt werden (Verfahren d).

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze oder Hydrochloride, Sulfate, Hemisulfate, Methylsulfonate, p-Toluolsulfonate, alle möglichen Phosphate sowie Salze der Aminosäuren, natürlicher Basen oder Carbonsäuren wie Lactate, Citrate, Tartrate, Acetate, Adipinate, Fumarate, Gluconate, Glutamate, Maleinate oder Pamoate.
Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Enthalten Verbindungen der Formel I saure Funktionalität, so lassen sich mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze bilden. Basische Gruppen der Verbindungen der Formel I, bilden mit Säuren Säureadditionssalze. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzolsulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin- oder Trifluoressigsäure in Frage.

Im Verfahrensschritt d) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, kann auch eine fraktionierte Kristallisation der mit einer optisch aktiven Base oder Säure als Hilfsstoff gebildeten diastereomeren Salze durchgeführt werden. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-Funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der Verbindungen der Formel I die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung des Protease-aktivierten Rezeptors 1 (PAR1) behandelbar sind. So eignen sich die erfindungsgemäßen Verbindungen sowohl für eine prophylaktische als auch für eine therapeutische Anwendung am Menschen. Sie eignen sich sowohl für eine akute Behandlung als auch für eine Langzeittherapie. Die Verbindungen der Formel I können eingesetzt werden in Patienten, die an Störungen des Wohlbefindens oder Krankheiten leiden, die mit Thrombosen, Embolien, Hyperkoagulabilität, fibrotischen Veränderungen oder entzündlichen Erkrankungen einhergehen.

Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können die Verbindungen der Formel I eingesetzt werden bei allen Eingriffen, die zu einem Kontakt des Blutes mit Fremdoberflächen führen wie bei Dialysepatienten und Patienten mit Verweilkathetern. Verbindungen der Formel I können eingesetzt werden, um die Thrombosegefahr nach chirurgischen Eingriffen wie bei Knie- und Hüftgelenksoperationen zu reduzieren.

Verbindungen der Formel I eignen für die Behandlung von Patienten mit disseminierter intravaskulärer Koagulation, Sepsis und anderen intravaskulären Ereignissen, die mit einer Entzündung einhergehen. Weiterhin eignen sich Verbindungen der Formel I für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. Verbindungen der Formel I eignen sich zur Verlangsamung oder Verhinderung solcher Prozesse.
Weitere Indikationen für den Einsatz der Verbindungen der Formel I sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formel I eignen sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung von Stents mit Verbindungen der Formel I und anderen Oberflächen, die im Körper mit Blut in Kontakt kommen, ist möglich.
Die Erfindung betrifft auch ein Verfahren zur Herstellung eines Arzneimittels, das dadurch gekennzeichnet ist, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.
Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser, physiologische Kochsalzlösung und ein- oder mehrwertige Alkohole wie Glycerin, genannt.
Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.
Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 10 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.
Verbindungen der Formel I können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden. Als Plättchenaggregationshemmer kommen dabei Cyclooxygenase 1-Inhibitoren wie Aspirin, irreversible P2Y₁₂-Antagonisten wie Clopidogrel oder Prasugrel, reversible P2Y₁₂-Antagonisten wie Cangrelor oder AZD6140 und Thromboxan A₂/Prostaglandin H₂-Antagonisten wie Terutroban in Frage. Additive Effekte von PAR 1-Blockade in Kombination mit P2Y₁₂-Blockade konnten beispielweise bereits gezeigt werden (Eur. Heart J. 2007, 28, Abstract Supplement, 188).

### Beispiele

Endprodukte wurden in der Regel durch eine chromatographisch-massenspektroskopische Methode (LCUV/ESI-MS-Kopplung) und ¹H-NMR charakterisiert. Beschrieben sind die Verbindungen durch Angabe der zugehörigen Retentionszeit im Ionenstrom (LCMS-Rt) und des entsprechenden M+H⁺-Signals bei positiver Ionisiation im zugehörigen Massenspektrum. Konnte kein M+H⁺-Massensignal erhalten werden, wurden alternativ die ¹H-NMR-Daten angegeben. Verwendete Abkürzungen sind entweder erläutert oder entsprechen den üblichen Konventionen. Soweit nicht anders angegeben, wurden chromatographische Trennungen an Kieselgel mit Ethylacetat/Heptan- oder Dichlormethan/Methanol-Gemischen als Laufmittel durchgeführt.
Das Abdampfen von Lösungsmitteln geschah in der Regel unter vermindertem Druck bei 35 °C bis 45 °C am Rotationsverdampfer und wird mit "vom Lösungsmittel befreit", "eingeengt", "einrotiert", "zur Trockne gebracht" oder "Lösungsmittel entfernt bzw. abgezogen" umschrieben. Wenn nicht anders aufgeführt, wurden die LCUV/MS-Analysen unter folgenden Bedingungen durchgeführt:

| | |
|---|---|
| System: | Agilent 1100 HPLC-System gekoppelt an 1100 LC/MSD |
| Säule: | YMC J'sphere ODS H80 20x2,1 mm, Packungsmaterial 4 µ |
| Laufmittel: | ACN:H₂O+0,05 % TFA (Fluss 1 ml/min) |
| Gradient: | 4:96 (0 min) → 95:5 (2 min) → 95:5 (2,4 min) →4:96 (2,45 min) |
| Ionisierung: | ESI⁺ |

Alternativ und mit "Met-b" gekennzeichnet wurden folgende Bedingungen gewählt:

| | |
|---|---|
| System: | Agilent 1200 HPLC-System gekoppelt an 6120 LC/MS |
| Säule: | Luna C18, 10 x 2,0 mm, Packungsmaterial 3 µm |
| Laufmittel: | ACN:H₂O+0,05% TFA (Fluss 1,1 ml/min) |
| Gradient: | 7:93 (0 min) → 95:5 (1 min) → 95:5 (1,45 min) → 7:93 (1,5 min) |
| Ionisierung: | ESI⁺ |

Präparative HPLC mit Reversed-Phase-(RP)-Kieselgel wurde mit den folgenden Methoden durchgeführt:
Methode A, Standard-Methode wenn im Text keine andere erwähnt ist

| | |
|---|---|
| Säule: | Merck (Darmstadt) Purosphere® RP18 25x250 mm, 10 µ |
| Laufmittel: | ACN:H₂O +0,05%TFA (Fluss 25 ml/min) |
| Gradient: | 10:90 (0 min) → 90:10 (40 min) |

Methode B

| | |
|---|---|
| Säule: | Merck (Darmstadt) Purosphere® RP18 25x250 mm, 10 µ |
| Laufmittel: | ACN:H₂O+0,05 % TFA (Fluss 25 ml/min) |
| Gradient: | 0:100 (0 min) → 0:100 (5 min) → 20:80 (20 min) |

In den Beispielen 22 d) und 23 c) wurde Methode C eingesetzt:
Methode C

| | |
|---|---|
| Säule: | Agilent Prep-C18, 30 x 250 mm, 10 µ |
| Laufmittel: | ACN:H₂O+0,05 % TFA (Fluss 75 ml/min) |
| Gradient: | 10:90 (0 min) → 90:10 (12,5 min) → 90:10 (15min) →10:90 (15,5 min) →10:90 (17.5 min) |

Die Umsetzungen fanden in Standard-Reaktionsapparaturen wie Ein- oder Mehrhalskolben statt, die wenn nicht anders beschrieben, dem Bedarf angemessen 5 ml bis 2000 ml Volumen fassten und je nach Anforderung mit Septum, Stopfen, Kühler, Rührer oder anderen Ausrüstungsgegenständen bestückt waren. Wenn nicht anders erwähnt, fanden alle Reaktionen unter Argon als Schutzgas statt und wurden mit Magnetrührern gerührt.
Mikrowellenreaktionen wurden im Emrys Optimizer von Personal Chemistry in dem Bedarf angepassten Gefäßen von 0,5 bis 10 ml Fassungsvermögen durchgeführt.

### Verwendete Abkürzungen:

- abs.: absolut
- ACN: Acetonitril
- Boc: Butoxycarbonyl
- DCM: Dichlormethan
- DIPEA: N,N-Diisopropylethylamin (Hünig-Base)
- DME: Dimethoxyethan
- DMF: Dimethylformamid
- DMSO: Dimethylsulfoxid
- h: Stunde(n)
- EE: Essigsäureethylester
- LCMS-Rt: Retentionszeit der Verbindung im Ionenstrom
- LCUV/MS: Flüssigkeitschromatographie Ultraviolett-/Massenspektroskopie
- min: Minunte(n)
- MtB-Ether: tertiär-Butylmethylether
- MeOH: Methanol
- RT: Raumtemperatur (20 °C bis 25 °C)
- TFA: Trifluoressigsäure
- THF: Tetrahydrofuran

### Beispiel 1

### 2-(1-Imino-1,3-dihydro-isoindol-2-yl)-1-(3-pentafluorsulfanyl-phenyl)-ethanon als Hydrobromid

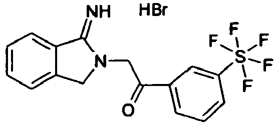

### 1a) 2-Brom-1-(3-pentafluorsulfanyl-phenyl)-ethanon

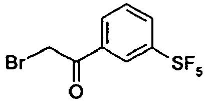

3-Pentafluorsulfanyl-acetophenon (400 mg) wurde in Eisessig (10 ml) vorgelegt und Brom (91 µl, gelöst in 1 ml Eisessig) langsam zugetropft. Nach 4 Stunden Rühren bei RT wurde über Nacht stehen gelassen und dann vom Lösungsmittel befreit. Der Rückstand wurde zwei Mal mit Toluol aufgenommen und zur Trockne gebracht. Das erhaltene Rohprodukt wurde mittels präparativer HPLC aufgereinigt. Die produktenthaltenden Fraktionen wurden vereinigt, vom Acetonitril befreit und gefriergetrocknet. Es wurden 252 mg der gewünschten Verbindung erhalten.

| | |
|---|---|
| LCMS-Rt: 1,69 min | [M+H]⁺: 324,9 |

### 1b) 3H-Isoindol-1-ylamin als Hydrobromid

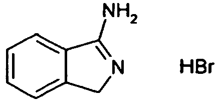

Ammoniak (250 ml) wurde in einen Kolben einkondensiert und bei -78°C α-Brom-o-tolunitril (20 g) in THF (100 ml) gelöst unter Rühren langsam über 1,5 h zugetropft. Nach einer Stunde Rühren wurde das Kältebad entfernt und über Nacht stehen gelassen, so dass das Ammoniak größtenteils verdampfen konnte. Der Rückstand wurde mit Wasser versetzt, das erhaltene Gemisch im Vakuum reduziert und mit Essigester versetzt. Die Essigesterphase wurde abgetrennt und der Rückstand erneut mit Essigester extrahiert. Die vereinigten Essigesterphasen wurden drei Mal mit Wasser gewaschen und die wässrigen Phasen mit dem wässrigen Rückstand vereinigt. Die wässrige Phase wurde im Vakuum auf die Hälfte reduziert und dann gefriergetrocknet. Es wurden 19,9 g der Titelverbindung erhalten.

| | |
|---|---|
| LCMS-Rt: 0,33 min | [M+H]⁺: 133,1 |

1c) 2-(1-Imino-1,3-dihydro-isoindol-2-yl)-1-(3-pentafluorsulfanyl-phenyl)-ethanon als Hydrobromid3H-Isoindol-1-ylamin als Hydrobromid (800 mg) wurde in wenig Wasser gelöst, mit festen Natriumhydroxid-Plätzchen versetzt und mehrmals mit Essigester extrahiert. Die vereinigten Essigesterphasen wurden über Magnesiumsulfat getrocknet, filtriert und eingeengt. Ein Teil der erhaltenen freien Base (30 mg) wurde in absolutem THF (5 ml) gelöst und unter Rühren 2-Brom-1-(3-pentafluorsulfanyl-phenyl)-ethanon (73 mg), gelöst in absolutem THF (1 ml), zugetropft. Das Gemisch wurde 6 h weiter bei RT gerührt und dann übers Wochenende in den Kühlschrank gestellt. Der gebildete Niederschlag wurde abgesaugt, in einem Wasser/Acetonitril-Gemisch gelöst und die Lösung gefriergetrocknet. Es wurden 58 mg der Titelverbindung erhalten.

| | |
|---|---|
| LCMS-Rt: 1,21 min | [M+H]⁺: 377,0 |

### Beispiel 2

### 2-[2-(3-Dimethylamino-5-pentafluorsulfanyl-phenyl)-2-oxo-ethyl]-6-ethoxy-3-imino-2,3-dihydro-1H-isoindol-5-carbonsäuremethylamid als Trifluoressigsäuresalz

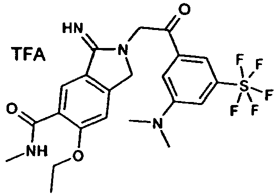

### 2a) 3-Amino-5-pentafluorsulfanyl-benzoesäure

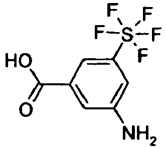

3-Pentafluorsulfanyl-benzoesäure (3,0 g) wurde in rauchender Salpetersäure (20 ml) gelöst und bei RT unter Feuchtigkeitsausschluss gerührt. Dann wurde konzentrierte Schwefelsäure (1,5 ml) zugegeben und bei 75 °C gerührt. Nach 6 h Rühren bei 75°C wurde über Nacht stehen gelassen, dann weitere Schwefelsäure (1,5 ml) zugegeben und 8 h unter Rühren auf 75°C erhitzt. Nach Stehenlassen über Nacht wurde auf Eiswasser gegeben und 2 h gerührt. Die einsetzende Kristallisation wurde über Nacht im Kühlschrank vervollständigt. Dann wurde der Niederschlag abgesaugt und am Hochvakuum getrocknet. Es wurden 2,7 g 3-Pentafluorsulfanyl-5-nitro-benzoesäure erhalten. Weitere 530 mg konnten aus der Mutterlauge nach dreimaligem Extrahieren mit Methylenchlorid, Trockenen der vereinigten Methylenchlorid-Phasen über Magnesiumsulfat und Einengen des Lösungsmittels erhalten werden. Anschließend wurden die 3-Pentafluorsulfanyl-5-nitro-benzoesäure (2,7 g) in Methanol (70 ml) gelöst, Raney-Nickel (etwa 500 mg) zugegeben und unter Wasserstoffatmosphäre (Wasserstoffballon) hydriert. Nach 2 h wurde der Katalysator abfiltriert und der Filterrückstand gut mit Methanol gewaschen. Das Filtrat wurde eingeengt und am Hochvakuum getrocknet. Es wurden 2,3 g Rohprodukt erhalten, die direkt in der nächsten Stufe umgesetzt wurden.

| | |
|---|---|
| LCMS-Rt: 1,21 min | [M+H]⁺: 264,0 |

### 2b) 3-Dimethylamino-5-pentafluorsulfanyl-benzoesäure

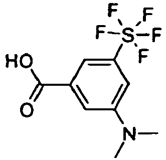

In zwei Mikrowellengefäßen wurden jeweils 3-Amino-5-pentafluorsulfanylbenzoe-säure (800 mg), Ameisensäure (6 ml) und 37 %ige Formalin-Lösung (4 ml) gegeben. Beide Gefäße wurden dann 30 min auf 110 °C erhitzt. Nach Abkühlen wurden die Lösungen vereinigt und auf Eiswasser gegeben. Nach dreimaliger Extraktion mit Essigester wurden die vereinigten organischen Phasen mit Magnesiumsulfat getrocknet, filtriert und eingeengt. Es wurden 1,76 g der Titelverbindung erhalten, die direkt in der nächsten Stufe umgesetzt wurden.

| | |
|---|---|
| LCMS-Rt: 1,48 min | [M+H]⁺: 292,0 |

### 2c) 3-Dimethylamino-N-methoxy-N-methyl-5-pentafluorsulfanyl-benzamid

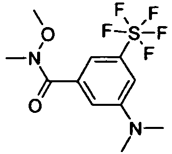

3-Dimethylamino-5-pentafluorsulfanyl-benzoesäure (1,0 g) wurde in Methylenchlorid (60 ml) gelöst. Unter Rühren wurde Thionylchlorid (5 ml) zugegeben und 2 h bei RT gerührt. Zur Vervollständigung der Reaktion wurde anschleißend 3 h auf Rückfluss erhitzt. Nach Abkühlen wurde das Lösungsmittel abgezogen, der Rückstand in Methylenchlorid (50 ml) gelöst, mit N,O-Dimethylhydroxylamin-Hydrochlorid (315 mg) versetzt und Hünig-Base (1 ml) zugegeben. Nach einer Stunde Rühren wurde das Lösungsmittel abgezogen, der Rückstand mit Essigester aufgenommen und 5 Mal mit Wasser gewaschen. Die organische Phase wurde mit Magnesiumsulfat getrocknet, filtriert und eingeengt. Es wurden 980 mg der Titelverbindung erhalten, die direkt in der nächsten Stufe umgesetzt wurden.

| | |
|---|---|
| LCMS-Rt: 1,53 min | [M+H]⁺: 335,0 |

### 2d) 1-(3-Dimethylamino-5-pentafluorsulfanyl-phenyl)-ethanon

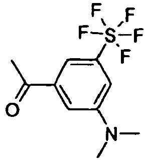

3-Dimethylamino-N-methoxy-N-methyl-5-pentafluorsulfanyl-benzamid (980 mg) wurde in abs. THF (50 ml) gelöst und bei 0 °C unter Rühren Methylmagnesium-bromid-Lösung (2,1 ml; 3 M Lösung in Diethylether) zugetropft. Nach beendeter Zugabe wurde das Eisbad entfernt und 1 h bei RT gerührt. Zur Vervollständigung der Reaktion wurde mehr Methylmagnesiumbromid-Lösung (0,3 ml) zugegeben und weitere 2 h gerührt. Nach Lagerung über Nacht im Kühlschrank wurde das Reaktionsgemisch unter Kühlung mit 1 N Salzsäure versetzt. Nach Zugabe von Wasser und Essigester wurde noch zwei Mal mit Essigester extrahiert, die vereinigten organischen Phasen mit Magnesiumsulfat getrocknet, filtriert und eingeengt. Der Rückstand wurde mittels Chromatographie an Kieselgel (n-Heptan/Essigester 100/0 auf 50/50 in 30 min) gereinigt. Die produktenthaltenden Fraktionen wurden vereinigt, das Lösungsmittel entfernt und der Rückstand im Hochvakuum getrocknet. Es wurden 650 mg der Titelverbindung erhalten.

| | |
|---|---|
| LCMS-Rt: 1,69 min | [M+H]⁺: 290,0 |

### 2e) [3-(1,1-Dimethoxy-ethyl)-5-pentafluosulfanyl-phenyl]-dimethyl-amin

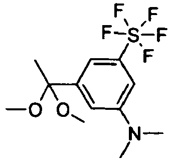

1-(3-Dimethylamino-5-pentafluorsulfanyl-phenyl)-ethanon (650 mg) wurde in Methanol (50 ml) gelöst und unter Rühren mit Trimethylorthoformiat (715 mg) und DL-10-Camphersulfonsäure (10 mg) versetzt. Nach 3 h Rühren wurde mehr Orthoformiat (200 mg) zugegeben, 2 h gerührt und über Nacht stehen gelassen. Dann wurde das Lösungsmittel abgezogen und der Rückstand im Hochvakuum getrocknet. Es wurden 730 mg Rohprodukt erhalten, die direkt in der nächsten Stufe umgesetzt wurden.
¹H-NMR (400 MHz, DMSO-d₆) [ppm]: 7,10 (1 H); 6,99 (1 H); 6,93 (1H); 3,10 (6 H); 2,98 (6 H); 1,47 (3 H)

### 2f) [3-(2-Brom-1,1-dimethoxy-ethyl)-5-pentafluorsulfanyl-phenyl]-dimethyl-amin und [3-(2-Brom-1,1-dimethoxy-ethyl)-5-pentafluorsulfanyl-phenyl]-methyl-amin

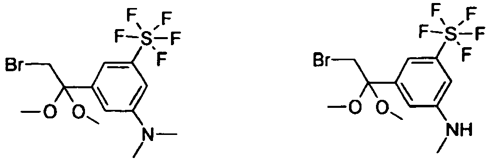

[3-(1,1-Dimethoxy-ethyl)-5-pentafluosulfanyl-phenyl]-dimethyl-amin (730 mg) wurde in einem Gemisch aus Methanol (15 ml) und THF (15 ml) gelöst. Unter Rühren wurde Phenyl-trimethyl-tribromid (818 mg) zugegeben. Nach 3 h wurde zur Vervollständigung der Reaktion mehr Phenyl-trimethyl-tribromid (205 mg) zugegeben und 2 h bei 60°C gerührt. Nach Stehen über Nacht wurden Natriumthiosulfat-Lösung, Wasser und Essigester zugegeben. Die wässrige Phase wurde noch drei Mal mit Essigester extrahiert. Die vereinigten Extrakte wurden mit Magnesiumsulfat getrocknet, filtriert und eingeengt. Der Rückstand wurde mittels Chromatographie an Kieselgel (n-Heptan/Essigester 100/0 auf 50/50 in 30 min) gereinigt. Die produktenthaltenden Fraktionen wurden vereinigt, das Lösungsmittel entfernt und der Rückstand im Hochvakuum getrocknet. Es wurden 490 mg der Dimethylamino- und 144 mg der Monomethylamino-Verbindung erhalten.
Dimethylamin-Derivat:
¹H-NMR (500 MHz, DMSO-d₆) [ppm]: 7,14 (1 H); 7,00 (1 H); 6,95 (1H); 3,85 (2 H); 3,14 (6 H); 2,99 (6 H)
Monomethylamin-Derivat:
¹H-NMR (500 MHz, DMSO-d₆) [ppm]: 7,02 (1 H); 6,91 (1 H); 6,84 (1 H); 6,34 (1H); 3,80 (2 H); 3,14 (6 H); 2,71 (3 H)

### 2g) 2-Brom-1-(3-dimethylamino-5-pentafluorsulfanyl-phenyl)-ethanon

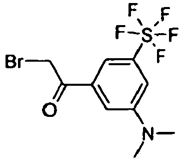

[3-(2-Brom-1,1-dimethoxy-ethyl)-5-pentafluorsulfanyl-phenyl]-dimethyl-amin (230 mg) wurde in Wasser (2,3 ml) suspendiert und dann unter Kühlung konzentrierte Schwefelsäure (2,3 ml) zugetropft. Nach 2 h Rühren bei RT wurde mit Wasser verdünnt (20 ml) und drei Mal mit Essigester extrahiert. Die vereinigten organischen Phasen wurden zwei Mal mit gesättigter Natriumhydrogencarbonat-Lösung gewaschen, über Magnesiumsulfat getrocknet, filtriert und eingeengt. Der Rückstand wurde in Acetonitril/Wasser gelöst, eingefroren und über Nacht gefriergetrocknet. Es wurden 170 mg der gewünschten Verbindung erhalten.

| | |
|---|---|
| LCMS-Rt: 1,80 min | [M+H]⁺: 367,9 |

### 2h) 2-[2-(3-Dimethylamino-5-pentafluorsulfanyl-phenyl)-2-oxo-ethyl]-6-ethoxy-3-imino-2,3-dihydro-1H-isoindol-5-carbonsäuremethylamid als

Trifluoressigsäuresalz 3-Amino-6-ethoxy-1H-isoindol-5-carbonsäuremethylamid (15 mg, gekauft bei Chembiotek) wurde in absolutem DMF (2 ml) gelöst und unter Rühren 2-Brom-1-(3-dimethylamino-5-pentafluorsulfanyl-phenyl)-ethanon (25 mg), gelöst in absolutem THF (2,5 ml), zugetropft. Da nach 3 h Rühren bei RT und Stehenlassen über Nacht kaum eine Umsetzung zu erkennen war, wurde das Lösungsmittel im Vakuum entfernt und der Rückstand mit Ethanol aufgenommen und das Gemisch auf Rückfluss erwärmt. Nach 2 h wurde das Reaktionsgemsich eingeengt und mittels präparativer HPLC aufgereinigt. Die produktenthaltende Fraktion wurde vom Acetonitril befreit und gefriergetrocknet. Es wurden 2 mg der gewünschten Verbindung erhalten.

| | |
|---|---|
| LCMS-Rt: 1,24 min | [M+H]⁺: 521,0 |

### Beispiel 3

### 6-Ethoxy-3-imino-2-[2-(3-methylamino-5-pentafluorsulfanyl-phenyl)-2-oxo-ethyl]-2,3-dihydro-1H-isoindol-5-carbonsäuremethylamid als Trifluoressigsäuresalz

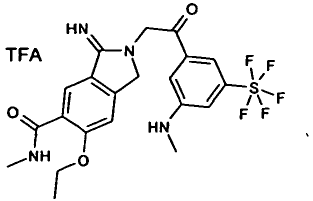

### 3a) 2-Brom-1-(3-methylamino-5-pentafluorsulfanyl-phenyl)-ethanon

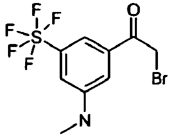

[3-(2-Brom-1,1-dimethoxy-ethyl)-5-pentafluorsulfanyl-phenyl]-methyl-amin (400 mg, Beispiel 2f) wurde in Wasser (4 ml) suspendiert und dann unter Eiskühlung mit konzentrierte Schwefelsäure (4 ml) versetzt. Nach 4 h Rühren bei RT wurde auf Eiswasser gegossen, mit gesättigter Natriumhydrogencarbonat-Lösung auf pH 8 gestellt und die wässrige Phase drei Mal mit Essigester extrahiert. Die vereinigten Extrakte wurden über Magnesiumsulfat getrocknet, filtriert und eingeengt. Es wurden 312 mg des gewünschten Produkts erhalten.

| | |
|---|---|
| LCMS-Rt: 1,67 min | [M+H]⁺: 353,9 |

### 3b) 6-Ethoxy-3-imino-2-[2-(3-methylamino-5-pentafluorsulfanyl-phenyl)-2-oxo-ethyl]-

2,3-dihydro-1H-isoindol-5-carbonsäuremethylamid als Trifluoressigsäuresalz 3-Amino-6-ethoxy-1H-isoindol-5-carbonsäuremethylamid (40 mg, gekauft bei Chembiotek) wurde mit absolutem DMF (8 ml) versetzt und unter Rühren 2-Brom-1-(3-methylamino-5-pentafluorsulfanyl-phenyl)-ethanon (61 mg), gelöst in absolutem DMF (1 ml), zugegeben. Nach 1 h Rühren und Stehengelassen über Nacht wurde das Reaktionsgemisch eingeengt und der Rückstand mittels präparativer HPLC gereinigt. Die produktenthaltenden Fraktionen wurden vereinigt, vom Acetonitril befreit und gefriergetrocknet. Es wurden 50 mg der gewünschten Verbindung erhalten.

| | |
|---|---|
| LCMS-Rt: 1,23 min | [M+H]⁺: 507,0 |

### Beispiel 4

### 6-Ethoxy-3-imino-2-{2-[3-pentafluorsulfanyl-5-(2,2,2-trifluor-acetylamino)-phenyl]-2-oxo-ethyl}-2,3-dihydro-1H-isoindol-5-carbonsäuremethylamid als Trifluoressigsäuresalz

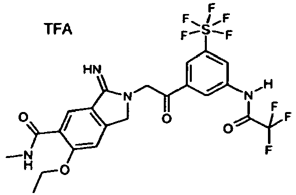

### 4a) N-Methoxy-N-methyl-5-nitro-3-pentafluorsulfanyl-benzamid

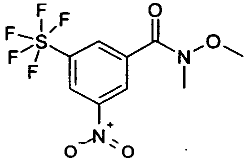

3-Pentafluorsulfanyl-5-nitro-benzoesäure (4,0 g, Beispiel 2a) wurde unter Rühren in Thionylchlorid (25ml) gelöst und 10 h unter Feuchtigkeitsausschluss unter Rückfluss gehalten. Nach Stehen über Nacht bei RT wurde überschüssiges Thionylchlorid im Vakuum entfernt, der erhaltene Rückstand in Dichlormethan (50 ml) gelöst und unter Rühren mit N,O-Dimethylhydroxylamin x HCl (1,25 g) und Diethylisopropylamin (1,66 g) versetzt. Nach 1 h Rühren bei RT wurde das Gemisch im Vakuum eingeengt, der Rückstand in Essigester gelöst und 5 Mal mit Wasser gewaschen. Die organische Phase wurde über Magnesiumsulfat getrocknet, filtriert und eingeengt. Die erhaltenen 4,2 g Rohprodukt wurden direkt in der nächsten Stufe eingesetzt.

| | |
|---|---|
| LCMS-Rt: 1,50 min | [M+H]⁺: 337,0 |

### 4b) 3-Amino-N-methoxy-N-methyl-5-pentafluorsulfanyl-benzamid

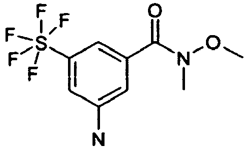

N-Methoxy-N-methyl-5-nitro-3-pentafluorsulfanyl-benzamid (4,2 g) wurde in Methanol (120 ml) gelöst und Raney - Nickel (ca. 700 mg) zugegeben. Mit aufgesetztem Wasserstoffballon wurde auf einem Magnetrührer hydriert. Nach 5 h wurde der Katalysator abfiltriert und mit Methanol gewaschen. Das Filtrat wurde im Vakuum eingeengt und der Rückstand mittels präparativer Chromatographie aufgereinigt. Die produktenthaltenden Fraktionen wurden vereinigt, vom Acetonitril befreit, mit Natriumhydrogencarbonat-Lösung basisch gestellt und mit Essigester drei Mal extrahiert. Die vereinigten Extrakte wurden über Magnesiumsulfat getrocknet, filtriert und eingeengt. Es wurden 1,73 g der gewünschten Verbindung erhalten.

| | |
|---|---|
| LCMS-Rt: 1,27 min | [M+H]⁺: 307,0 |

### 4c) N-Methoxy-N-methyl-5-pentafluorsulfanyl-3-(2,2,2-trifluor-acetylamino)-benzamid

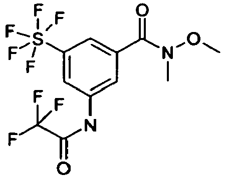

3-Amino-N-methoxy-N-methyl-5-pentafluorsulfanyl-benzamid (1,45 g) wurde in Methylenchlorid (15 ml) gelöst und unter Rühren Triethylamin (0,8 ml) gefolgt von Trifluoressigsäureanhydrid (0,85 ml) unter Feuchtigkeitsausschluss zugesetzt. Nach 3 h Rühren bei RT und Stehen über Nacht wurden Wasser und gesättigte Natriumhydrogencarbonat-Lösung zugegeben, die Phasen getrennt und die Methylenchlorid-Phase noch drei Mal mit Wasser gewaschen, über Magnesiumsulfat getrocknet, filtriert und eingeengt. Das erhaltene Produkt (1,75 g) wurde ohne weitere Reinigung in der nächsten Stufe eingesetzt.

| | |
|---|---|
| LCMS-Rt: 1,53 min | [M+H]⁺: 403,0 |

### 4d) N-(3-Acetyl-5-pentafluorsulfanyl-phenyl)-2,2,2-trifluor-acetamid und 1-[3-Amino-5-(pentafluorsulfanyl)-phenyl]-ethanon-Trifluoressigsäuresalz

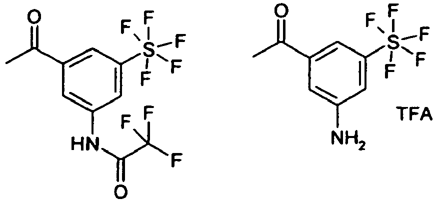

N-Methoxy-N-methyl-5-pentafluorsulfanyl-3-(2,2,2-trifluor-acetylamino)-benzamid (540 mg) wurde in absolutem THF (30 ml) gelöst und bei 0 °C mit Lithiumhexmethyldisilazan (312 µl, 23% in tert.-Butylmethylether) 30 min gerührt. Bei 0°C wurde dann unter Rühren Methylmagnesiumbromid (2,5 ml, 3 M in Diethylether) zugetropft. Nach 6 h Rühren bei RT und Stehenlassen über Nacht wurde unter Kühlung 1 N Salzsäure zugetropft, gefolgt von Wasser und Essigester. Die organische Phase wurde abgetrennt und die Wasserphase noch zwei Mal mit Essigester extrahiert. Die vereinigten Essigesterphasen wurden über Magnesiumsulfat getrocknet, filtriert und eingeengt. Das Rohprodukt wurde mittels präparativer Chromatographie aufgereinigt. Die produktenthaltenden Fraktionen wurden vereinigt, vom Acetonitril befreit und mit Essigester drei Mal extrahiert. Die vereinigten Extrakte wurden über Magnesiumsulfat getrocknet, filtriert und eingeengt. Es wurden 315 mg N-(3-Acetyl-5-pentafluorsulfanyl-phenyl)-2,2,2-trifluor-acetamid

| | |
|---|---|
| LCMS-Rt:1,60 min | [M+H]⁺: 358,0 |

und 42 mg 1-[3-Amino-5-(pentafluorsulfanyl)-phenyl]-ethanon-Trifluoressigsäuresalz erhalten.

| | |
|---|---|
| LCMS-Rt: 1,37 min | [M+H]⁺: 262,0 |

### 4e) N-[3-(2-Brom-acetyl)-5-pentafluorsulfanyl-phenyl]-2,2,2-trifluor-acetamid

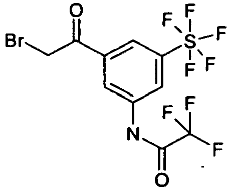

N-(3-Acetyl-5-pentafluorsulfanyl-phenyl)-2,2,2-trifluor-acetamid (110 mg) wurde in einem Gemisch aus Methanol (2 ml) und THF (2 ml) gelöst. Unter Rühren wurde Phenyl-trimethyl-tribromid (115 mg) fest zugegeben. Nach 3 h Rühren und Stehenlassen über Nacht wurde weiteres Phenyl-trimethyl-tribromid (29 mg) zugegeben und auf 60°C erwärmt. Nach 2 h wurde das erkaltete Reaktionsgemisch in verdünnte Zitronensäure gegeben und mit Essigester drei Mal extrahiert. Nach Abziehen des Lösungsmittels wurde der Rückstand in Acetonitril (3 ml) gelöst und mit 2 N Schwefelsäure (2 ml) versetzt. Nach 2 h Rühren wurden Wasser und Essigester zugegeben, die Phasen getrennt und die wässrige Phase drei Mal mit Essigester extrahiert. Die vereinigten Extrakte wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit, mit gesättigter Natriumhydrogencarbonat-Lösung versetzt und drei Mal mit Essigester extrahiert. Die vereinigten Extrakte wurden über Magnesiumsulfat getrocknet, filtriert und eingeengt. Es wurden 58 mg der gewünschten Verbindung erhalten.

| | |
|---|---|
| LCMS-Rt: 1,72 min | [M+H]⁺: 436,0 |

### 4f) 6-Ethoxy-3-imino-2-{2-[3-pentafluorsufanyl-5-(2,2,2-trifluor-acetylamino)-phenyl]-2-oxo-ethyl}-2,3-dihydro-1H-isoindol-5-carbonsäuremethylamid als Trifluoressigsäuresalz

3-Amino-6-ethoxy-1H-isoindol-5-carbonsäuremethylamid (30 mg, gekauft bei Chembiotek) wurde mit N-[3-(2-Brom-acetyl)-5-pentafluorsulfanyl-phenyl]-2,2,2-trifluoracetamid (50 mg) analog zu Beispiel 3b) umgesetzt. Es wurden 31 mg der gewünschten Verbindung erhalten.

| | |
|---|---|
| LCMS-Rt: 1,30 min | [M+H]⁺: 589,0 |

### Beispiel 5

### 6-Ethoxy-2-(2-{3-[ethyl-(2,2,2-trifluor-acetyl)-amino]-5-pentafluorsulfanyl-phenyl}-2-oxo-ethyl)-3-imino-2,3-dihydro-1H-isoindol-5-carbonsäuremethylamid als Trifluoressigsäuresalz

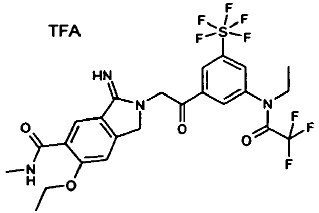

### 5a) N-(3-Acetyl-5-pentafluorsulfanyl-phenyl)-N-ethyl-2,2,2-trifluor-acetamid

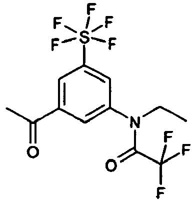

N-(3-Acetyl-5-pentafluorsulfanyl-phenyl)-2,2,2-trifluor-acetamid (100 mg, Beispiel 4d) wurde in absolutem Dimethoxyethan (5 ml) in einem Mikrowellen-Einsatz gelöst und mit gepulvertem Kaliumcarbonat (40 mg) versetzt. Nach Zugabe von Jodethan (60 µl) wurde das Gemisch in der Mikrowelle 30 min auf 100°C erhitzt. Anschließend wurde weiteres Jodmethan (60 µl) zugegeben und erneut 30 min auf 100°C erhitzt. Dieser Vorgang wurde noch einmal wiederholt. Dann wurde das Gemisch zur Aufarbeitung auf ein Gemisch aus Wasser, Essigester und 1 N HCl gegeben. Nach Abtrennen der organischen Phase wurde noch drei Mal mit Essigester extrahiert. Die vereinigten Extrakte wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit und drei Mal mit Essigester extrahiert. Die vereinigten Extrakte wurden über Magnesiumsulfat getrocknet, filtriert und eingeengt. Es wurden 50 mg der gewünschten Verbindung erhalten.

| | |
|---|---|
| LCMS-Rt: 1,68 min | [M+H]⁺: 386,0 |

### 5b) N-[3-(2-Brom-acetyl)-5-pentafluorsulfanyl-phenyl]-N-ethyl-2,2,2-trifluor-acetamid

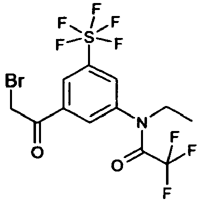

N-(3-Acetyl-5-pentafluorsulfanyl-phenyl)-N-ethyl-2,2,2-trifluor-acetamid (50 mg) wurde analog Beispiel 4e) bromiert, wobei in diesem Fall die Reaktion nach 4 h Rühren bei RT aufgearbeitet wurde. Nach Reinigung wurden 40 mg des gewünschten Produkts erhalten.

| | |
|---|---|
| LCMS-Rt: 1,79 min | [M+H]⁺: 464,0 |

### 5c) 6-Ethoxy-2-(2-{3-[ethyl-(2,2,2-trifluor-acetyl)-amino]-5-pentafluorsulfanylphenyl}-2-oxo-ethyl)-3-imino-2,3-dihydro-1H-isoindol-5-arbonsäuremethylamid als Trifluoressigsäuresalz

3-Amino-6-ethoxy-1H-isoindol-5-carbonsäuremethylamid (18 mg, gekauft bei Chembiotek) wurde mit N-[3-(2-Brom-acetyl)-5-pentafluorsulfanyl-phenyl]-N-ethyl-2,2,2-trifluor-acetamid (35 mg) analog zu Beispiel 3b) umgesetzt. Es wurden 16 mg der gewünschten Verbindung erhalten.

| | |
|---|---|
| LCMS-Rt: 1,28 min | [M+H]⁺: 617,2 |

### Beispiel 6

### 6-Ethoxy-2-[2-(3-ethylamino-5-pentafluorsulfanyl-phenyl)-2-oxo-ethyl]-3-imino-2,3-dihydro-1H-isoindol-5-carbonsäuremethylamid als Hydrochlorid

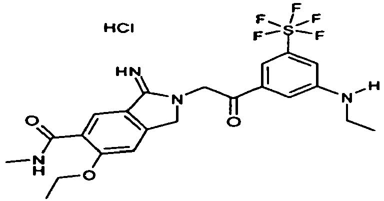

6-Ethoxy-2-(2-{3-[ethyl-(2,2,2-trifluor-acetyl)-amino]-5-pentafluorsulfanyl-phenyl}-2-oxo-ethyl)-3-imino-2,3-dihydro-1H-isoindol-5-arbonsäuremethylamid-Trifluoressigsäuresalz (11 mg, Beispiel 5c) wurde mit Wasser (1,5 ml) versetzt und unter Rühren und Eiskühlung konz. Schwefelsäure (1,5 ml) zugegeben. Anschließend wurde 5 h auf 80°C erwärmt. Nach Abkühlen wurde die Lösung unter Eiskühlung und Rühren langsam in ein Gemisch aus Essigester und 10 N Natronlauge gegeben. Die wässrige Phase wurde fünf Mal mit Essigester extrahiert. Die vereinigten Extrakte wurden über Magnesiumsulfat getrocknet, filtriert und eingeengt. Der Rückstand wurde in einem Gemisch aus Wasser und Acetonitril gelöst. Mit 0,1 N HCl wurde auf pH 2 gestellt und anschließend gefriergetrocknet. Es wurden 5 mg der gewünschten Verbindung erhalten.

| | |
|---|---|
| LCMS-Rt: 1,29 min | [M+H]⁺: 521,1 |

### Beispiel 7

### 2-[2-(3-Amino-5-pentafluorsulfanyl-phenyl)-2-oxo-ethyl]-6-ethoxy-3-imino-2,3-dihydro-1H-isoindol-5-carbonsäuremethylamid als Trifluoressigsäuresalz

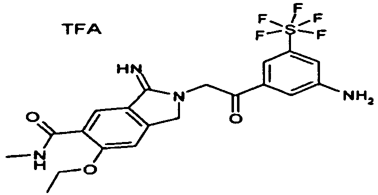

6-Ethoxy-3-imino-2-{2-[3-pentafluorsulfanyl-5-(2,2,2-trifluor-acetylamino)-phenyl]-2-oxo-ethyl}-2,3-dihydro-1H-isoindol-5-carbonsäuremethylamid-Trifluoressigsäuresalz (20 mg, Beispiel 4f) wurde mit 2 N Schwefelsäure (2 ml) und DMF (0,75 ml) versetzt und nach Zugabe eines Tropfens konz. Schwefelsäure 3 h auf 80°C erwärmt. Nach Erkalten wurde mit gesättigter Kaliumcarbonat-Lösung auf pH 9 gestellt und die Wasserphase 3 Mal mit Essigester extrahiert. Die vereinigten Extrakte wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit und gefriergetrocknet. Es wurden 3,4 mg der gewünschten Verbindung erhalten.

| | |
|---|---|
| LCMS-Rt: 1,15 min | [M+H]⁺: 493,0 |

### Beispiel 8

### 2-[2-(3-Acetylamino-5-pentafluorsulfanyl-phenyl)-2-oxo-ethyl]-6-ethoxy-3-imino-2,3-dihydro-1H-isoindol-5-carbonsäuremethylamid als Trifluoressigsäuresalz

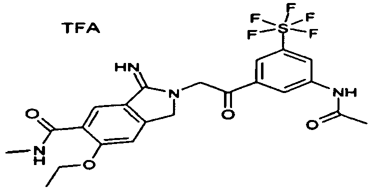

### 8a) N-[3-Acetyl-5-(pentafluorsulfanyl)-phenyl]-acetamid

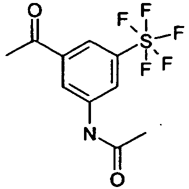

1-[3-Amino-5-(pentafluorsulfanyl)-phenyl]-ethanon-Trifluoressigsäuresalz (100 mg, Beispiel 4d) wurde in Methylenchlorid (10 ml) gelöst und Triethylamin (120 µl) zugesetzt. Unter Rühren wurde dann Essigsäureanhydrid (30 µl) zugetropft. Nach 4 h Rühren wurde über Nacht stehengelassen und dann weiteres Essigsäureanhydrid (30 µl) zugegeben. Nach weiteren 6 h Rühren wurde das Reaktionsgemisch erneut über Nacht stehengelassen und dann eingeengt. Der Rückstand wurde mit Essigester und Wasser aufgenommen, mit gesättigter Natriumhydrogencarbonat-Lösung auf pH 9 gestellt und die wässrige Phase noch zweimal mit Essigester extrahiert. Die vereinigten Extrakte wurden über Magnesiumsulfat getrocknet, filtriert und eingeengt. Es wurden 80 mg der gewünschten Verbindung erhalten.

| | |
|---|---|
| LCMS-Rt: 1,34 min | [M+H]⁺: 304,0 |

### 8b) N-[3-(2-Brom-acetyl)-5-pentafluorsulfanyl-phenyl]-acetamid

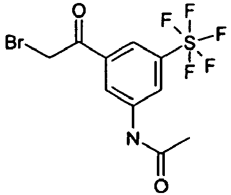

N-[3-Acetyl-5-(pentafluorsulfanyl)-phenyl]-acetamid (80 mg) wurde in einem Gemisch aus Methanol (2,5 ml) und THF (2,5 ml) gelöst und unter Rühren Phenyl-trimethyl-tribromid (100 mg) zugegeben. Nach 5 h Rühren bei RT wurde weiteres Phenyl-trimethyl-tribromid (25 mg) zugegeben und 2 h auf 60°C erwärmt. Nach dem Erkalten wurde das Reaktionsgemisch in 2 N Schwefelsäure gegeben und die wässrige Phase 3 Mal mit Essigester extrahiert. Die vereinigten Extrakte wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit, mit Natriumhydrogencarbonat basisch gestellt und 3 Mal mit Essigester extrahiert. Die vereinigten Extrakte wurden über Magnesiumsulfat getrocknet, filtriert und eingeengt. Es wurden 24 mg der gewünschten Verbindung erhalten.

| | |
|---|---|
| LCMS-Rt: 1,49 min | [M+H]⁺: 382,0 |

8c) 2-[2-(3-Acetylamino-5-pentafluorsulfanyl-phenyl)-2-oxo-ethyl]-6-ethoxy-3-imino-2,3-dihydro-1H-isoindol-5-carbonsäuremethylamid als Trifluoressigsäuresalz 3-Amino-6-ethoxy-1H-isoindol-5-carbonsäure-methylamid (13 mg, gekauft bei Chembiotek) wurde mit N-[3-(2-Brom-acetyl)-5-pentafluorsulfanyl-phenyl]-acetamid (21 mg) analog zu Beispiel 3b) umgesetzt. Es wurden 11 mg der gewünschten Verbindung erhalten.

| | |
|---|---|
| LCMS-Rt: 1,17 min | [M+H]⁺: 535,0 |

### Beispiel 9

### 2-(5,6-Diethoxy-7-fluor-1-imino-1,3-d ihydro-isoindol-2-yl)-1-[3-methylamino-5-(pentamethyl-sulfanyl)-phenyl]-ethanon als Trifluoressigsäuresalz

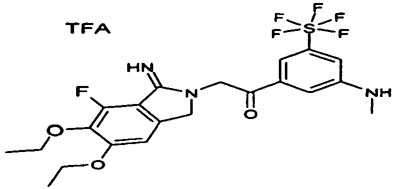

5,6-Diethoxy-7-fluor-3H-isoindol-1-ylamin (60 mg, hergestellt nach EP 1391451 oder CA 2515715) wurde in absolutem DMF (5 ml) vorgelegt und unter Rühren 2-Brom-1-(3-methylamino-5-pentafluorsulfanyl-phenyl)-ethanon (98 mg, Beispiel 3a), gelöst in DMF (1 ml), bei RT zugetropft. Nach 6 h Rühren bei 60°C wurde das Lösungsmittel im Vakuum abgezogen und der Rückstand mittels präparativer HPLC gereinigt. Die produktenthaltenden Fraktionen wurden vereinigt, vom Acetonitril befreit und gefriergetrocknet. Es wurden 36 mg der Titelverbindung erhalten.

| | |
|---|---|
| LCMS-Rt: 1,31 min | [M+H]⁺: 512,3 |

### Beispiel 10

### 6-Ethoxy-3-imino-2-{2-[3-dimethansulfonylamino-5-(pentafluorsulfanyl)-phenyl]-2-oxo-ethyl}-2,3-dihydro-1H-isoindol-5-carbonsäuremethylamid als Trifluoressigsäuresalz

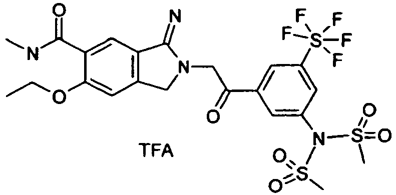

### 10a) 1-(3-Dimethansulfonylamino-5-pentafluorsulfanyl-phenyl)-ethanon

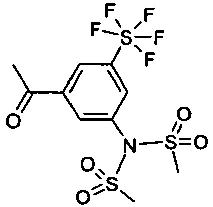

1-[3-Amino-5-(pentafluorsulfanyl)-phenyl]-ethanon-Trifluoressigsäuresalz (100 mg, Beispiel 4d) wurde in Methylenchlorid (10 ml) gelöst, Triethylamin (120 µl) zugesetzt und unter Rühren Methansulfonylchlorid (21 µl) zugegeben. Nach 2 h wurde über Nacht stehengelassen und dann weiteres Methansulfonylchlorid (20 µl) zugegeben.

Nach weiteren 2 h wurde das Lösungsmittel abgezogen und der Rückstand wurde mit Essigester und Wasser aufgenommen, mit gesättigter Natriumhydrogencarbonat-Lösung auf pH 9 gestellt und die wässrige Phase noch zweimal mit Essigester extrahiert. Die vereinigten Extrakte wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit und gefriergetrocknet. Es wurden 36 mg der Titelverbindung erhalten.
¹H-NMR (400 MHz, DMSO-d₆) [ppm]: 8,49 (1 H); 8,40 (1 H); 8,31 (1H); 3,62 (6 H); 2,71 (3 H)

### 10b) 2-Brom-1-(3-dimethansulfonylamino-5-pentafluorsulfanyl-phenyl)-ethanon

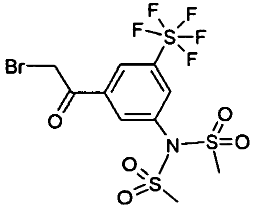

1-(3-Dimethansulfonylamino-5-pentafluorsulfanyl-phenyl)-ethanon (33 mg) wurde analog Beispiel 8b) bromiert, wobei das Reaktionsgemisch 2 h in der 2 N Schwefelsäure gerührt wurde und auf eine präparative Aufreinigung verzichtet werden konnte. Es wurden 40 mg der Titelverbindung erhalten.
¹H-NMR (400 MHz, CDCl₃) [ppm]: 8,47 (1 H); 8,09 (1 H); 7,97 (1H); 4,42 (2 H); 3,46 (6 H)

### 10c) 6-Ethoxy-3-imino-2-{2-[3-dimethansulfonylamino-5-(pentafluorsulfanyl)-phenyl]-2-oxo-ethyl}-2,3-dihydro-1H-isoindol-5-carbonsäuremethylamid als Trifluoressigsaäuresalz

3-Amino-6-ethoxy-1H-isoindol-5-carbonsäuremethylamid (9 mg, gekauft bei Chembiotek) wurde mit N-[3-(2-Brom-acetyl)-5-pentafluorsulfanyl-phenyl]-N-ethyl-2,2,2-trifluor-acetamid (19 mg) analog zu Beispiel 3b) umgesetzt. Es wurden 6 mg der gewünschten Verbindung erhalten.

| | |
|---|---|
| LCMS-Rt: 1,20 min | [M+H]⁺: 649,0 |

### Beispiel 11

### 2-{2-[3-(Acetyl-methyl-amino)-5-(pentafluorsulfanyl)-phenyl]-2-oxo-ethyl}-6-ethoxy-3-imino-2,3-dihydro-1H-isoindol-5-carbonsäuremethylamid als Trifluoressigsäuresalz

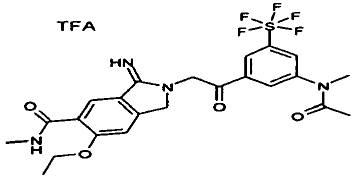

### 11a) N-[3-Acetyl-5-(pentafluorsulfanyl)-phenyl]-2,2,2-trifluor-N-methyl-acetamid

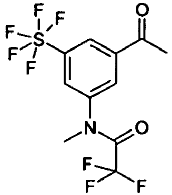

In einem Mikrowelleneinsatz wurde N-(3-Acetyl-5-pentafluorsulfanyl-phenyl)-2,2,2-trifluor-acetamid (250 mg, Beispiel 4d) in absolutem Dimethoxyethan (7,5 ml) gelöst, gepulvertes Kaliumcarbonat zugegeben und mit Jodmethan versetzt. Anschließend wurde in der Mikrowelle 40 min auf 100°C erhitzt. Nachdem weiters N-(3-Acetyl-5-pentafluorsulfanyl-phenyl)-2,2,2-trifluor-acetamid (3 x 250 mg) in beschriebener Weise umgesetzt worden war, wurden die vier Ansätze gemeinsam aufgearbeitet, wobei vom Kaliumcarbonat unter Eiskühlung in 1 N Salzsäure dekantiert wurde. Nach mehrmaligem Waschen des Kaliumcarbonat-Rückstands mit Dimethoxyethan wurde die wässrige Phase 5 Mal mit Essigester extrahiert. Die vereinigten Extrakte wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit und 5 Mal mit Essigester extrahiert. Die vereinigten Extrakte wurden über Magnesiumsulfat getrocknet, filtriert und eingeengt. Es wurden 650 mg der gewünschten Verbindung erhalten.

| | |
|---|---|
| LCMS-Rt: 1,62 min | [M+H]⁺: 372,0 |

### 11b) N-[3-(2-Brom-acetyl)-5-(pentafluorsulfanyl)-phenyl]-2,2,2-trifluor-N-methylacetamid

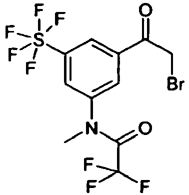

N-[3-Acetyl-5-(pentafluorsulfanyl)-phenyl]-2,2,2-trifluor-N-methyl-acetamid (650 mg) wurde analog Beispiel 8b) bromiert. Es wurden 780 mg der Titelverbindung erhalten, die ohne Aufreinigung weiter umgesetzt wurden.

| | |
|---|---|
| LCMS-Rt: 1,73 min | [M+H]⁺: 449,9 |

### 11c) 2-Brom-1-[3-methylamino-5-(pentafluorsulfanyl)-phenyl]-ethanon

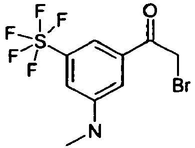

N-[3-(2-Brom-acetyl)-5-(pentafluorsulfanyl)-phenyl]-2,2,2-trifluor-N-methyl-acetamid (780 mg) wurde zur Abspaltung der Trifluoracetyl-Gruppe entsprechend Beispiel 6) umgesetzt. Das Rohprodukt wurde mittels präparativer HPLC gereinigt. Die produktenthaltenden Fraktionen wurden vereinigt, vom Acetonitril befreit, mit Natriumhydrogencarbonat basisch gestellt und 3 Mal mit Essigester extrahiert. Die vereinigten Extrakte wurden über Magnesiumsulfat getrocknet, filtriert und eingeengt. Es wurden 124 mg der gewünschten Verbindung erhalten

| | |
|---|---|
| LCMS-Rt: 1,67 min | [M+H]⁺: 353,9 |

### 11d) N-[3-(2-Brom-acetyl)-5-(pentafluorsulfanyl)-phenyl]-N-methyl-acetamid

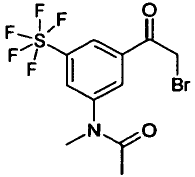

2-Brom-1-[3-methylamino-5-(pentafluorsulfanyl)-phenyl]-ethanon (100 mg) wurde in Methylenchlorid (5 ml) vorgelegt und unter Rühren Acetylbromid (21 µl) zugegeben. Nach 2 h Rühren wurde weiteres Acylbromid (21 µl) zugegeben und erneut 2 h gerührt. Nach Stehenlassen über Nacht wurde nochmals Acetylbromid (21 µl) nachgegeben. Nach weiteren 4 h wurde das Reaktionsgemisch eingeengt und der Rückstand mittels präparativer HPLC gereinigt. Die produktenthaltenden Fraktionen wurden vereinigt, vom Acetonitril befreit, mit Natriumhydrogencarbonat basisch gestellt und 3 Mal mit Essigester extrahiert. Die vereinigten Extrakte wurden über Magnesiumsulfat getrocknet, filtriert und eingeengt. Es wurden 50 mg der gewünschten Verbindung erhalten.

| | |
|---|---|
| LCMS-Rt: 1,47 min | [M+H]⁺: 396,0 |

### 11e) 2-{2-[3-(Acetyl-methyl-amino)-5-(pentafluorsulfanyl)-phenyl]-2-oxo-ethyl}-6-ethoxy-3-imino-2,3-dihydro-1H-isoindol-5-carbonsäuremethylamid als Trifluoressigsäuresatz

3-Amino-6-ethoxy-1H-isoindol-5-carbonsäure-methylamid (9 mg, gekauft bei Chembiotek) wurde mit N-[3-(2-Brom-acetyl)-5-(pentafluorsulfanyl)-phenyl]-N-methylacetamid(15 mg) analog zu Beispiel 3b) umgesetzt. Es wurden 9 mg der gewünschten Verbindung erhalten.

| | |
|---|---|
| LCMS-Rt: 1,09 min | [M+H]⁺: 549,0 |

### Beispiel 12

### N-[3-[2-(5,6-Diethoxy-7-fluor-1-imino-1,3-dihydro-isoindol-2-yl)-acetyl]-5-(pentafluorsulfanyl)-phenyl]-N-methyl-acetamid als Trifluoressigsäuresalz

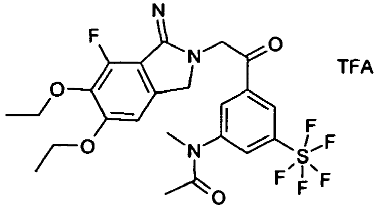

5,6-Diethoxy-7-fluor-3H-isoindol-1-ylamin (10 mg, hergestellt nach EP 1391451 oder CA 2515715) wurde in absolutem DMF (1,5 ml) vorgelegt und unter Rühren N-[3-(2-Brom-acetyl)-5-(pentafluorsulfanyl)-phenyl]-N-methyl-acetamid(16 mg, Beispiel 11d), gelöst in DMF (0,5 ml), bei RT zugetropft. Nach 3 h Rühren bei RT wurde über Nacht stehen gelassen, dann das Lösungsmittel im Vakuum abgezogen. Der Rückstand wurde mittels präparativer HPLC gereinigt. Die produktenthaltenden Fraktionen wurden vereinigt, vom Acetonitril befreit und gefriergetrocknet. Es wurden 15 mg der Titelverbindung erhalten.

| | |
|---|---|
| LCMS-Rt: 1,23 min | [M+H]⁺: 554,0 |

### Beispiel 13

### N-[3-[2-(5,6-Diethoxy-7-fluor-1-imino-1,3-dihydro-isoindol-2-yl)-acetyl]-5-(pentafluorsulfanyl)-phenyl]-isobutyramid als Trifluoressigsäuresalz

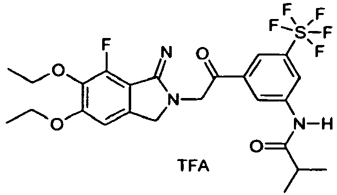

### 13a) N-[3-(2-Brom-acetyl)-5-(pentafluorsulfanyl)-phenyl]-isobutyramid

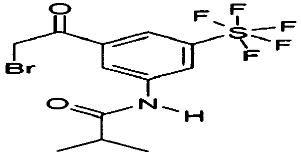

Ausgehend von 1-[3-Amino-5-(pentafluorsulfanyl)-phenyl]-ethanon-Trifluoracetat (80 mg, Beispiel 4d) wurden analog Beispiel 8a) und b) 30 mg der Titelverbindung dargestellt. Als Acylierungsmittel wurde statt Essigsäureanhydrid Isobuttersäurechlorid eingesetzt.

| | |
|---|---|
| LCMS-Rt: 1,67 min | [M+H]⁺: 410,0 |

### 13b) N-[3-[2-(5,6-Diethoxy-7-fluor-1-imino-1,3-dihydro-isoindol-2-yl)-acetyl]-5-(pentafluorsulfanyl)-phenyl]-isobutyramid als Trifluoressigsäuresalz

5,6-Diethoxy-7-fluor-3H-isoindol-1-ylamin (7 mg, hergestellt nach EP 1391451 oder CA 2515715) wurde mit N-[3-(2-Brom-acetyl)-5-(pentafluorsulfanyl)-phenyl]-isobutyramid (12 mg) entsprechend Beispiel 12 umgesetzt. Es wurden 7 mg des gewünschten Produkts erhalten.

| | |
|---|---|
| LCMS-Rt: 1,34 min | [M+H]⁺: 568,2 |

### Beispiel 14

### N-[3-[2-(5,6-Diethoxy-7-fluor-1-imino-1,3-dihydro-isoindol-2-yl)-acetyl]-5-pentafluorsulfanyl)-phenyl]-acetamid als Hydrobromid

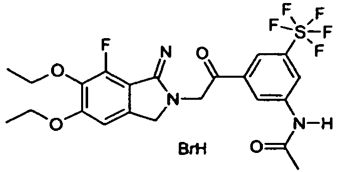

### 14a) 3-Acetylamino-N-methoxy-N-methyl-5-(pentafluorsulfanyl)-benzamid

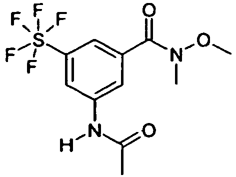

3-Amino-N-methoxy-N-methyl-5-pentafluorsulfanyl-benzamid (1,2 g, Beispiel 4b) wurde analog Beispiel 4c) statt mit Trifluoressigsäure- mit Essigsäureanhydrid umgesetzt. Es wurden 1,3 g der gewünschten Verbindung isoliert.

| | |
|---|---|
| LCMS-Rt: 1,26 min | [M+H]⁺: 349,0 |

### 14b) N-[3-Acetyl-5-(pentafluorsulfanyl)-phenyl]-acetamid

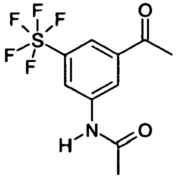

3-Acetylamino-N-methoxy-N-methyl-5-(pentafluorsulfanyl)-benzamid (1,2 g) wurde analog Beispiel 4d) umgesetzt. Es wurde eine Rohproduktreinigung durchgeführt, die über Kieselgel mit Dichlormethan-Methanol als Laufmittel erfolgte. Es wurden 859 mg der gewünschten Verbindung erhalten.

| | |
|---|---|
| LCMS-Rt: 1,34 min | [M+H]⁺: 304,0 |

### 14c) N-[3-(2-Brom-acetyl)-5-(pentafluorsulfanyl)-phenyl]-acetamid

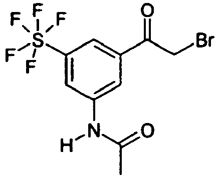

N-[3-Acetyl-5-(pentafluorsulfanyl)-phenyl]-acetamid (859 mg) wurde entsprechend Beispiel 8b) bromiert. Die Rohproduktreinigung erfolgte allerdings über Kieselgel mit Essigester/Heptan als Laufmittel. Es wurden 480 mg der gewünschten Verbindung erhalten.

| | |
|---|---|
| LCMS-Rt: 1,47 min | [M+H]⁺: 382,0 |

### 14d) N-[3-[2-(5,6-Diethoxy-7-fluor-1-imino-1,3-dihydro-isoindol-2-yl)-acetyl]-5-pentafluorsulfanyl)-phenyl]-acetamid als Hydrobromid

Bei 7°C wurde 5,6-Diethoxy-7-fluor-3H-isoindol-1-ylamin (149 mg, hergestellt nach EP 1391451 oder CA 2515715) in absolutem THF (6 ml) gelöst und unter Rühren zu einer Lösung aus N-[3-(2-Brom-acetyl)-5-(pentafluorsulfanyl)-phenyl]-acetamid (281 mg) in absolutem THF (2 ml) getropft. Dann wurde das Kältebad entfernt und 6 h bei RT gerührt. Nach Stehenlassen über Nacht wurde der gebildete Niederschlag abgesaugt, mit wenig THF gewaschen und in einem Acetonitril-Wasser-Gemisch gelöst. Nach Gefriertrocknung wurden 200 mg der Titelverbindung erhalten.

| | |
|---|---|
| LCMS-Rt: 1,28 min | [M+H]⁺: 540,2 |

### Beispiel 15

### N-[5-[2-(5,6-Diethoxy-7-fluor-1-imino-1,3-dihydro-isoindol-2-yl)-acetyl]-4-methyl-2-(pentafluorsulfanyl)-phenyl]-acetamid als Trifluoressigsäuresalz

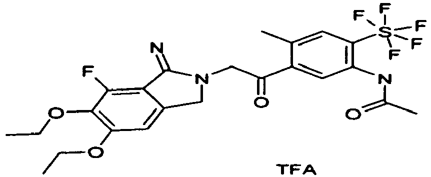

### 15a) 5-Amino-N-methoxy-2,N-dimethyl-4-(pentafluorsulfanyl)-benzamid

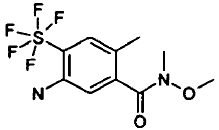

5-Amino-2-methyl-4-(pentafluorsulfanyl)-benzoesäure (500 mg, WO 2005/47239) wurde in Methylenchlorid (30 ml) gelöst und unter Rühren mit N,O-Dimethylhydroxylamin-Hydrochlorid (176 mg), 1-Propanphosphonsäureanhydrid (1,1 ml) und Trithylamin (0,25 ml) versetzt. Nach 4 h Rühren und Stehenlassen über Nacht wurde das Reaktionsgemisch eingeengt, der Rückstand mit Essigester aufgenommen und die Lösung zwei Mal mit Kaliumhydrogensulfat- und zwei Mal mit Natriumhydrogencarbonat-Lösung gewaschen. Nach Trocknen über Magnesiumsulfat wurde die organische Phase filtriert und eingeengt. Das erhaltene Rohprodukt (500 mg) konnte direkt weiter umgesetzt werden.

| | |
|---|---|
| LCMS-Rt: 1,33 min | [M+H]⁺: 321,0 |

### 15b) 5-Acetylamino-N-methoxy-2,N-dimethyl-4-(pentafluorsulfanyl)-benzamid

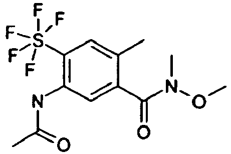

5-Amino-N-methoxy-2,N-dimethyl-4-(pentafluorsulfanyl)-benzamid (200 mg) wurde in Dichlormethan (10 ml) gelöst und dann unter Rühren Triethylamin (100 µl) und Acetylchlorid (50 µl) zugegeben. Nach 2 h wurde weiteres Acetylchlorid zugegeben (50 µl) und 4 h gerührt. Dann wurde das Reaktionsgemisch mit Wasser und gesättigter Natriumhydrogencarbonat-Lösung versetzt, die organische Phase abgetrennt und drei Mal mit Wasser gewaschen. Nach Trocknen über Magnesiumsulfat wurde die organische Phase filtriert und eingeengt. Der Rückstand wurde mittels präparativer HPLC gereinigt. Die produktenthaltenden Fraktionen wurden vereinigt, vom Acetonitril befreit, mit gesättigter Natriumhydrogencarbonat-Lösung versetzt und drei Mal mit Essigester extrahiert. Die vereinigten Extrakte wurden über Magnesiumsulfat getrocknet, filtriert und eingeengt. Es wurden 170 mg der gewünschten Verbindung erhalten.

| | |
|---|---|
| LCMS-Rt: 1,12 min | [M+H]⁺: 363,0 |

### 15c) N-[5-Acetyl-4-methyl-2-(pentfluorsutfanyl)-phenyl]-acetamid

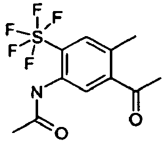

5-Acetylamino-N-methoxy-2,N-dimethyl-4-(pentafluorsulfanyl)-benzamid (170 mg) wurde analog Beispiel 4d) umgesetzt. Es wurden 130 mg Rohprodukt erhalten, das direkt in der nächsten Stufe eingesetzt wurde.

| | |
|---|---|
| LCMS-Rt: 1,22 min | [M+H]⁺: 318,0 |

### 15d) N-[5-(2-Brom-acetyl)-4-methyl-2-(pentafluorsulfanyl)-phenyl]-acetamid

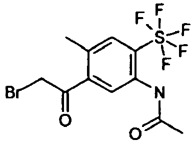

N-[5-Acetyl-4-methyl-2-(pentfluorsulfanyl)-phenyl]-acetamid (130 mg) wurde entsprechend Beispiel 8b) bromiert. Es wurden 41 mg der gewünschten Verbindung erhalten.

| | |
|---|---|
| LCMS-Rt: 1,39 min | [M+H]⁺: 395,9 |

### 15e) N-[5-[2-(5,6-Diethoxy-7-fluor-1-imino-1,3-dihydro-isoindol-2-yl)-acetyl]-4-methyl-2-(pentafluorsulfanyl)-phenyl]-acetamid als Trifluoressigsäuresalz

5,6-Diethoxy-7-fluor-3H-isoindol-1-ylamin (11 mg, hergestellt nach EP 1391451 oder CA 2515715) wurde mit N-[5-(2-Brom-acetyl)-4-methyl-2-(pentafluorsulfanyl)-phenyl]-acetamid (18 mg) entsprechend Beispiel 12 umgesetzt. Es wurden 15 mg des gewünschten Produkts erhalten.

| | |
|---|---|
| LCMS-Rt: 1,24 min | [M+H]⁺: 554,2 |

### Beispiel 16

### 1-[5-Amino-2-methyl-4-(pentafluorsulfanyl)-phenyl]-2-(5,6-diethoxy-7-fluor-1-imino-1,3-dihydro-isoindol-2-yl)-ethanon als Trifluoressigsäuresalz

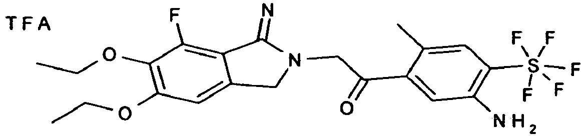

N-[5-[2-(5,6-Diethoxy-7-fluor-1-imino-1,3-dihydro-isoindol-2-yl)-acetyl]-4-methyl-2-(pentafluor-sulfanyl)-phenyl]-acetamid-Trifluoressigsäuresalz (9 mg, Beispiel 15) wurde analog Beispiel 6 umgesetzt. Zur Aufarbeitung wurde mit Wasser verdünnt und die wässrige Phase drei Mal mit Essigester extrahiert. Die vereinigten Extrakte wurden mit gesättigter Natriumhydrogencarbonat-Lösung säurefrei gewaschen, mit Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit und gefriergetrocknet. Es wurden 4 mg der Titelverbindung erhalten.

| | |
|---|---|
| LCMS-Rt: 1,38 min | [M+H]⁺: 512,2 |

### Beispiel 17

### N-{3-[2-(5,6-Diethoxy-7-fluor-1-imino-1,3-dihydro-isoindol-2-yl)-acetyl]-5-pentafluorsulfanyl-phenyl}-3,3-dimethyl-butyramid als Trifluoressigsäuresalz

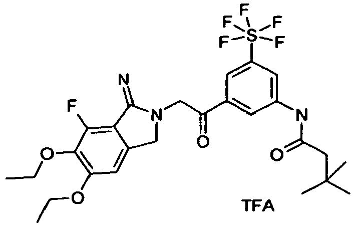

### 17a) N-[3-(2-Brom-acetyl)-5-(pentafluorsulfanyl)-phenyl]-3,3-dimethyl-butyramid

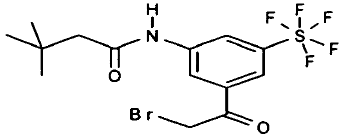

N-[3-(2-Brom-acetyl)-5-(pentafluorsulfanyl)-phenyl]-3,3-dimethyl-butyramid (100 mg) wurde ausgehend von 3-Amino-N-methoxy-N-methyl-5-pentafluorsulfanyl-benzamid (200 mg, Beispiel 4b)) analog Beispiel 14a-c) synthetisiert.

| | |
|---|---|
| LCMS-Rt: 1,82 min | [M+H]⁺: 438,0 |

### 17b) N-{3-[2-(5,6-Diethoxy-7-fluor-1-imino-1,3-dihydro-isoindol-2-yl)-acetyl]-5-pentafluorsulfanyl-phenyl}-3,3-dimethyl-butyramid als Trifluoressigsäuresalz5,6-

Diethoxy-7-fluor-3H-isoindol-1-ylamin (13 mg, hergestellt nach EP 1391451 oder CA 2515715) wurde mit N-[3-(2-Brom-acetyl)-5-(pentafluorsulfanyl)-phenyl]-3,3-dimethyl-butyramid (24 mg) entsprechend Beispiel 12 umgesetzt. Es wurden 13 mg des gewünschten Produkts erhalten.

| | |
|---|---|
| LCMS-Rt: 1,39 min | [M+H]⁺: 596,2 |

### Beispiel 18

### 1-{3-[2-(5,6-Diethoxy-7-fluor-1-imino-1,3-dihydro-isoindol-2-yl)-acetyl]-5-pentafluorsulfanyl-phenyl}-pyrrolidin-2,5-dion als Trifluoressigsäuresalz

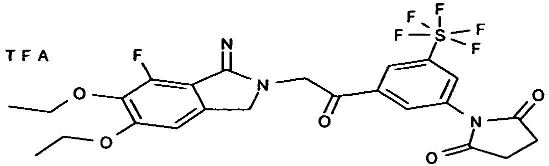

### 18a) 1-[3-Amino-5-(pentafluorsulfanyl)-phenyl]-ethanon

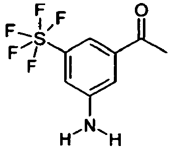

N-(3-Acetyl-5-pentafluorsulfanyl-phenyl)-2,2,2-trifluor-acetamid (3,3 g, Beispiel 4d) wurde entsprechend Beispiel 6 umgesetzt. Das erhaltene Rohprodukt wurde mit Essigester/n-Heptan über Kieselgel gereinigt. Es wurden 1,1 g des gewünschten Produkts erhalten.

| | |
|---|---|
| LCMS-Rt: 1,37 min | [M+H]⁺: 262,0 |

### 18b) 1-[3-Acetyl-5-(pentafluorsulfanyl)-phenyl]-pyrrolidin-2,5-dion dion

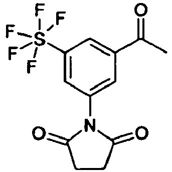

1-[3-Amino-5-(pentafluorsulfanyl)-phenyl]-ethanon (200 mg) wurde mit Bernsteinsäure (90 mg) in einem Kolben vermischt und dann Polyphosphorsäure (10 ml) dazugegeben. Es wurde unter Rühren 8 h auf 130°C erwärmt und nach Stehenlassen über Nacht weitere Bernsteinsäure (50 mg) zugegeben und weitere 4 h auf 130°C erwärmt. Nach Abkühlen wurde das Gemisch in Eiswasser eingerührt und die wässrige Lösung fünf Mal mit Essigester extrahiert. Die vereinigten Extrakte wurden mit gesättigter Natriumhydrogencarbonat-Lösung gewaschen, über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit, mit gesättigter Natriumhydrogencarbonat-Lösung versetzt und drei Mal mit Essigester extrahiert. Die vereinigten Extrakte wurden über Magnesiumsulfat getrocknet, filtriert und eingeengt. Es wurden 42 mg der gewünschten Verbindung erhalten
¹H-NMR (400 MHz, CDCl₃) [ppm]: 8,33 (1 H); 8,09 (1 H); 7,97 (1H), 2,97 (4 H); 2,67 (3 H)

### 18c) 1-[3-(2-Brom-acetyl)-5-(pentafluorsulfanyl)-phenyl]-pyrrolidin-2,5-dion

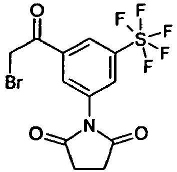

1-[3-Acetyl-5-(pentafluorsulfanyl)-phenyl]-pyrrolidin-2,5-dion (42 mg) wurde analog Beispiel 8b) bromiert. Es wurden 19 mg des gewünschten Bromids erhalten.
¹H-NMR (400 MHz, DMSO-d₆) [ppm]: 8,47 (1 H); 8,18 (2 H); 5,04 (2 H); 2,80 (4 H)

### 18d) 1-{3-[2-(5,6-Diethoxy-7-fluor-1-imino-1,3-dihydro-isoindol-2-yl)-acetyl]-5-pentafluorsulfanyl-phenyl}-pyrrolidin-2,5-dion als Trifluoressigsäuresalz

5,6-Diethoxy-7-fluor-3H-isoindol-1-ylamin (9 mg, hergestellt nach EP 1391451 oder CA 2515715) wurde mit 1-[3-(2-Brom-acetyl)-5-(pentafluorsulfanyl)-phenyl]-pyrrolidin-2,5-dion (18 mg) entsprechend Beispiel 12 umgesetzt. Es wurden 9 mg des gewünschten Produkts erhalten.

| | |
|---|---|
| LCMS-Rt: 1,26 min | [M+H]⁺: 580,2 |

### Beispiel 19

### 1-[2-Amino-4-(pentafluorsulfanyl)-phenyl]-2-(5,6-diethoxy-7-fluor-1-imino-1,3-dihydro-isoindol-2-yl)-ethanon als Trifluoressigsäuresalz

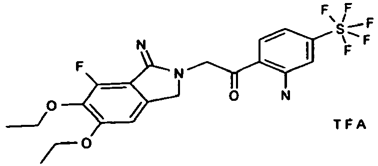

### 19a) 2-Amino-4-(pentafluorsulfanyl)-benzoesäure

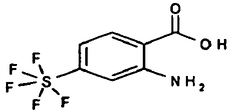

4-(Pentafluorsulfanyl)-benzoesäure (4 g) wurde analog Beispiel 2a) zunächst nitriert und anschließend reduziert. Es wurden 2,1 g der gewünschten Verbindung erhalten.

| | |
|---|---|
| LCMS-Rt: 1,35 min | [M+H]⁺: 264,0 |

### 19b) 2-Amino-N-methoxy-N-methyl-4-(pentafluorsulfanyl)-benzamid

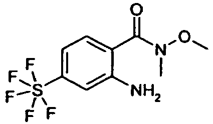

2-Amino-4-(pentafluorsulfanyl)-benzoesäure (2,0 g) wurde analog Beispiel 15a) mit N,O-Dimethylhydroxylamin-Hydrochlorid umgesetzt. Es wurden 2,3 g des gewünschten Produkts erhalten.

| | |
|---|---|
| LCMS-Rt: 1,33 min | [M+H]⁺: 307,0 |

### 19c) N-Methoxy-N-methyl-4-(pentafluorsulfanyl)-2-(2,2,2-trifluor-acetylamino)-benzamid

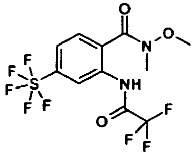

2-Amino-N-methoxy-N-methyl-4-(pentafluorsulfanyl)-benzamid (2,3 g) wurde analog Beispiel 4c) mit Trifluoressigsäureanhydrid umgesetzt. Es wurden 2,7 g des gewünschten Produkts erhalten.

| | |
|---|---|
| LCMS-Rt: 1,54 min | [M+H]⁺: 403,0 |

### 19d) N-[2-Acetyl-5-(pentaflursulfanyl)-phenyl]-2.2,2-trifluor-acetamid und 1-[2-Amino-4-(pentafluorsulfanyl)-phenyl]-ethanon

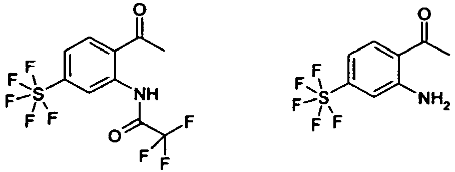

N-Methoxy-N-methyl-4-(pentafluorsulfanyl)-2-(2,2,2-trifluor-acetylamino)-benzamid (2,7 g) wurde analog Beispiel 4d) umgesetzt, wobei allerdings die Essigesterphase vor dem Trocknen mit Magnesiumsulfat mit gesättigter Natriumhydrogencarbonat-Lösung säurefrei gewaschen wurde und der Rückstand über Kieselgel mit Dichlormethan als Laufmittel gereinigt wurde. Es wurden 1,45 g N-[2-Acetyl-5-(pentaflursulfanyl)-phenyl]-2,2,2-trifluor-acetamid

| | |
|---|---|
| LCMS-Rt: 1,77 min | [M+H]⁺: 358,0 |

und
310 mg 1-[2-Amino-4-(pentafluorsulfanyl)-phenyl]-ethanon erhalten.

| | |
|---|---|
| LCMS-Rt: 1,52 min | [M+H]⁺: 262,0 |

### 19e) 1-[2-Amino-4-(pentafluorsulfanyl)-phenyl]-2-brom-ethanon

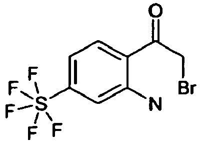

N-[2-Acetyl-5-(pentaflursulfanyl)-phenyl]-2,2,2-trifluoro-acetamid (200 mg) wurde entsprechend Beispiel 8b) bromiert. Es wurden 28 mg der gewünschten Verbindung erhalten. Alternativ konnte 1-[2-Amino-4-(pentafluorsulfanyl)-phenyl]-2-brom-ethanon auch wie in Beispiel 20b) beschrieben dargestellt werden.

| | |
|---|---|
| LCMS-Rt: 1,61 min | [M+H]⁺: 339,9 |

### 19f) 1-[2-Amino-4-(pentafluorsulfanyl)-phenyl]-2-(5,6-diethoxy-7-fluor-1-imino-1,3-dihydro-isoindol-2-yl)-ethanon als Trifluoressigsäuresalz

5,6-Diethoxy-7-fluor-3H-isoindol-1-ylamin (19 mg, hergestellt nach EP 1391451 oder CA 2515715) wurde mit 1-[2-Amino-4-(pentafluorsulfanyl)-phenyl]-2-brom-ethanon (27 mg) entsprechend Beispiel 12 umgesetzt. Es wurden 21 mg des gewünschten Produkts erhalten.

| | |
|---|---|
| LCMS-Rt: 1,26 min | [M+H]⁺: 498,0 |

### Beispiel 20

### 1-[2-Amino-5-brom-4-(pentafluorsulfanyl)-phenyl]-2-(5,6-diethoxy-7-fluor-1-imino-1,3-dihydro-isoindol-2-yl)-ethanon als Trifluoressigsäuresalz

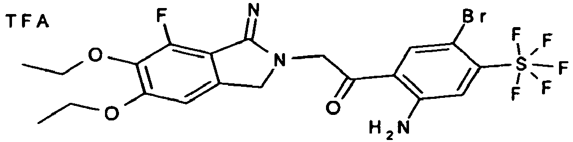

20a) N-[2-Acetyl-5-(pentafluorsulfanyl)-phenyl]-acetamid

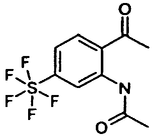

1-[2-Amino-4-(pentafluorsulfanyl)-phenyl]-ethanon (150 mg, Beispiel 19d) wurde in Methylenchlorid (5 ml) gelöst. Nach Zugabe von Triethylamin (95 µl) und Acetylchlorid (51 µl) wurde 4 h bei RT gerührt. Dann wurde Methylenchlorid, Wasser und gesättigte Natriumhydrogencarbonat-Lösung zugesetzt. Die organische Phase wurde abgetrennt, drei Mal mit Wasser gewaschen, über Magnesiumsulfat getrocknet, filtriert und eingeengt. Es wurden 165 mg des gewünschten Produkts erhalten.

| | |
|---|---|
| LCMS-Rt: 1,45 min | [M+H]⁺: 304,0 |

20b) 1-[2-Amino-5-brom-4-(pentafluorsulfanyl)-phenyl]-2-brom-ethanon und 1-[2-Amino-4-(pentafluorsulfanyl)-phenyl]-2-bromethanon

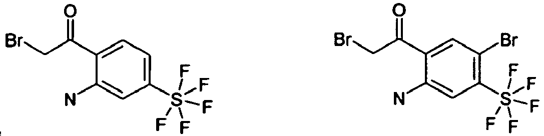

N-[2-Acetyl-5-(pentafluorsulfanyl)-phenyl]-acetamid (80 mg) wurde entsprechend Beispiel 8b) umgesetzt. Nach Aufarbeitung und Chromatographie wurden 21 mg 1-[2-Amino-4-(pentafluorsulfanyl)-phenyl]-2-brom-ethanon

| | |
|---|---|
| LCMS-Rt: 1,61 min | [M+H]⁺: 339,9 |

und 24 mg 1-[2-Amino-5-brom-4-(pentafluorsulfanyl)-phenyl]-2-brom-ethanon erhalten.

| | |
|---|---|
| LCMS-Rt: 1,73 min | [M+H]⁺: 417,8 |

### 20c) 1-[2-Amino-5-brom-4-(pentafluorsulfanyl)-phenyl]-2-(5,6-diethoxy-7-fluor-1-imino-1,3-dihydro-isoindol-2-yl)-ethanon als Trifluoressigsäuresalz

5,6-Diethoxy-7-fluor-3H-isoindol-1-ylamin (13 mg, hergestellt nach EP 1391451 oder CA 2515715) wurde mit 1-[2-Amino-5-brom-4-(pentafluorsulfanyl)-phenyl]-2-bromethanon (23 mg) entsprechend Beispiel 12 umgesetzt. Es wurden 10 mg des gewünschten Produkts erhalten.

| | |
|---|---|
| LCMS-Rt: 1,31 min | [M+H]⁺: 576,0 |

### Beispiel 21

### 2-(5,6-Diethoxy-7-fluor-1-imino-1,3-dihydro-isoindol-2-yl)-1-[3-methoxy-5-(pentafluorsulfanyl)-phenyl]-ethanon als Trifluoressigsäuresalz

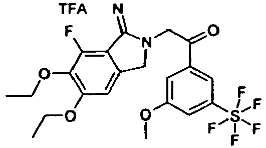

### 21a) 3-Hydroxy-5-(pentafluorsulfanyl)-benzoesäure

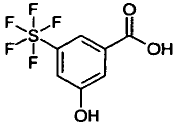

3-Amino-5-pentafluorsulfanyl-benzoesäure (1,5 g Beispiel 2a)) wurden in 35%iger Schwefelsäure (45 ml) gelöst, auf -5°C abgekühlt und innerhalb von 10 min eine Lösung aus Natriumnitrit (387 mg) in Wasser (40 ml) zugetropft. Nach 40 min wurde das Kältebad entfernt und das Gemisch auf 100°C erhitzt. Nach 4 h wurde abgekühlt und die Lösung dekantiert. Die klare, saure Lösung wurde 5 Mal mit Essigester extrahiert. Die vereinigten Extrakte wurden über Magnesiumsulfat getrocknet, filtriert und eingeengt. Das Rohprodukt wurde aus Essigester/Heptan umkristallisiert. Es wurde 1 g der gewünschten Verbindung erhalten. ¹H-NMR (400 MHz, DMSO-d₆) [ppm]: 10,72 (1 H); 7,71 (1 H); 7,57 (1 H); 7,46 (1H);

### 21b) 3-Methoxy-5-(pentafluorsulfanyl)-benzoesäure-methylester

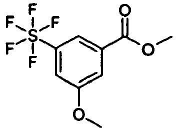

3-Hydroxy-5-(pentafluorsulfanyl)-benzoesäure (84 mg) wurde in absolutem DMF (5 ml) gelöst. Unter Rühren wurde dann Jodmethan (100 µl) zugegeben, gefolgt von feingepulvertem Kaliumcarbonat (175 mg). Nach 3 Stunden Rühren bei 40°C wurde das Gemisch abgekühlt und mit Wasser versetzt (20 ml). Das Gemisch wurde dann 4 Mal mit Ether (10 ml) extrahiert. Die vereinigten Extrakte wurden je ein Mal mit 1 N Natronlauge (10 ml) und Wasser (10 ml) gewaschen, über Magnesiumsulfat getrocknet, filtriert und eingeengt. Es wurden 81 mg der gewünschten Verbindung erhalten.

| | |
|---|---|
| LCMS-Rt: 1,71 min | [M+H]⁺: 293,0 |

### 21c) 3,N-Dimethoxy-N-methyl-5-(pentafluorsulfanyl)-benzamid

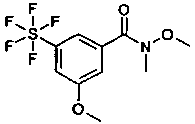

3-Methoxy-5-(pentafluorsulfanyl)-benzoesäure-methylester (81 mg) wurde in absolutem THF (5 ml) gelöst und N,O-Dimethylhydroxylamin-Hydrochlorid (42 mg) zugegeben. Dann wurde auf -15°C abgekühlt und Isopropylmagnesiumbromid-Lösung (0,21 ml, 2 M in THF) zugetropft. Nach 20 min wurde das Kältebad entfernt und 1 h bei RT gerührt. Zur Vervollständigung der Reaktion wurde noch 2 Mal auf -15°C abgekühlt, Isopropylmagnesiumbromid-Lösung (jeweils 0,1 ml) zugetropft und 1 h bei RT gerührt. Dann wurde Ammoniumchlorid-Lösung zugegeben (10 ml) und die wässrige Phase 3 Mal mit Essigester extrahiert. Die vereinigten Extrakte wurden über Magnesiumsulfat getrocknet, filtriert und eingeengt. Es wurden 70 mg der gewünschten Verbindung erhalten.

| | |
|---|---|
| LCMS-Rt: 1,49 min | [M+H]⁺: 322,0 |

### 21d) 1-[3-Methoxy-5-(pentafluorsulfanyl)-phenyl]-ethanon

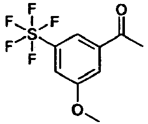

3,N-Dimethoxy-N-methyl-5-(pentafluorsulfanyl)-benzamid (70 mg) wurde in absolutem THF gelöst und bei 0°C unter Rühren Methylmagnesiumbromid (185 µl, 2 M in Diethylether) zugetropft. Nach Zugabe wurde das Eisbad entfernt und 2 h bei RT gerührt. Unter Kühlung wurde dann 1 N Salzsäure zugetropft, gefolgt von Wasser und Essigester. Die organische Phase wurde abgetrennt und die wässrige noch 2 Mal mit Essigester extrahiert. Die vereinigten Extrakte wurden über Magnesiumsulfat getrocknet, filtriert und eingeengt. Der Rückstand wurde mittels präparativer HPLC gereinigt. Die produktenthaltenden Fraktionen wurden vereinigt, vom Acetonitril befreit und drei Mal mit Essigester extrahiert. Die vereinigten Extrakte wurden über Magnesiumsulfat getrocknet, filtriert und eingeengt. Es wurden 34 mg der gewünschten Verbindung erhalten.

| | |
|---|---|
| LCMS-Rt: 1,60 min | [M+H]⁺: 277,0 |

### 21e) 2-Brom-1-[3-methoxy-5-(pentafluorsulfanyl)-phenyl]-ethanon

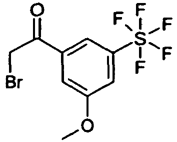

1-[3-Methoxy-5-(pentafluorsulfanyl)-phenyl]-ethanon (34 mg) wurde in absolutem Methanol (3 ml) gelöst. Dazu wurden unter Rühren DL-Camphersulfonsäure (0,6 mg) und Trimethylorthoformat (40 mg) gegeben. Nach 4 h Rühren wurde über Nacht stehengelassen und dann unter Rühren absolutes THF (3 ml) und Phenyltrimethylammoniumtribromid (40 mg) zugegeben. Nach 2 h Rühren bei RT wurde 3 h bei 50°C gerührt, übers Wochenende stehengelassen und nach Zugabe weiteren Phenyltrimethylammoniumtribromid (20 mg) nochmals 5 h bei 50°C gerührt. Das Reaktionsgemisch wurde eingeengt, der Rückstand mit Acetonitril aufgenommen und unter Rühren mit 5 N Schwefelsäure versetzt. Nach 2 h Rühren wurde das Gemisch eingeengt und der Rückstand wurde mittels präparativer HPLC gereinigt. Die produktenthaltenden Fraktionen wurden vereinigt, vom Acetonitril befreit, mit gesättigter Natriumhydrogencarbonat-Lösung versetzt und drei Mal mit Essigester extrahiert. Die vereinigten Extrakte wurden über Magnesiumsulfat getrocknet, filtriert und eingeengt. Es wurden 15 mg der gewünschten Verbindung erhalten.

| | |
|---|---|
| LCMS-Rt: 1,71 min | [M+H]⁺: 354,9 |

21f) 2-(5,6-Diethoxy-7-fluor-1-imino-1,3-dihydro-isoindol-2-yl)-1-[3-methoxy-5-(pentafluorsulfanyl)-phenyl]-ethanon als Trifluoressigsäuresalz 5,6-Diethoxy-7-fluor-3H-isoindol-1-ylamin (9 mg, hergestellt nach EP 1391451 oder CA 2515715) wurde mit 2-Brom-1-[3-methoxy-5-(pentafluorsulfanyl)-phenyl]-ethanon (15 mg) entsprechend Beispiel 12 umgesetzt. Es wurden 8 mg des gewünschten Produkts erhalten.

| | |
|---|---|
| LCMS-Rt: 1,35 min | [M+H]⁺: 513,1 |

### Beispel 22

### Cyclobutancarbonsäure [3-[2-(5,6-diethoxy-7-fluor-1-imino-1,3-dihydro-isoindol-2-yl)-acetyl]-5-(pentafluorsulfanyl)-phenyl]-amid als Hydrochlorid

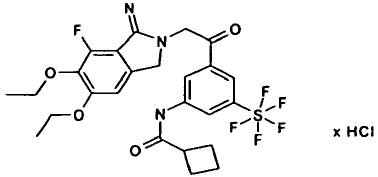

### 22a) 1-[3-Amino-5-(pentafluorsulfanyl)-phenyl]-ethanon

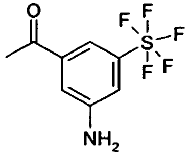

N-(3-Acetyl-5-pentafluorsulfanyl-phenyl)-2,2,2-trifluor-acetamid (4g, Herstellung siehe Beispiel 4d) wurde in 6 ml Wasser und 12 ml Acetonitril suspendiert und zu dieser Suspension 6 ml konzentrierte Schwefelsäure getropft. Nach 4 h Rühren bei 75 °C und Stehenlassen bei RT über Nacht wurden zur vollständigen Umsetzung nochmals 2 ml konzentrierte Schwefelsäure zugegeben und das Gemisch 3 h bei 75 °C erhitzt. Das Reaktionsgemisch wurde auf Eiswasser gegossen und mit Essigester extrahiert. Die Essigesterphase wurde mit gesättigter Natriumhydrogencarbonatlösung neutral gewaschen und über Magnesiumsulfat getrocknet, filtriert und eingeengt. Das Rohprodukt wurde mittels Chromatographie an Kieselgel aufgereinigt. Die produktenthaltenden Fraktionen wurden vereinigt und das Lösungsmittel (Heptan /Essigester) im Vakuum entfernt. Es wurden 2,53 g 1-[3-Amino-5-(pentafluorsulfanyl)-phenyl]-ethanon erhalten.

| | |
|---|---|
| LCMS-Rt: 1,32 min | [M+H]⁺: 262,0 |

### 22b) N-[3-Acetyl-5-(pentafluorsulfanyl)-phenyl]-cyclobutylamid

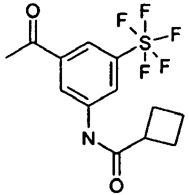

1-[3-Amino-5-(pentafluorsulfanyl)-phenyl]-ethanon (2 g) wurde in Methylenchlorid (30 ml) gelöst und Triethylamin (2,13 ml) zugesetzt und anschließend unter Rühren Cyclobutan-carbonsäurechlorid (1,362 g) zugegeben. Nach 2 h Rühren wurden das Reaktionsgemisch mit Wasser versetzt und die Phasen getrennt. Die wässrige Phase wurde mit Methylenchlorid extrahiert, die vereinigten Extrakte über Magnesiumsulfat getrocknet, filtriert und eingeengt. Das Rohprodukt wurde mittels Chromatographie an Kieselgel aufgereinigt. Die produktenthaltenden Fraktionen wurden vereinigt und das Lösungsmittel (Heptan / Essigester) im Vakuum entfernt. Es wurden 2,31 g N-[3-Acetyl-5-(pentafluorsulfanyl)-phenyl]-cyclobutylamid erhalten.

| | |
|---|---|
| LCMS-Rt: 1,53 min | [M+H]⁺: 344,06 |

### 22c) N-[3-(2-Brom-acetyl)-5-pentafluorsulfanyl-phenyl]-cyclobutylamid

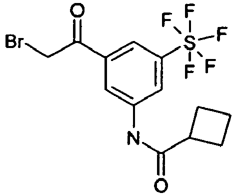

N-[3-Acetyl-5-(pentafluorsulfanyl)-phenyl]-cyclobutylamid (2,3 g) wurde in einem Gemisch aus Methanol (30 ml) und THF (30 ml) gelöst und unter Rühren Phenyl-trimethyl-tribromid (3,778 g) zugegeben. Nach 5 h Rühren bei RT wurde das Reaktionsgemisch 2 h auf 50°C erwärmt und bei RT über Nacht stehen gelassen. Das Gemisch wurde auf eine wässrige Lösung von Zitronen-säure gegossen und nach 60 min. Rühren 2 Mal mit Essigester extrahiert. Die vereinigten Extrakte wurden über Magnesiumsulfat getrocknet, filtriert und eingeengt. Der Rückstand wurde mittels Chromatographie an Kieselgel gereinigt. Die produktenthaltenden Fraktionen wurden vereinigt und das Lösungsmittel (Heptan/Essigester) im Vakuum entfernt. Der Rückstand wurde in Methylenchlorid gelöst, das Lösungsmittel im Vakuum und Hochvakuum entfernt. Es wurden 1,7 g der gewünschten Verbindung erhalten.

| | |
|---|---|
| LCMS-Rt: 1,68 min | [M+H]⁺: 421,9 |

### 22d) Cyclobutancarbonsäure [3-[2-(5,6-diethoxy-7-fluor-1-imino-1,3-dihydro-isoindol-2-yl)-acetyl]-5-(pentafluorsulfanyl)-phenyl]-amid als Hydrochlorid

5,6-Diethoxy-7-fluor-3H-isoindol-1-ylamin (150 mg, hergestellt nach EP 1391451 oder CA 2515715) und N-[3-(2-Brom-acetyl)-5-pentafluorsulfanyl-phenyl]-cyclobutylamid (292 mg) wurden in absolutem DMF (5 ml) 4 h bei RT gerührt und das Reaktionsgemisch über Nacht stehen gelassen. Das Lösungsmittel wurde im Vakuum entfernt und der Rückstand mittels präparativer HPLC gereinigt. Die produktenthaltenden Fraktionen wurden vereinigt, vom Acetonitril befreit und gefriergetrocknet. Zur Überführung ins Hydrochlorid wurde der Rückstand mit 2 Äquivalenten 1 N Salzsäure gefriergetrocknet. Es wurden 110 mg der Titelverbindung erhalten.

| | |
|---|---|
| LCMS-Rt: 1,32 min | [M+H]⁺: 580,2 |

### Beispiel 23

### Cyclopropancarbonsäure-[3-[2-(5,6-diethoxy-7-fluor-1-imino-1,3-dihydro-isoindol-2-yl)-acetyl]-5-(pentafluorsulfanyl)-phenyl]-amid als Hydrochlorid

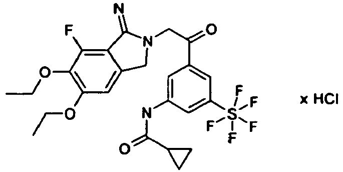

### 23a) N-[3-Acetyl-5-(pentafluorsulfanyl)-phenyl]-cyclopropylamid

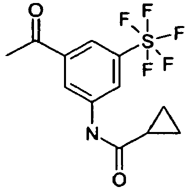

1-[3-Amino-5-(pentafluorsulfanyl)-phenyl]-ethanon (2 g, siehe Beispiel 22a) wurde in Methylenchlorid (30 ml) gelöst und Triethylamin (2,13 ml) zugesetzt und anschließend unter Rühren Cyclopropan-carbonsäurechlorid (1,2 g) zugegeben. Nach 2 h Rühren wurde das Reaktionsgemisch mit Wasser versetzt und die Phasen getrennt. Die wässrige Phase wurde mit Methylenchlorid extrahiert, die vereinigten Extrakte über Magnesiumsulfat getrocknet, filtriert und eingeengt. Das Rohprodukt wurde mittels Chromatographie an Kieselgel aufgereinigt. Die produktenthaltenden Fraktionen wurden vereinigt und das Lösungsmittel (Heptan / Essigester) im Vakuum entfernt. Es wurden 2,29 g N-[3-Acetyl-5-(pentafluorsulfanyl)-phenyl]-cyclopropylamid erhalten.

| | |
|---|---|
| LCMS-Rt: 1,51 min | [M+H]⁺: 330,1 |

### 23b) N-[3-(2-Brom-acetyl)-5-pentafluorsulfanyl-phenyl]-cyclobutylamid

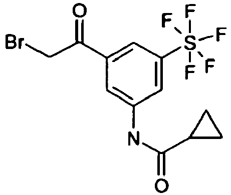

N-[3-Acetyl-5-(pentafluorsulfanyl)-phenyl]-cyclopropylamid (2,29 g) wurde in einem Gemisch aus Methanol (30 ml) und THF (30 ml) gelöst und unter Rühren Phenyl-trimethyl-tribromid (3,921 g) zugegeben. Nach 2 h Rühren bei RT wurde das Reaktionsgemisch 2 h auf 50°C erwärmt und bei RT über Nacht stehen gelassen. Das Gemisch wurde auf eine wässrige Lösung von Zitronen- säure gegossen und nach 60 min. Rühren 2 Mal mit Essigester extrahiert. Die vereinigten Extrakte wurden über Magnesiumsulfat getrocknet, filtriert und eingeengt. Der Rückstand wurde mittels Chromatographie an Kieselgel gereinigt. Die produktenthaltenden Fraktionen wurden vereinigt und das Lösungsmittel (Heptan/Essigester) im Vakuum entfernt. Der Rückstand wurde in Methylenchlorid gelöst, das Lösungsmittel im Vakuum entfernt, der so erhaltene Rückstand in 100 ml Acetonitril gelöst, mit 30 ml 2N Schwefelsäure versetzt und über Nacht bei RT stehen gelassen. Das Reaktionsgemisch wurde mit Wasser verdünnt und mit Essigester extrahiert. Die organische Phase wurde mit gesättigter Natriumhydrogencarbonat-Lösung gewaschen, über Magnesiumsulfat getrocknet, filtriert und eingeengt. Der Rückstand wurde mittels Chromatographie an Kieselgel gereinigt. Die produktenthaltenden Fraktionen wurden vereinigt und das Lösungsmittel (Heptan/Essigester) im Vakuum entfernt. Es wurden 888 mg der gewünschten Verbindung erhalten.

| | |
|---|---|
| LCMS-Rt: 1,59 min | [M+H]⁺: 407,9 |

### 23c) Cyclopropancarbonsäure [3-[2-(5,6-diethoxy-7-fluor-1-imino-1,3-dihydro-isoindol-2-yl)-acetyl]-5-(pentafluorsulfanyl)-phenyl]-amid als Hydrochlorid

5,6-Diethoxy-7-fluor-3H-isoindol-1-ylamin (150 mg, hergestellt nach EP 1391451 oder CA 2515715) und N-[3-(2-Brom-acetyl)-5-pentafluorsulfanyl-phenyl]-cyclopropylamid (283 mg) wurden in absolutem DMF (5 ml) 4 h bei RT gerührt und das Reaktionsgemisch über Nacht stehen gelassen. Das Lösungsmittel wurde im Vakuum entfernt und der Rückstand durch 2-malige präparativer HPLC und anschließende Chromatographie über Kieselgel mit Methylenchlorid/Ethanol gereinigt. Die produktenthaltenden Fraktionen wurden vereinigt, vom Lösungsmittel befreit und gefriergetrocknet. Zur Überführung ins Hydrochlorid wurde der Rückstand mit 2 Äquivalenten 1 N Salzsäure gefriergetrocknet. Es wurden 57 mg der Titelverbindung erhalten.

| | |
|---|---|
| LCMS-Rt: 1,26 min | [M+H]⁺: 566,1 |

### Beispiel 24

### 2-(5,6-Diethoxy-7-fluor-1-imino-1,3-dihydro-isoindol-2-yl)-1-[3-ethoxy-5-(pentafluorsulfanyl)-phenyl]-ethanon als Hydrobromid

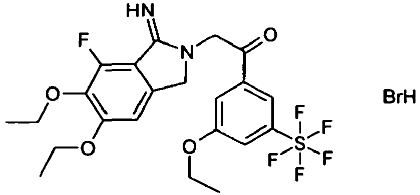

### 24a) 3-Ethoxy-5-(pentafluorsulfanyl)-benzoesäure-ethylester

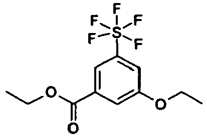

3-Hydroxy-5-(pentafluorsulfanyl)-benzoesäure (4,76 g; Beispiel 21a) wurde analog Beispiel 21b) mit Ethyljodid (7,27 ml) in Gegenwart von Kaliumcarbonat umgesetzt. Es wurden 4,8 g der Titelverbindung erhalten.

| | |
|---|---|
| LCMS-Rt: 1,97 min | [M+H]⁺: 321,0 |

### 24b) 3-Ethoxy-N-methoxy-N-methyl-5-(pentafluorsulfanyl)-benzamid

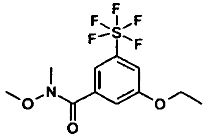

3-Ethoxy-5-(pentafluorsulfanyl)-benzoesäure-ethylester (4,8 g) wurde in THF (150 ml) gelöst und mit N,O-Dimethylhydroxylamin-Hydrochlorid (2,20 g) versetzt. Unter Argon wurde auf ca -15°C gekühlt und innerhalb von 10 min Isopropylmagnesiumchlorid (22,5 ml, 2 M in THF) zugetropft. Nach 20 min Rühren bei -15°C wurde das Kältebad entfernt und noch 1 h bei RT gerührt. Dann wurde mit gesättigter Ammoniumchlorid-Lösung versetzt und das Gemisch dreimal mit EE extrahiert. Die vereinigten organischen Phasen wurden über Magnesiumsulfat getrocknet, filtriert und eingeengt. Der Rückstand wurde über Kieselgel (120 g Kartusche, n-Heptan/MtB-Ether-Gradient von 20-100% in 60 min) aufgereinigt. Es wurden 2,80 g der Titelverbindung erhalten.

| | |
|---|---|
| LCMS-Rt: 1,60 min | [M+H]⁺: 336,0 |

### 24c) 1-[3-Ethoxy-5-(pentafluorsulfanyl)-phenyl]-ethanon

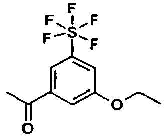

3-Ethoxy-N-methoxy-N-methyl-5-(pentafluorsulfanyl)-benzamid (2,6 g) wurde in THF (60 ml) gelöst. Nach Kühlen auf 0°C wurde Methylmagnesiumbromid (6,46 ml, 3 M in Ether) zugetropft. Danach wurde das Kältebad entfernt und bei RT weiter gerührt. Nach 1 h wurde mit 1 N Salzsäure sauer gestellt, mit Wasser versetzt und zweimal mit EE ausgeschüttelt. Die EE-Phasen wurden über Magnesiumsulfat getrocknet, filtriert und eingeengt. Es wurden 2,25 g der Titelverbindung erhalten.

| | |
|---|---|
| LCMS-Rt: 1,72 min | [M+H)⁺:291,0 |

### 24d) 2-Brom-1-[3-ethoxy-5-(pentafluorsulfanyl)-phenyl]-ethanon

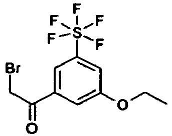

1-[3-Ethoxy-5-(pentafluorsulfanyl)-phenyl]-ethanon (2,07 g) wurde in THF (80 ml) gelöst und unter Rühren mit Phenyltrimethylammoniumtribromid (2,90 g) versetzt. Nach 3 h Rühren bei RT wurde mit DCM verdünnt und einmal mit 5%iger Natriumthiosulfat-Lösung gewaschen. Die DCM-Phase wurde über Magnesiumsulfat getrocknet, filtriert und eingeengt. Der Rückstand wurde über Kieselgel (120 g Kartusche, n-Heptan/MtB-Ether-Gradient von 0-20% in 60 min) aufgereinigt. Es wurden 2,07 g der Titelverbindung erhalten.

| | |
|---|---|
| LCMS-Rt: 1,83 min | [M+H]⁺: 368,9 |

24e) 2-(5,6-Diethoxy-7-fluor-1-imino-1,3-dihydro-isoindol-2-yl)-1-[3-ethoxy-5-(pentafluorsulfanyl)-phenyl]-ethanon als Hydrobromid 5,6-Diethoxy-7-fluor-3H-isoindol-1-ylamin (30 mg, hergestellt nach EP 1391451 oder CA 2515715) wurde in THF (5 ml) suspendiert. Nach 5 min Rühren bei RT wurde 2-Brom-1-[3-ethoxy-5-(pentafluorsulfanyl)-phenyl]-ethanon (46 mg, gelöst in 5 ml THF) zugetropft. Nach 7 h Rühren bei RT wurde über Nacht stehen gelassen und dann der Niederschlag abgesaugt. Nach Trocknen im Hochvakuum wurden 29 mg der Titelverbindung erhalten.
Präparative HPLC-Reinigung der Mutterlauge lieferte nach Gefriertrocknung weitere 15 mg in Form des Trifluoressigsäuresalzes.

| | |
|---|---|
| LCMS-Rt: 1,33 min | [M+H]⁺: 527,2 |

### Beispiel 25

### 6-Ethoxy-3-imino-2-{2-[3-methoxy-5-(pentafluorsulfanyl)-phenyl]-2-oxo-ethyl}-2,3-dihydro-1H-isoindol-5-carbonsäure-methylamid als Trifluoressigsäuresalz

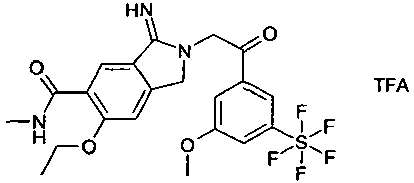

3-Amino-6-ethoxy-1H-isoindol-5-carbonsäuremethylamid (20 mg, gekauft bei Chembiotek) wurden mit 2-Brom-1-[3-methoxy-5-(pentafluorsulfanyl)-phenyl]-ethanon (31 mg, Beispiel 21e) analog Beispiel 12) umgesetzt, aufgearbeitet und gereinigt. Es wurden 25 mg der Titelverbindung erhalten.

| | |
|---|---|
| LCMS-Rt: 1,19 min | [M+H]⁺: 508,0 |

### Beispiel 26

### 1-[3-Brom-5-(pentafluorsulfanyl)-phenyl]-2-(5,6-diethoxy-7-fluor-1-imino-1,3-dihydro-isoindol-2-yl)-ethanon als Trifluoressigsäuresalz

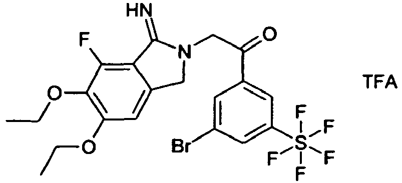

### 26a) 3-Brom-5-(pentafluorsulfanyl)-benzoesäure

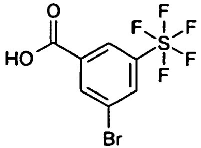

3-Amino-5-(pentafluorsulfanyl)-benzoesäure (1,1 g; Beispiel 2a) wurde in 24%iger Bromwasserstoff-Lösung (40 ml) gelöst. Unter Rühren und Kühlung wurde Natriumnitrit-Lösung (290 mg in 20 ml Wasser gelöst) zugetropft, wobei die Temperatur 5°C nicht übersteigen sollte. Nach vollständiger Bildung des Diazoniumsalzes wurde Kupfer(I)bromid-Lösung (720 mg in 15 ml 48 %iger Bromwasserstoffsäure gelöst) unter Rühren auf 0°C abgekühlt und obige Diazoniumsalz-Lösung langsam zugetropft. Dann wurde das Kältebad entfernt und 3 h bei RT gerührt. Danach wurde das Reaktionsgemisch mit Wasser verdünnt und mit EE mehrmals extrahiert. Die vereinigten EE-Phasen wurden über Magnesiumsulfat getrocknet, filtriert und eingeengt. Der Rückstand wurde in 500 ml EE gelöst und über eine Glasfritte filtriert, welche mit einer 10 cm Kieselgelschicht gefüllt war. Die Filterschicht wurde gut mit EE gewaschen. Das klare Filtrat wurde eingeengt, und Lösungsmittelreste am Hochvakuum abgezogen. Es wurden 800 mg der Titelverbindung erhalten.
Weiteres Produkt wurde erhalten, indem das Kieselgel mit 550 ml eines DCM/Methanol-Gemisches (10: 1) gewaschen wurde. Nach Einengen und Trocknen wurden so weitere 550 mg Produkt isoliert.
¹H-NMR (400 MHz, DMSO-d₆) [ppm]: 8,46 (1 H); 8,32 (1 H); 8,25 (1H);

### 26b) 3-Brom-N-methoxy-N-methyl-5-(pentafluorsulfanyl)-benzamid

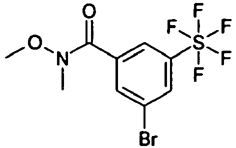

3-Brom-5-(pentafluorsulfanyl)-benzoesäure (1,35 g) wurde unter Rühren in Thionylchlorid (10 ml) gelöst und 5 h unter Feuchtigkeitsausschluss unter Rückfluss gehalten. Dann wurde das Thionylchlorid abgezogen, der Rückstand in DCM (40 ml) aufgenommen und die Lösung mit N,O-Dimethylhydroxylamin-Hydrochlorid (381 mg) versetzt. Anschließend wurde noch Hünigbase (0,7 ml) zugegeben und dann 2 h gerührt. Danach wurde zur Trockne gebracht, der Rückstand mit EE aufgenommen und die Lösung fünfmal mit Wasser gewaschen. Die EE-Phase wurde über Magnesiumsulfat getrocknet, filtriert und eingeengt. Der Rückstand wurde über Kieselgel (60 g Kartusche, n-Heptan/EE 4:1) aufgereinigt. Es wurden 1.1g der Titelverbindung erhalten.

| | |
|---|---|
| LCMS-Rt: 1,13 min | [M+H]⁺: 370,0 (Met-b) |

26c) 1-[3-Brom-5-(pentafluorsulfanyl)-phenyl]-ethanon

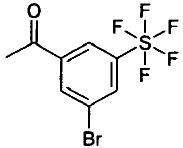

3-Brom-N-methoxy-N-methyl-5-(pentafluorsulfanyl)-benzamid (1,1 g) wurde analog 21d) umgesetzt und aufgearbeitet. Eine Reinigung mittels HPLC war nicht notwendig. Es wurden 935 mg der Titelverbindung erhalten.

| | |
|---|---|
| LCMS-Rt: 1,18 min | [M+H]⁺: 324,9 (Met-b) |

### 26d) 2-Brom-1-[3-brom-5-(pentafluorsulfanyl)-phenyl]-ethanon

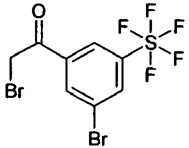

1-[3-Brom-5-(pentafluorsulfanyl)-phenyl]-ethanon (50 mg) wurde in Eisessig (3 ml) gelöst vorgelegt und Brom (50 µl einer Lösung aus 475 mg Brom in 1 ml Eisessig) langsam zugetropft. Nach 30 min Rühren bei RT wurde 3 h auf 60°C erwärmt und dann weitere 15 µl der Brom-Lösung zugegeben. Nach 2 h ließ man über Nacht stehen, erwärmte dann erneut auf 60°C und gab weitere 10 µl der Bromlösung zu. Nach 1 h wurde das Gemisch mit Toluol verdünnt und zur Trockne gebracht. Der Rückstand wurde mittels präparativer HPLC gereinigt, die Produkt enthaltenden Fraktionen wurden vereinigt, vom Acetonitril befreit, mit gesättigter Natriumhydrogencarbonat-Lösung alkalisch gestellt und dreimal mit EE extrahiert. Die vereinigten EE-Phasen wurden über Magnesiumsulfat getrocknet, filtriert und eingeengt. Es wurden 16 mg der Titelverbindung erhalten.
¹H-NMR (400 MHz, DMSO-d₆) [ppm]: 8,50 (1 H); 8,48 (1 H); 8,33 (1H); 5,08 (2 H)

### 26e) 1-[3-Brom-5-(pentafluorsulfanyl)-phenyl]-2-(5,6-diethoxy-7-fluor-1-imino-1,3-dihydro-isoindol-2-yl)-ethanon als Trifluoressigsäuresalz

5,6-Diethoxy-7-fluor-3H-isoindol-1-ylamin (18 mg, hergestellt nach EP 1391451 oder CA 2515715) wurde in THF (2 ml) suspendiert. Nach 5 min Rühren bei RT wurde 2-Brom-1-[3-brom-5-(pentafluorsulfanyl)-phenyl]-ethanon (30 mg, gelöst in 2 ml THF) zugetropft. Nach 5 h Rühren bei RT ließ man über Nacht stehen und zog dann das Lösungsmittel ab. Der Rückstand wurde mittels präparativer HPLC gereinigt und dann die Produkt enthaltenden Fraktionen vereinigt, vom Acetonitril befreit und gefriergetrocknet. Es wurden 7,5 mg der Titelverbindung erhalten.

| | |
|---|---|
| LCMS-Rt: 1,33 min | [M+H]⁺: 561,0 |

### Beispiel 27

### 1-[3-Chlor-5-(pentafluorsulfanyl)-phenyl]-2-(5,6-diethoxy-7-fluor-1-imino-1,3-dihydro-isoindol-2-yl)-ethanon als Trifluoressigsäuresalz

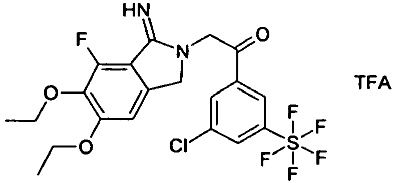

### 27a) 1-[3-Chlor-5-(pentafluorsulfanyl)-phenyl]-ethanon

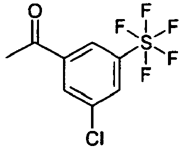

1-[3-Amino-5-(pentafluorsulfanyl)-phenyl]-ethanon (150 mg, Bsp. 22a) wurde in halbkonzentrierter Salzsäure (6 ml) gelöst. Unter Rühren und Kühlung wurde Natriumnitrit-Lösung (40 mg in Wasser gelöst) zugetropft, wobei die Temperatur 5°C nicht übersteigen sollte. Nach vollständiger Bildung des Diazoniumsalzes wurde Kupfer(I)chlorid-Lösung (68 mg in 2 ml konzentrierter Salzsäure gelöst) unter Rühren auf 0 °C abgekühlt und obige Diazoniumsalz-Lösung langsam zugetropft. Dann wird das Kältebad entfernt, 3 h bei RT gerührt und über Nacht stehen gelassen. Dann wurde das Reaktionsgemisch mit Wasser verdünnt und mit EE mehrmals extrahiert.

Die vereinigten EE-Phasen wurden mit gesättigter Natriumhydrogencarbonat-Lösung gewaschen, über Magnesiumsulfat getrocknet, filtriert und eingeengt. Der Rückstand wurde mittels präparativer HPLC gereinigt. Die Produkt enthaltenden Fraktionen wurden vereinigt, vom Acetonitril befreit, mit gesättigter Natriumhydrogencarbonat-Lösung versetzt und dreimal mit Essigester extrahiert. Die vereinigten Extrakte wurden über Magnesiumsulfat getrocknet, filtriert und eingeengt. Es wurden 104 mg der gewünschten Verbindung erhalten.
¹H-NMR (400 MHz, DMSO-d₆) [ppm]: 8,38 (1 H); 8,32 (1 H); 8,22 (1H): 2,67 (3 H)

### 27b) 2-Brom-1-[3-chlor-5-(pentafluorsulfanyl)-phenyl]-ethanon

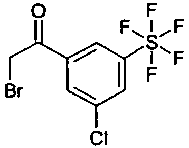

1-[3-Chlor-5-(pentafluorsulfanyl)-phenyl]-ethanon (100 mg) wurde analog Beispiel 26d) umgesetzt, aufgearbeitet und isoliert. Es wurden 60 mg der Titelverbindung erhalten.

| | |
|---|---|
| LCMS-Rt: 1,80 min | [M+H]⁺: 358,9 |

### 27c) 1-[3-Chlor-5-(pentafluorsulfanyl)-phenyl]-2-(5,6-diethoxy-7-fluor-1-imino-1,3-dihydro-isoindol-2-yl)-ethanon als Trifluoressigsäuresalz

5,6-Diethoxy-7-fluor-3H-isoindol-1-ylamin (20 mg, hergestellt nach EP 1391451 oder CA 2515715) und 2-Brom-1-[3-chlor-5-(pentafluorsulfanyl)-phenyl]-ethanon (30 mg) wurden analog Beispiel 26e) umgesetzt. Es wurden 15 mg der Titelverbindung erhalten.

| | |
|---|---|
| LCMS-Rt: 1,32 min | [M+H]⁺: 517,0 |

### Beispiel 28

### 1-[2-Brom-4-(pentafluorsulfanyl)-phenyl]-2-(5,6-diethoxy-7-fluor-1-imino-1,3-dihydro-isoindol-2-yl)-ethanon als Trifluoressigsäuresalz

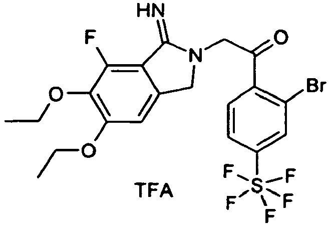

### 28a) 1-[2-Brom-4-(pentafluorsulfanyl)-phenyl]-ethanon und 2-Brom-1-[2-brom-4-(pentafluorsulfanyl)-phenyl]-ethanon

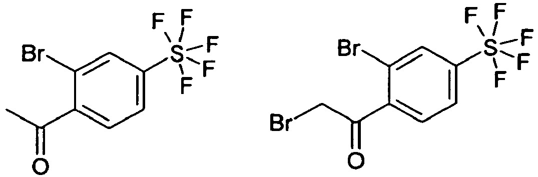

1-[2-Amino-4-(pentafluorsulfanyl)-phenyl]-ethanon (130 mg, Beispiel 19d) wurde in 24%iger Bromwasserstoff-Lösung (6 ml) gelöst. Unter Rühren und Kühlung wurde Natriumnitrit-Lösung (35 mg in 3 ml Wasser gelöst) zugetropft. Nach vollständiger Bildung des Diazoniumsalzes wurde Kupfer(I)bromid-Lösung (86 mg in 2 ml 48%iger Bromwasserstoffsäure gelöst) unter Rühren auf 0 °C abgekühlt und obige Diazoniumsalz-Lösung langsam zugetropft. Dann wurde das Kältebad entfernt und 5 h bei RT gerührt und übers Wochenende stehen gelassen. Danach wurde das Gemisch 4 h auf 50°C erwärmt. Nach Abkühlen wurde das Reaktionsgemisch mit Wasser verdünnt und mit EE mehrmals extrahiert. Die vereinigten EE-Phasen wurden mit gesättigter Natriumhydrogencarbonat-Lösung gewaschen, über Magnesiumsulfat getrocknet, filtriert und eingeengt. Der Rückstand wurde mittels präparativer HPLC gereinigt. Die jeweiligen Produkt enthaltenden Fraktionen wurden vereinigt, vom Acetonitril befreit, mit gesättigter Natriumhydrogencarbonat-Lösung versetzt und dreimal mit Essigester extrahiert. Die vereinigten Extrakte wurden über Magnesiumsulfat getrocknet, filtriert und eingeengt. Es wurden 36 mg 1-[2-brom-4-(pentafluorsulfanyl)-phenyl]-ethanon und 36 mg 2-Brom-1-[2-brom-4-(pentafluorsulfanyl)-phenyl]-ethanon erhalten.
1-[2-Brom-4-(pentafluorsulfanyl)-phenyl]-ethanon
¹H-NMR (400 MHz, DMSO-d₆) [ppm]: 8,28 (1 H); 8,08 (1 H); 7,86 (1H); 2,60 (3 H)
2-Brom-1-[2-brom-4-(pentafluorsulfanyl)-phenyl]-ethanon
¹H-NMR (400 MHz, DMSO-d₆) [ppm]: 8,34 (1 H); 8,14 (1 H); 7,98 (1H); 4,90 (2 H)

### 28b) 1-[2-Brom-4-(pentafluorsulfanyl)-phenyl]-2-(5,6-diethoxy-7-fluor-1-imino-1,3-dihydro-isoindol-2-yl)-ethanon als Trifluoressigsäuresalz

5,6-Diethoxy-7-fluor-3H-isoindol-1-ylamin (20 mg, hergestellt nach EP 1391451 oder CA 2515715) und 2-Brom-1-[2-brom-4-(pentafluorsulfanyl)-phenyl]-ethanon (34 mg) wurden analog Beispiel 26e) umgesetzt, aufgearbeitet und gereinigt. Es wurden 11 mg der Titelverbindung erhalten.

| | |
|---|---|
| LCMS-Rt: 1,33 min | [M+H]⁺: 561,0 |

### Beispiel 29

### 1-[2-Chlor-4-(pentafluorsulfanyl)-phenyl]-2-(5,6-diethoxy-7-fluor-1-imino-1,3-dihydro-isoindol-2-yl)-ethanon als Trifluoressigsäuresalz

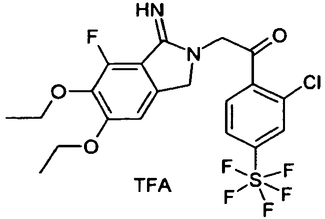

### 29a) 1-[2-Chlor-4-(pentafluorsulfanyl)-phenyl]-ethanon

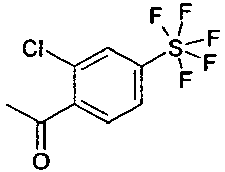

1-[2-Amino-4-(pentafluorsulfanyl)-phenyl]-ethanon (170 mg, Beispiel 19d) wurde analog Beispiel 27a) umgesetzt, aufgearbeitet und gereinigt. Es wurden 86 mg der Titelverbindung erhalten.
¹H-NMR (400 MHz, DMSO-d₆) [ppm]: 8,18 (1 H); 8,04 (1 H); 7,90 (1H); 2,61 (3 H)

### 29b) 2-Brom-1-[2-chlor-4-(pentafluorsulfanyl)-phenyl]-ethanon

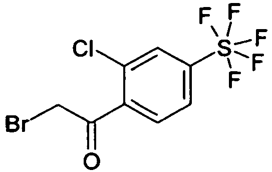

1-[2-Chlor-4-(pentafluorsulfanyl)-phenyl]-ethanon (70 mg) wurde analog Beispiel 8b) bromiert. Allerdings wurde das Gemisch 4 h bei 50°C in der 2 N Schwefelsäure gerührt. Es wurden 31 mg der Titelverbindung erhalten.
¹H-NMR (400 MHz, DMSO-d₆) [ppm]: 8,25 (1 H); 8,10 (1 H); 8,03 (1H); 4,91 (2 H)

### 29c) 1-[2-Chlor-4-(pentafluorsulfanyl)-phenyl]-2-(5,6-diethoxy-7-fluor-1-imino-1,3-dihydro-isoindol-2-yl)-ethanon als Trifluoressigsäuresalz

5,6-Diethoxy-7-fluor-3H-isoindol-1-ylamin (19 mg, hergestellt nach EP 1391451 oder CA 2515715) und 2-Brom-1-[2-chlor-4-(pentafluorsulfanyl)-phenyl]-ethanon (29 mg) wurden analog Beispiel 26e) umgesetzt. Es wurden 18 mg der Titelverbindung erhalten.

| | |
|---|---|
| LCMS-Rt: 1,30 min | [M+H]⁺: 517,0 |

### Beispiel 30

### 2-(5,6-Diethoxy-7-fluor-1-imino-1,3-dihydro-isoindol-2-yl)-1-[3-morpholin-4-yl-5-(pentafluorsulfanyl)-phenyl]-ethanon als Trifluoressigsäuresalz

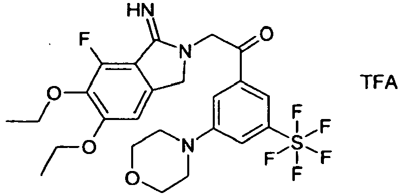

### 30a) N-Methoxy-N-methyl-3-morpholin-4-yl-5-(pentafluorsulfanyl)-benzamid

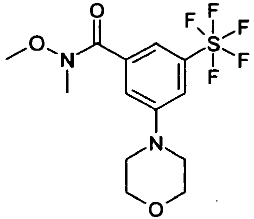

3-Amino-N-methoxy-N-methyl-5-(pentafluorsulfanyl)-benzamid (6,3 g, Beispiel 4b) wurde in DMF (80 ml) gelöst und Cäsiumcarbonat (10,1g), Natriumiodid (0,62 g) und Bis-(2-bromethyl)ether (19,37 g) zugegeben. Das Gemisch wurde auf 10 Mikrowellengefäße verteilt, die in der Mikrowelle jeweils 3 h auf 130 °C erhitzt wurden. Anschließend wurden die Ansätze vereinigt und vom Lösungsmittel befreit. Der Rückstand wurde in EE aufgenommen und mit Wasser gewaschen. Die EE-Phase wurde getrocknet und eingeengt. Der Rückstand wurde über Kieselgel (120 g Kartusche, n-Heptan/EE-Gradient von 0-100 % in 30 min) aufgereinigt. Es wurden 2,48 g der Titelverbindung erhalten.

| | |
|---|---|
| LCMS-Rt: 1,41 min | [M+H]⁺: 377,0 |

### 30b) 1-[3-Morpholin-4-yl-5-(pentafluorsulfanyl)-phenyl]-ethanon

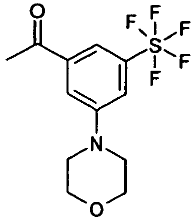

N-Methoxy-N-methyl-3-morpholin-4-yl-5-(pentafluorsulfanyl)-benzamid (2,38 g) wurde in THF (50 ml) gelöst, bei 0°C Methylmagnesiumbromid (4,22 ml, 3 M in Ether) zugetropft und anschließend 2 h bei RT gerührt. Dann wurde mit 1 N Salzsäure (100 ml) versetzt, mit Wasser verdünnt und dreimal mit EE ausgeschüttelt. Danach wurden die vereinigten EE-Phasen über Magnesiumsulfat getrocknet, filtriert und eingeengt. Der Rückstand wurde über Kieselgel (80 g Kartusche, n-Heptan/EE-Gradient von 0-70% in 40 min) aufgereinigt. Es wurden 1.1g der Titelverbindung erhalten.

| | |
|---|---|
| LCMS-Rt:1,57 min | [M+H]⁺: 332,0 |

### 30c) 2-Brom-1-[3-morpholin-4-yl-5-(pentafluorsulfanyl)-phenyl]-ethanon

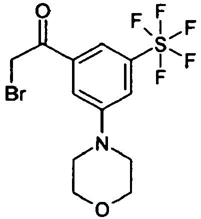

1-[3-Morpholin-4-yl-5-(pentafluorsulfanyl)-phenyl]-ethanon (1,1 g) wurde in Methanol/THF (20/20 ml) gelöst und unter Rühren mit Phenyltrimethylammoniumtribromid (1,25 g) versetzt. Nach 27 h Rühren bei RT wurde 20%ige Zitronensäure (50 ml) zugegeben und 1 h gerührt. Nach Zugabe von DCM (100 ml) wurde die DCM-Phase abgetrennt, getrocknet und eingeengt. Der Rückstand wurde in Acetonitril (100 ml) gelöst, und zur Lösung 2 N Schwefelsäure (20 ml) gegeben. Nach 24 h Rühren bei RT wurde mit Wasser versetzt und mit EE extrahiert. Die EE-Phase wurde mit gesättigter Natriumhydrogencarbonat-Lösung gewaschen, getrocknet und eingeengt. Der Rückstand wurde über Kieselgel (80 g Kartusche, n-Heptan/EE-Gradient von 0-60% in 40 min) aufgereinigt. Es wurden 866 mg der Titelverbindung erhalten.

| | |
|---|---|
| LCMS-Rt: 1,69 min | [M+H]⁺: 410,0 |

### 30d) 2-(5,6-Diethoxy-7-fluor-1-imino-1,3-dihydro-isoindol-2-yl)-1-[3-morpholin-4-yl-5-(pentafluorsulfanyl)-phenyl]-ethanon als Trifluoressigsäuresalz

5,6-Diethoxy-7-fluor-3H-isoindol-1-ylamin (9 mg, hergestellt nach EP 1391451 oder CA 2515715) und 2-Brom-1-[3-morpholin-4-yl-5-(pentafluorsulfanyl)-phenyl]-ethanon (15 mg) wurden analog Beispiel 26e) umgesetzt, aufgearbeitet und gereinigt. Es wurden 15 mg der Titelverbindung erhalten.

| | |
|---|---|
| LCMS-Rt: 0,97 min | [M+H]⁺: 568,2 (Met-b) |

### Beispiel 31

### 2-(5,6-Diethoxy-7-fluor-1-imino-1,3-dihydro-isoindol-2-yl)-1-[3-(2-methoxy-ethoxy)-5-(pentafluorsulfanyl)-phenyl]-ethanon-Hydrochlorid

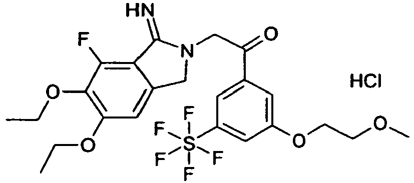

### 31 a) 1-[3-Amino-5-(pentafluorsulfanyl)-phenyl]-ethanon

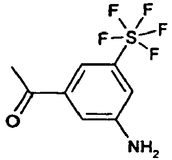

N-(3-Acetyl-5-pentafluorsulfanyl-phenyl)-2,2,2-trifluor-acetamid (9,4 g, Beispiel 4d) wurde mit halbkonzentrierter Schwefelsäure versetzt (200 ml) und zum Lösen DCM (15 ml) zugegeben. Nach 7 h Rühren bei 100°C wurde über Nacht stehen gelassen. Dann wurde der Ansatz auf Eiswasser gegeben und mit EE extrahiert. Die vereinigten EE-Phasen wurden mit gesättigter Natriumhydrogencarbonat-Lösung gewaschen, über Magnesiumsulfat getrocknet, filtriert und einrotiert. Es wurden 6,36 g der Titelverbindung erhalten.

| | |
|---|---|
| LCMS-Rt: 1,38 min | [M+H]⁺: 262,0 |

### 31b) 1-[3-Hydroxy-5-(pentafluorsulfanyl)-phenyl]-ethanon

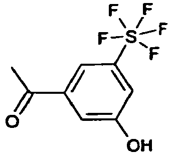

1-[3-Amino-5-(pentafluorsulfanyl)-phenyl]-ethanon (3,00 g) wurde in 35 %iger wässriger Schwefelsäure (25 ml) in der Wärme gelöst. Die Lösung wurde auf -5°C abgekühlt und eine Lösung von Natriumnitrit (780 mg) in 15 ml Wasser innerhalb von 10 min zugetropft. Nach 40 min bei -5°C wurde das Kältebad entfernt und 2 h auf 100°C erhitzt. Nach Abkühlen wurde mit EE zweimal extrahiert. Die vereinigten EE-Phasen wurden mit gesättigter Natriumhydrogencarbonat-Lösung gewaschen, getrocknet, filtriert und eingeengt. Der Rückstand wurde über Kieselgel (80 g Kartusche, n-Heptan/MtB-Ether-Gradient von 0-100 % in 40 min) aufgereinigt. Es wurden 1,62 g der Titelverbindung erhalten.
¹H-NMR (500 MHz, DMSO-d6) [ppm]: 10,71 (1 H); 7,73 (1 H); 7,59 (1 H); 7,47 (1 H); 2,61 (3 H)

### 31c) 1-[3-(2-Methoxy-ethoxy)-5-(pentafluorsulfanyl)-phenyl]-ethanon

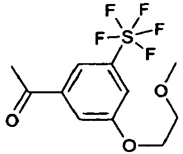

1-[3-Hydroxy-5-(pentafluorsulfanyl)-phenyl]-ethanon (592 mg) und 1-Brom-2-methoxyethan (255 µl) wurden in DMF (14,8 ml) gelöst und Natriumhydrid (65 mg) zugesetzt. Nach 2 h Rühren bei RT wurde weiteres Bromid (80 µl) zugesetzt und 12 h auf 50°C erwärmt. Dann wurde das DMF abgezogen und der Rückstand in EE aufgenommen, mit Wasser gewaschen, getrocknet, filtriert und eingeengt. Es wurden 578 mg der Titelverbindung erhalten.

| | |
|---|---|
| LCMS-Rt: 1,58 min | [M+H]⁺: 321,1 |

### 31d) 2-Brom-1-[3-(2-methoxy-ethoxy)-5-(pentafluorsulfanyl)-phenyl]-ethanon

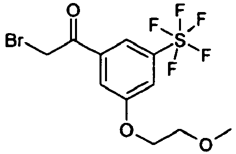

1-[3-(2-Methoxy-ethoxy)-5-(pentafluorsulfanyl)-phenyl]-ethanon (578 mg) wurde analog Beispiel 30c) bromiert. Es wurden 290 mg der Titelverbindung erhalten.

| | |
|---|---|
| LCMS-Rt: 1,70 min | [M+H]⁺: 399,0 |

### 31e) 2-(5,6-Diethoxy-7-fluor-1-imino-1,3-dihydro-isoindol-2-yl)-1-[3-(2-methoxy-ethoxy)-5-(pentafluorsulfanyl)-phenyl]-ethanon-Hydrochlorid

5,6-Diethoxy-7-fluor-3H-isoindol-1-ylamin (25 mg, hergestellt nach EP 1391451 oder CA 2515715) und 2-Brom-1-[3-(2-methoxy-ethoxy)-5-(pentafluorsulfanyl)-phenyl]-ethanon (43 mg) wurden analog Beispiel 26e) umgesetzt, aufgearbeitet und gereinigt. Es wurden 37 mg der Titelverbindung erhalten.

| | |
|---|---|
| LCMS-Rt: 0,97 min | [M+H]⁺: 568,2 (Met-b) |

### Beispiel 32

### 2-(5,6-Diethoxy-7-fluor-1-imino-1,3-dihydro-isoindol-2-yl)-1-[3-ethoxy-5-(pentafluorsulfanyl)-phenyl]-propan-1-on als Trifluoressigsäuresalz

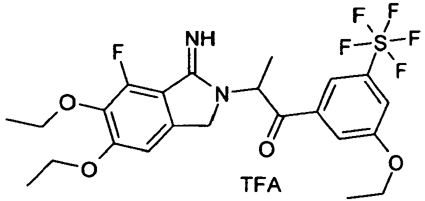

### 32a) 1-[3-Ethoxy-5-(pentafluorsulfanyl)-phenylj-propan-1-on

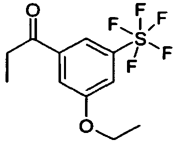

3-Ethoxy-N-methoxy-N-methyl-5-(pentafluorsulfanyl)-benzamid (170 mg, Beispiel 24b) wurde in absolutem THF (5 ml) gelöst und bei 0°C unter Rühren Ethylmagnesiumbromid (0,65 ml; 2 M in Diethylether) zugetropft. Nach Zugabe wurde das Eisbad entfernt und 2 h bei RT gerührt. Dann wurde weiteres Ethylmagnesiumbromid (0,1 ml) zugegeben und noch einmal 2 h gerührt. Unter Kühlung wurde anschließend 1 N Salzsäure zugetropft, gefolgt von Wasser und Essigester. Die organische Phase wurde abgetrennt und die wässrige noch zweimal mit Essigester extrahiert. Die vereinigten Extrakte wurden über Magnesiumsulfat getrocknet, filtriert und eingeengt. Es wurden 150 mg der gewünschten Verbindung erhalten.

| | |
|---|---|
| LCMS-Rt: 1,26 min | [M+H]⁺: 305,1 (Met-b) |

### 32b) 2-Brom-1-[3-ethoxy-5-(pentafluorsulfanyl)-phenyl]-propan-1-on

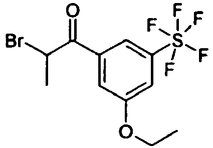

1-[3-Ethoxy-5-(pentamethylsulfanyl)-phenyl]-propan-1-on (150 mg) wurde in THF (15 ml) gelöst und unter Rühren bei RT Phenyltrimethylammoniumtribromid (185 mg) zugegeben. Nach 5 h Rühren bei RT wurde mit Wasser und gesättigter Natriumhydrogencarbonat-Lösung versetzt und EE zugegeben. Die EE-Phase wurde abgetrennt und die alkalische Wasserphase dreimal mit EE extrahiert. Die vereinigten organischen Phasen wurden einmal mit gesättigter Kochsalz-Lösung gewaschen, über Magnesiumsulfat getrocknet, filtriert und eingeengt. Das Rohprodukt wurde mittels präparativer HPLC gereinigt. Die sauberen Produktfraktionen wurden vereinigt, im Vakuum vom Acetonitril befreit, mit gesättigter Natriumhydrogencarbonat-Lösung alkalisch gestellt und fünfmal mit EE extrahiert. Die vereinigten organischen Phasen wurden über Magnesiumsulfat getrocknet und nach Abfiltrieren des Trockenmittels im Vakuum zur Trockne gebracht. Es wurden 120 mg der Titelverbindung isoliert.

| | |
|---|---|
| LCMS-Rt: 1,29 min | [M+H]⁺: 383,0 (Met-b) |

### 32c) 2-(5,6-Diethoxy-7-fluor-1-imino-1,3-dihydro-isoindol-2-yl)-1-[3-ethoxy-5-(pentafluorsulfanyl)-phenyl]-propan-1-on als Trifluoressigsäuresalz

5,6-Diethoxy-7-fluor-3H-isoindol-1-ylamin (5 mg, hergestellt nach EP 1391451 oder CA 2515715) wurde in THF (5 ml) suspendiert und nach 5 min Rühren bei RT wurde 2-Brom-1-[3-ethoxy-5-(pentafluorsulfanyl)-phenyl]-propan-1-on (40 mg, gelöst in 3 ml THF) zugetropft. Nach 12 h Rühren bei RT wurde mit Wasser und EE versetzt und die Phasen getrennt. Die Wasserphase wurde noch dreimal mit EE extrahiert. Die vereinigten EE-Phasen wurden mittels präparativer HPLC gereinigt, die Produkt enthaltenden Fraktionen vereinigt, vom Acetonitril befreit und gefriergetrocknet. Zur weiteren Reinigung wurde der Rückstand über Kieselgel (DCM/Methanol 40:1) gegeben. Die vereinigten sauberen Fraktionen wurden zur Trockne gebracht, mit ACN/Wasser (mit 0,05 % TFA) aufgenommen und gefriergetrocknet. Es wurden 6 mg der Titelverbindung erhalten.

| | |
|---|---|
| LCMS-Rt: 1,04 min | [M+H]⁺: 541,2 (Met-b) |

### Beispiel 33

### 2-(5,6-Diethoxy-7-fluor-1-imino-1,3-dihydro-isoindol-2-yl)-1-[3-(pentafluorsulfanyl)-5-pyrrolidin-1-yl-phenyl]-ethanon als Trifluoressigsäuresalz

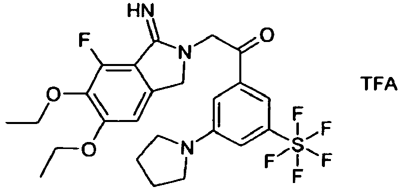

### 33a) 1-Brom-3-(1,1-dimethoxy-ethyl)-5-(pentafluorsulfanyl)-benzol

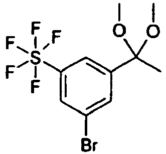

1-[3-Brom-5-(pentafluorsulfanyl)-phenyl]-ethanon (835 mg, Beispiel 26c) wurde in Methanol (50 ml) gelöst und bei RT mit DL-10-Camphersulfonsäure (0,9 mg) und mit Trimethylorthoformiat (0,85 ml) versetzt. Das Reaktionsgemisch rührte 2 h bei RT und stand dann über Nacht. Dann wurde weitere 5 h bei RT gerührt. Anschließend wurde mit gesättigter Natriumhydrogencarbonat-Lösung auf pH 9 gestellt, mit viel EE versetzt und Wasser zugegeben. Die organische Phase wurde abgetrennt und die wässrige fünfmal mit EE extrahiert. Die vereinigten EE-Phasen wurden über Magnesiumsulfat getrocknet, filtriert und eingeengt. Es wurden 900 mg der Titelverbindung erhalten.
¹H-NMR (400 MHz, DMSO-d6) [ppm]: 8,14 (1 H); 7,87 (1 H); 7,81 (1 H); 3,10 (6 H); 1,50 (3 H)

### 33b) 1-[3-(Pentafluorsulfanyl)-5-pyrrolidin-1-yl-phenyl]-ethanon

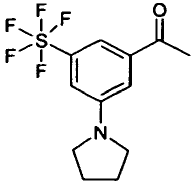

1-Brom-3-(1,1-dimethoxy-ethyl)-5-(pentafluorsulfanyl)-benzol (200 mg) wurde unter Argon in DME (30 ml) bei RT vorgelegt. Dem DME wurden 2 Tropfen Wasser zugesetzt. Mit einem starken Argonstrom wurde 45 min aus der Lösung der Sauerstoff verdrängt. Danach wurde Pyrrolidin (45 µl) über ein Septum zugetropft, gefolgt von (+)-2,2'-Bis-(diphenylphosphino)-1,1'-binaphthalin (5 mg), sowie Natrium-tert.-butylat (73 mg) und Palladium(II)-acetat (1,2 mg). Das Reaktionsgemisch wurde 3 h auf 85°C erwärmt. Dann wurde dem Reaktionsgemisch EE zugesetzt und nach Abtrennung der EE-Phase die wässrige Phase noch dreimal mit EE extrahiert. Die vereinigten EE-Phasen wurden über Magnesiumsulfat getrocknet, filtriert und eingeengt. Der Rückstand wurde in ACN / Wasser gelöst und mit 1 N Salzsäure auf pH 2 gestellt. Nach 30 min Stehen wurde eingeengt und der Rückstand mittels präparativer HPLC gereinigt. Die Produkt enthaltenden Fraktionen wurden vereinigt, vom Acetonitril befreit, mit gesättigter Natriumhydrogencarbonat-Lösung versetzt und drei Mal mit Essigester extrahiert. Die vereinigten Extrakte wurden über Magnesiumsulfat getrocknet, filtriert und eingeengt. Es wurden 55 mg der gewünschten Verbindung erhalten.

| | |
|---|---|
| LCMS-Rt: 1,24 min | [M+H]⁺: 316,1 (Met-b) |

### 33c) 2-Brom-1-[3-(pentafluorsulfanyl)-5-pyrrolidin-1-yl-phenyl]-ethanon

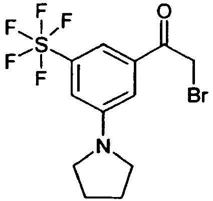

1-[3-(Pentafluorsulfanyl)-5-pyrrolidin-1-yl-phenyl]-ethanon (78 mg) wurde in Methanol (5 ml) gelöst und unter Rühren mit Trimethylorthoformiat (80 mg) und DL-10-Camphersulfonsäure (57 mg) versetzt. Nach 3 h Rühren bei RT wurde THF (5 ml) zugegeben, gefolgt von Phenyltrimethylammoniumtribromid (83 mg). Nach 3 h Rühren bei RT wurde über Nacht stehen gelassen. Dann wurde das Gemisch 3 h auf 50°C erwärmt und anschließend zur Trockne gebracht. Der Rückstand wurde in ACN aufgenommen, mit 2 N Schwefelsäure versetzt und 30 min gerührt. Dann wurde mit Wasser versetzt und eingeengt. Der Rückstand wurde mittels präparativer HPLC gereinigt. Die Produkt enthaltenden Fraktionen wurden vereinigt, vom Acetonitril befreit, mit gesättigter Natriumhydrogencarbonat-Lösung versetzt und dreimal mit Essigester extrahiert. Die vereinigten Extrakte wurden über Magnesiumsulfat getrocknet, filtriert und eingeengt. Es wurden 9 mg der gewünschten Verbindung erhalten.

| | |
|---|---|
| LCMS-Rt: 1,23 min | [M+H]⁺: 394,0 (Met-b) |

### 33d) 2-(5,6-Diethoxy-7-fluor-1-imino-1,3-dihydro-isoindol-2-yl)-1-[3-(pentafluorsulfanyl)-5-pyrrolidin-1-yl-phenyl]-ethanon als Trifluoressigsäuresalz

5,6-Diethoxy-7-fluor-3H-isoindol-1-ylamin (5 mg, hergestellt nach EP 1391451 oder CA 2515715) und 2-Brom-1-[3-(pentafluorsulfanyl)-5-pyrrolidin-1-yl-phenyl]-ethanon (8 mg) wurden analog Beispiel 26e) umgesetzt, aufgearbeitet und gereinigt. Es wurden 5 mg der Titelverbindung erhalten.

| | |
|---|---|
| LCMS-Rt: 1,05 min | [M+H]⁺: 552,2 (Met-b) |

### Biologische Beispiele

### PAR 1 Bestimmungsmethode: Hemmung der PAR1 mediierten

### Thrombozytenaggregation

Die biologische Testung der Substanzen erfolgte in der durch TRAP (Thrombinrezeptor aktivierendes Peptid) induzierten Thrombozytenaggregation im 96-well Format. Dazu wurde von gesunden freiwilligen Spendern Blut in 20 ml Spritzen abgenommen, in denen 2 ml 3,13 %-ige Natriumcitratlösung vorgelegt war. Nach einer 20-minütigen Zentrifugation bei 150 x g wurde das Plättchenreiche Plasma (PRP) abgetrennt und mit 1 µl PGE1-Lösung (500 µg/ml in Ethanol) / ml PRP versetzt. Nach 5 Minuten Inkubation bei RT wurde 15 Minuten bei 120 x g zentrifugiert, um die Leukozyten zu entfernen. Das Leukozytenfreie PRP wurde in 5 ml Portionen in 15 ml PP Röhrchen überführt und 15 Minuten bei 360 x g abzentrifugiert, um die Plättchen zu pelletieren. Anschließend wurde das Plasma dekantiert und das Plättchensediment aus 5 ml PRP in 1 ml Tyrode (120 mM NaCl, 2,6 mM KCI, 12 mM NaHCO₃, 0,39 mM NaH₂PO₄ x H₂O, 10 mM HEPES, 0,35% BSA, 5,5 mM Glukose, pH 7,4) resuspendiert und mit Tyrode auf eine Plättchenzahl von 3x10⁵ / Mikroliter (µL) eingestellt. 13 ml dieser Zellsuspension wurde dann mit 866 µL 10 mM CaCl₂-Lösung versetzt und 120 µL davon pro well einer 96-well-Platte pipettiert, in dem 15 µL der zu testenden Substanz vorgelegt waren. Nach 30 Minuten Inkubation bei RT im Dunklen wurden 15 µL einer TRAP-Lösung (70-100 µM) als Agonist zugegeben und in einem SpectraMax 340 bei 650 nm über 20 Minuten bei 37° C unter Schütteln eine Kinetik aufgezeichnet. Die Flächen unter den Kurven von Negativkontrolle (Tyrode/ DMSO) und Positivkontrolle (15 µl Agonist /DMSO) wurden berechnet und die Differenz als 100 % Wert festgelegt. Die zu testenden Substanzen wurden in Doppelbestimmung als Verdünnungsreihen pipettiert, ebenfalls die AUC jeder Substanzkonzentration bestimmt und die % Hemmung der AUC gegen die Kontrolle errechnet. Anhand der % Hemmung wurde mit Hilfe nichtlinearer Regressionsanalyse gemäß der 4 Parameter-Gleichung die IC₅₀ berechnet.

Tabelle 2 zeigt die Ergebnisse.

**Tabelle 2:**

| Verbindung aus Beispiel | Hemmung der Thrombozytenaggregation IC₅₀ [mikro M] | Verbindung aus Beispiel | Hemmung der Thrombozytenaggregation IC₅₀ [mikro M] |
|---|---|---|---|
| 2 | 0,597 | 3 | 0,002 |
| 8 | 0,017 | 10 | 0,718 |
| 12 | 0,387 | 16 | 10,6 |
| 18 | 0,175 | 19 | 0,098 |
| 21 | 0,028 | | |

### PAR1-Bindungstest

Die synthetisierten Substanzen wurden in einem PAR1-Bindungstest untersucht. Hierbei wurde geprüft, ob die Substanzen die Bindung eines radioaktiv markierten, literaturbekannten PAR1-Agonisten an den PAR1-Rezeptor inhibieren können (Ho-Sam Ahn, Mol Pharm, 51:350-356, 1997).
Der humane PAR1-Rezeptor wurde transient in High Five Insektenzellen exprimiert. Aus diesen wurde gemäß Standardmethoden nach 48 Stunden eine Membranpräparation hergestellt, in 10 mM Tris-HCl; 0,3 mM EDTA; 1 mM EGTA; 250 mM Sucrose pH 7,5 aliquotiert und bei -80°C gelagert.
Die Substanzen wurden 15 Minuten bei RT mit der Membran vorinkubiert, dann erfolgte die Zugabe des Radioliganden (ALA-(para-F-Phe)-Arg-ChA-homoArg-(3,4-³H-Tyr)-NH₂; ca. 40 Ci/mMol). Die Endkonzentration des Radioliganden im Testpuffer (50 mM Tris-HCl; 10 mM MgCl₂; 1 mM EGTA; 0,1 % BSA; 2% DMSO) betrug 20 nM, die der Membran 1 mg/ml. Nach einer Inkubationszeit von 60 Minuten wurden 25 µL des Ansatzes in eine 96-well MultiScreenHTS FB Mikrotiterfiltrationsplatte (Fa. Millipore) übertragen, die zuvor 5 Stunden bei RT mit einer 0,75%-igen wässrigen Polyethylenimin-Lösung vorbehandelt worden war. Danach wurde unter Vakuumabsaugung jedes well viermal mit 300 µL Puffer (50 mM Tris-HCl; 10 mM MgCl₂; 1 mM EGTA) gewaschen. Die Platte wurde dann über Nacht getrocknet, 100 µl Szintillator pro Well zugegeben und nach 6 Stunden in einem Wallac MicroBeta (Fa. PerkinElmer) Flüssigszintillationzähler vermessen. Die unspezifische Bindung wurde in Gegenwart von 100 µM SCH79797 (PAR-1 Antagonist; Fa. Tocris Cat. No 1592) bestimmt und von allen Messwerten subtrahiert. Als 100% Wert diente eine Kontrolle ohne Inhibitor. Aus den % Hemmungswerten einer Substanzverdünnungsreihe wurde mit Hilfe nichtlinearer Regressionsanalyse gemäß der 4 Parameter-Gleichung die IC₅₀ berechnet.

Tabelle 2 zeigt die Ergebnisse.

**Tabelle 2:**

| Verbindung aus Beispiel | Hemmung der Bindung IC₅₀ [mikro M] |
|---|---|
| 4 | 4,80 |
| 14 | 0,227 |
| 17 | 0,360 |
| 20 | 0,128 |
| 24 | 0,512 |
| 27 | 1,7 |
| 30 | 0,225 |
| 34 | 0,268 |

## Patentansprüche

1. Verbindung der Formel I 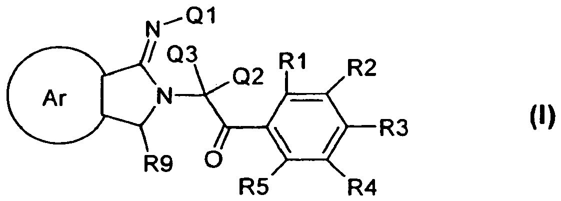 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei Ar für einen annelierten Benzol-, Pyridin-, Pyrimidin-, Pyridazin- oder Pyrazin-Ring steht, wobei der annilierte Ring unsubstituiert oder ein-, zwei-, drei- oder vierfach unabhängig voneinander durch -(C₁-C₆)-Alkyl, -(C₃-C₆)-Cycloalkyl, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, -(C₀-C₄)-Alkylen-N(R11)-R12, -(C₀-C₄)-Alkylen-N(R11)-C(O)-R12, Halogen, OH, -CN, -NO₂, -SO₂CH₃, -SO₂CF₃, -SF₅, -Si[-(C₁-C₄)-Alkyl]₃, -(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₄-C₁₅₎-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₉)-Alkyl oder -O-(C₃-C₆)-Cycloalkyl substituiert ist, oder -O-(C₁-C₉)-Alkylen-O-(C₁-C₉)-Alkyl, substituiert ist,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH⁻, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
-O-(C₃-C₆)-Cycloalkyl substituiert ist,
wobei in Alkyl, Alkylen oder Cycloalkyl die Wasserstoffatome ganz oder teilweise durch Fluor ersetzt sein können,
Q1 für Wasserstoffatom, -(C₁-C₆)-Alkyl, -(C₃-C₆)-Cycloalkyl, -OH, -O-(C₁-C₆)-Alkyl oder -O-(C₃-C₆)-Cycloalkyl steht, wobei Alkyl und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können, Q2 und Q3 gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
R11 und R12 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R11 und R12 in den Fragmenten "N(R11)-R12" und "N(R11)-C(O)-R12" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
R1, R2, R3, R4 und R5 gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₆)-Alkyl, -O-(C₃-C₆)-Cycloalkyl, -(C₀-C₄)-Alkylen-(CO)-N(R21)-R22, -SO₂CH₃, -SO₂CF₃, -(C₀-C₄)-Alkylen-C(O)-O-R21. Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -O-(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -Sil-(C₁-C₄)-Alkyl]₃ oder -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, stehen,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
-O-(C₃-C₆)-Cycloalkyl, substituiert ist,
wobei in Alkyl, Alkylen oder Cycloalkyl die Wasserstoffatome ganz oder teilweise durch Fluor ersetzt sein können,
mit der Maßgabe, das mindestens ein R1, R2, R3, R4 oder R5 für -SF₅ steht, R9 für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, steht, und wobei in Alkyl, Alkylen oder Cycloalkyl die Wasserstoffatome ganz oder teilweise durch Fluor ersetzt sein können,
R21 und R22 unabhängig voneinander für Wasserstoffatom, -(C₁-C6)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
wobei der Rest -(C₄-C₁₅)-Het für ein Ringsystem mit 4 bis 15 Kohlenstoffatomen steht, das in ein, zwei oder drei miteinander verbundenen Ringsystemen vorliegt und das je nach Ringgröße ein, zwei, drei oder vier gleiche oder verschiedene Heteroatome aus der Reihe Sauerstoff, Stickstoff oder Schwefel enthält.

2. Verbindung der Formel I gemäß Anspruch 1, wobei
Ar für einen annelierten Benzol- oder Pyridin-Ring steht,
wobei der annilierte Ring unsubstituiert oder ein-, zwei-, drei- oder vierfach unabhängig voneinander durch -(C₁-C₆)-Alkyl, -(C₃-C₆)-Cycloalkyl, OH, -CN, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-Alkylen-C(O)-R11, SO₂CH3, -(C₀-C₄)-Alkylen-N(R11)-R12, -(C₀-C₄)-Alkylen-N(R11)-C(O)-R12, Halogen, -SO₂CF₃, -Si[-(C₁-C₄)-Alkyl]₃, -(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder-O-(C₃-C₆)-Cycloalkyl substituiert ist, oder -O-(C₁-C₆)-Alkylen-O-(C₁-C₆)-Alkyl substituiert ist,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder
-O-(C₃-C₆)-Cycloalkyl substituiert ist,
wobei in Alkyl, Alkylen oder Cycloalkyl die Wasserstoffatome ganz oder teilweise durch Fluor ersetzt sein können,
Q1, Q2 und Q3 gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
R11 und R12 unabhängig voneinander für Wasserstoffatom, -(C₁-C₆)-Alkyl, -(C₃-C₆)-Cycloalkyl, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R11 und R12 in den Fragmenten "N(R11)-R12" und "N(R11)-C(O)-R12" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
R1, R2, R3, R4 und R5 gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₆)-Alkyl, -O-(C₃-C₆)-Cycloalkyl, -(C₀-C₄)-Alkylen-(CO)-N(R21)-R22, -SO₂CH₃, -SO₂CF₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -O-(C₁-C₆)-Alkylen-O-(C₁-C₆)-Alkyl, -(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl, substituiert ist, -Si[-(C₁-C₄)-Alkyl]₃ oder -(C₄-C₁₅)-Het, wobei Het unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, stehen,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH, -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C3-C6)-Cycloalkyl, OH, -O-(C₁-C₆)-Alkyl oder -O-(C₃-C₆)-Cycloalkyl substituiert ist,
-(C₄-C₁₅₎-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, oder -O-(C₃-C₆)-Cycloalkyl, substituiert ist,
wobei in Alkyl, Alkylen oder Cycloalkyl die Wasserstoffatome ganz oder teilweise durch Fluor ersetzt sein können,
mit der Maßgabe, das mindestens ein R1, R2, R3, R4 oder R5 für -SF₅ steht, R9 für Wasserstoffatom, -(C₁-C₆)-Alkyl, -(C₃-C₆)-Cycloalkyl, -(C₀-C₄)-Alkylen-(C₆-C₁₄)-Aryl, wobei Aryl unsubstituiert oder ein-, zwei- oder dreifach unabhängig voneinander durch -O-(C₁-C₆)-Alkyl, -(C₁-C₄)-Alkyl, OH, -(C₃-C₆)-Cycloalkyl oder -O-(C₃-C₆)-Cycloalkyl substituiert ist, oder -(C₀-C₄)-Alkylen-(C₄-C₁₅)-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist, steht, und wobei in Alkyl, Alkylen oder Cycloalkyl die Wasserstoffatome ganz oder teilweise durch Fluor ersetzt sein können,
R21 und R22 unabhängig voneinander für Wasserstoffatom, -(C₁-C₆)-Alkyl, -(C₃-C₆)-Cycloalkyl, -(C₀-C₄)-Alkylen-(C₆-C₁₄₎-Aryl, -(C₀-C₄)-Alkylen-(C₄-C₁₅)-Het, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen 5- bis 8-gliedrigen Ring stehen, der gemeinsam mit dem Stickstoffatom "N" oder der Gruppe "N-C(O)" gebildet wird, wobei cyclische Amine, Imide oder Lactame gebildet werden, die bis zu 2 weitere Heteroatome aus der Gruppe N, O oder S enthalten, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
wobei der Rest -(C₄-C₁₅)-Het für ein Ringsystem mit 4 bis 15 Kohlenstoffatomen steht, das in ein, zwei oder drei miteinander verbundenen Ringsystemen vorliegt und das je nach Ringgröße ein, zwei, drei oder vier gleiche oder verschiedene Heteroatome aus der Reihe Sauerstoff, Stickstoff oder Schwefel enthält..

3. Verbindung der Formel I gemäß den Ansprüchen 1 oder 2, wobei
Ar für einen annelierten Benzol- oder Pyridin-Ring steht,
wobei der annilierte Ring unsubstituiert oder ein-, zwei-, drei- oder vierfach unabhängig voneinander durch -(C₁-C₆)-Alkyl, -(C₃-C₆)-Cycloalkyl, OH, -CN, -O-(C₁-C₈)-Alkyl, -O-(C₃-C₆)-Cycloalkyl, -(C₀-C₄)-Alkylen-C(O)-N(R11)-R12, -(C₀-C₄)-Alkylen-C(O)-O-R11, -(C₀-C₄)-A)ky)en-C(O)-R11, SO₂CH₃, -(C₀-C₄)-Alkylen-N(R11)-R12, -(C₀-C₄)-Alkylen-N(R11)-C(O)-R12, Halogen, -Si[-(C₁-C₄)-Alkyl]₃, -(C₁-C₆)-Alkylen-O-(C₁-C₆)-Alkyl oder -O-(C₁-C₆)-Alkylen-O-(C₁-C₆)-Alkyl substituiert ist,
wobei Alkyl, Alkylen und Cycloalkyl jeweils unsubstituiert oder ein-, zwei- oder dreifach unabhängig voneinander durch -(C₁-C₄)-Alkyl, -(C₃-C₆)-Cycloalkyl, OH⁻. -O-(C₁-C₆)-Alkyl, -(C₆-C₁₄)-Aryl, wobei Aryl unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder -O-(C₃-C₆)-Cycloalkyl substituiert ist,
-(C₄-C₁₅₎-Het, wobei Het unsubstituiert oder ein-, zwei-, drei-, vier- oder fünffach unabhängig voneinander durch Halogen, -(C₁-C₄)-Alkyl, -(C₃-C₆)-Cycloalkyl, OH, -O-(C₁-C₆)-Alkyl oder-O-(C₃-C₆)-Cycloalkyl substituiert ist, oder
-O-(C₃-C₆)-Cycloalkyl substituiert ist,
wobei in Alkyl, Alkylen oder Cycloalkyl die Wasserstoffatome ganz oder teilweise durch Fluor ersetzt sein können,
Q1, Q2 und Q3 gleich oder verschieden sind und unabhängig voneinander für Wasserstoffatom, -(C₁-C₆)-Alkyl oder -(C₃-C₆)-Cycloalkyl stehen, wobei in Alkyl oder Cycloalkyl die Wasserstoffatome ganz oder teilweise durch Fluor ersetzt sein können,
R11 und R12 unabhängig voneinander für Wasserstoffatom, -(C₁-C₆)-Alkyl, -(C₃-C₆)-Cycloalkyl oder -SO₂CH₃ stehen,
wobei in Alkyl, Alkylen oder Cycloalkyl die Wasserstoffatome ganz oder teilweise durch Fluor ersetzt sein können, oder
R11 und R12 in den Fragmenten "N(R11)-R12" und "N(R11)-C(O)-R12" für einen 5- bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl substituiert ist, wobei in Alkyl oder Cycloalkyl die Wasserstoffatome ganz oder teilweise durch Fluor ersetzt sein können,
R1, R2, R3, R4 und R5 gleich oder verschieden sind und unabhängig voneinander für Wasserstoffatom, -(C₁-C₆)-Alkyl, -(C₃-C₆)-Cycloalkyl, OH, -CN, -NO₂, -O-(C₁-C₆)-Alkyl, -O-(C₃-C₆)-Cycloalkyl, -(C₀-C₄)-Alkylen-(CO)-N(R21 )-R22, -SO₂CH₃, -(C₀-C₄)-Alkylen-C(O)-O-R21, Halogen, -SF₅, -(C₀-C₄)-Alkylen-C(O)-R21, -(C₀-C₄)-Alkylen-N(R21)-R22, -(C₀-C₄)-Alkylen-N(R21)-C(O)-R22, -(C₁-C₆)-Alkylen-O-(C₁-C₆)-Alkyl, -O-(C₁-C₆)-Alkylen-O-(C₁-C₆)-Alkyl, -Si[-(C₁-C₄)-Alkyl]₃ oder-(C₄-C₁₅)-Het stehen,
wobei Alkyl, Alkylen und Cycloalkyl jeweils unsubstituiert oder ein-, zwei- oder dreifach unabhängig voneinander durch durch -(C₁-C₄)-Alkyl, OH, -(C₃-C₆)-Cycloalkyl, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
mit der Maßgabe, das ein R1, R2, R3, R4 oder R5 für -SF₅ steht,
R9 für Wasserstoffatom, -(C₁-C₆)-Alkyl oder -(C₃-C₆)-Cycloalkyl steht, wobei in Alkyl oder Cycloalkyl die Wasserstoffatome ganz oder teilweise durch Fluor ersetzt sein können,
R21 und R22 unabhängig voneinander für Wasserstoffatom, -(C₁-C₆)-Alkyl, -(C₃-C₆)-Cycloalkyl, -(C₀-C₄)-Alkylen-(C₆-C₁₄)-Aryl, -(C₀-C₄)-Alkylen-(C₄-C₁₅)-Het, -SO₂CH₃ oder -SO₂CF₃ stehen,
wobei in Alkyl, Alkylen oder Cycloalkyl die Wasserstoffatome ganz oder teilweise durch Fluor ersetzt sein können, oder
R21 und R22 in den Fragmenten "N(R21)-R22" und "N(R21)-C(O)-R22" für einen 5- bis 8-gliedrigen Ring stehen, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl, wobei der Ring unsubstituiert oder ein- oder zweifach unabhängig voneinander durch -(C₁-C₄)-Alkyl, -(C₃-C₆)-Cycloalkyl, OH, -O-(C₁-C₆)-Alkyl oder -O-(C₃-C₆)-Cycloalkyl, substituiert ist, wobei in Alkyl oder Cycloalkyl die Wasserstoffatome ganz oder teilweise durch Fluor ersetzt sein können,
wobei der Rest -(C₄-C₁₅)-Het für ein Ringsystem mit 4 bis 15 Kohlenstoffatomen steht, das in ein, zwei oder drei miteinander verbundenen Ringsystemen vorliegt und das je nach Ringgröße ein, zwei, drei oder vier gleiche oder verschiedene Heteroatome aus der Reihe Sauerstoff, Stickstoff oder Schwefel enthält..

4. Verbindung der Formel I gemäß den Ansprüchen 1 bis 3, wobei
Ar für einen annelierten Benzol-Ring steht,
wobei der annilierte Ring unsubstituiert oder ein- oder zweifach unabhängig voneinander durch -O-(C₁-C₆)-Alkyl, -C(O)-N(R11)-R12 oder Halogen substituiert ist,
Q1, Q2 und Q3 gleich sind und für jeweils ein Wasserstoffatom stehen, R11 und R12 unabhängig voneinander für Wasserstoffatom oder -(C₁-C₆)-Alkyl stehen,
R1, R2, R3, R4 und R5 gleich oder verschieden sind und unabhängig voneinander für Wasserstoffatom. -(C₁-C₆)-Alkyl, -O-(C₁-C₆)-Alkyl, Halogen, -SF₅, -(C₀-C₄)-Alkylen-N(R21)-R22, -O-(C₁-C₆)-Alkylen-O-(C₁-C₆)-Alkyl oder -(C₀-C₄)-Alkylen-N(R21)-C(O)-R22 stehen,
mit der Maßgabe, das ein R1, R2, R3, R4 oder R5 für -SF₅ steht,
R9 für Wasserstoffatom steht,
R21 und R22 unabhängig voneinander für Wasserstoffatom, -(C₁-C₆)-Alkyl, -(C₃-C₆)-Cycloalkyl, CF₃ oder -SO₂CH₃ stehen,
wobei in Alkyl, Alkylen oder Cycloalkyl die Wasserstoffatome ganz oder teilweise durch Fluor ersetzt sein können, oder
R21 und R22 in dem Fragment "N(R21)-R22" für einen Ring steht, ausgewählt aus der Gruppe Azetidinyl, Pyrrolidinyl, Piperidinyl, Piperazinyl, Azepinyl, Morpholinyl, Thiomorpholinyl, Pyrrolidin-2,5-dionyl, Piperidin-2,6-dionyl, Piperazin-2,6-dionyl, Morpholin-3,5-dionyl, Pyrrolidin-2-onyl, Piperidin-2-onyl, Piperazin-2-onyl und Morpholin-3-onyl.

5. Verbindung der Formel I gemäß den Ansprüchen 1 bis 4, wobei die Verbindung der Formel I ausgewählt is aus der Gruppe 2-(1-Imino-1,3-dihydro-isoindol-2-yl)-1-(3-pentafluorsulfanyl-phenyl)-ethanon als Hydrobromid, 2-[2-(3-Dimethylamino-5- pentafluorsulfanyl-phenyl)-2-oxo-ethyl]-6-ethoxy-3-imino-2,3-dihydro-1H-isoindol-5-carbonsäuremethylamid, 6-Ethoxy-3-imino-2-[2-(3-methylamino-5-pentafluorsulfanyl-phenyl)-2-oxo-ethyl]-2,3-dihydro-1H-isoindol-5-carbonsäuremethylamid, 6-Ethoxy-3-imino-2-{2-[3-pentafluorsulfanyl-5-(2,2,2-trifluor-acetylamino)-phenyl]-2-oxo-ethyl}-2,3-dihydro-1H-isoindol-5-carbonsäuremethyl-amid, 6-Ethoxy-2-(2-{3-[ethyl-(2,2,2-trifluor-acetyl)-amino]-5-pentafluorsulfanyl-phenyl}-2-oxo-ethyl)-3-imino-2,3-dihydro-1H-isoindol-5-carbonsäuremethylamid, 6-Ethoxy-2-[2-(3-ethylamino-5-pentafluorsulfanyl-phenyl)-2-oxo-ethyl]-3-imino-2,3-dihydro-1H-isoindol-5-carbonsäuremethylamid als Hydrochlorid, 2-[2-(3-Amino-5-pentafluorsulfanyl-phenyl)-2-oxo-ethyl]-6-ethoxy-3-imino-2,3-dihydro-1H-isoindol-5-carbonsäuremethylamid, 2-[2-(3-Acetylamino-5-pentafluorsulfanyl-phenyl)-2-oxo-ethyl]-6-ethoxy-3-imino-2,3-dihydro-1H-isoindol-5-carbonsäuremethylamid, 2-(5,6-Diethoxy-7-fluor-1-imino-1,3-dihydro-isoindol-2-yl)-1-[3-methylamino-5-(pentamethyl-sulfanyl)-phenyl]-ethanon, 6-Ethoxy-3-imino-2-{2-[3-dimethansulfonylamino-5-(pentafluorsulfanyl)-phenyl]-2-oxo-ethyl}-2,3-dihydro-1H-isoindol-5-carbonsäuremethylamid, 2-{2-[3-(Acetyl-methyl-amino)-5-(pentafluorsulfanyl)-phenyl]-2-oxo-ethyl}-6-ethoxy-3-imino-2,3-dihydro-1H-isoindol-5-carbonsäuremethylamid, N-[3-[2-(5,6-Diethoxy-7-fluor-1-imino-1,3-dihydro-isoindol-2-yl)-acetyl]-5-(pentafluorsulfanyl)-phenyl]-N-methyl-acetamid, N-[3-[2-(5,6-Diethoxy-7-fluor-1-imino-1,3-dihydro-isoindol-2-yl)-acetyl]-5-(pentafluorsulfanyl)-phenyt]-isobutyramid, N-[3-[2-(5,6-Diethoxy-7-fluor-1-imino-1,3-dihydro-isoindol-2-yl)-acetyl]-5-pentafluorsulfanyl)-phenyl]-acetamid als Hydrobromid, N-[5-[2-(5,6-Diethoxy-7-fluor-1-imino-1,3-dihydro-isoindol-2-yl)-acetyl]-4-methyl-2-(pentafluor-sulfanyl)-phenyl]-acetamid, 1-[5-Amino-2-methyl-4-(pentafluorsulfanyl)-phenyl]-2-(5,6-diethoxy-7-fluor-1-imino-1,3-dihydro-isoindol-2-yl)-ethanon, N-{3-[2-(5,6-Diethoxy-7-fluor-1-imino-1,3-dihydro-isoindol-2-yl)-acetyl]-5-pentafluorsulfanyl-phenyl}-3,3-dimethyl-butyramid, 1-{3-[2-(5,6-Diethoxy-7-fluor-1-imino-1,3-dihydro-isoindol-2-yl)-acetyl]-5-pentafluorsulfanyl-phenyl}-pyrrolidin-2,5-dion, 1-[2-Amino-4-(pentafluorsulfanyl)-phenyl]-2-(5,6-diethoxy-7-fluor-1-imino-1,3-dihydro-isoindol-2-yl)-ethanon, 1-[2-Amino-5-brom-4-(pentafluorsulfanyl)-phenyl]-2-(5,6-diethoxy-7-fluor-1-imino-1,3-dihydro-isoindol-2-yl)-ethanon, 2-(5,6-Diethoxy-7-fluor-1-imino-1,3-dihydro-isoindol-2-yl)-1-[3-methoxy-5-(pentafluor-sulfanyl)-phenyl]-ethanon, Cyclobutancarbonsäure [3-[2-(5,6-diethoxy-7-fluor-1-imino-1,3-dihydro-isoindol-2-yl)-acetyl]-5-(pentafluorsulfanyl)-phenyl]-amid als Hydrochlorid, Cyclopropancarbonsäure-[3-[2-(5,6-diethoxy-7-fluor-1-imino-1,3-dihydro-isoindol-2-yl)-acetyl]-5-(pentafluorsulfanyl)-phenyl]-amid als Hydrochlorid, 2-(5,6-Diethoxy-7-fluor-1-imino-1,3-dihydro-isoindol-2-yl)-1-[3-ethoxy-5-(pentafluor-sulfanyl)-phenyl]-ethanon, 6-Ethoxy-3-imino-2-{2-[3-methoxy-5-(pentafluorsulfanyl)-phenyl]-2-oxo-ethyl}-2,3-dihydro-1H-isoindol-5-carbonsäure-methylamid, 1-[3-Brom-5-(pentafluor-sulfanyl)-phenyl]-2-(5,6-diethoxy-7-fluor-1-imino-1,3-dihydro-isoindol-2-yl)-ethanon, 1-[3-Chlor-5-(pentafluorsulfanyl)-phenyl]-2-(5,6-diethoxy-7-fluor-1-imino-1,3-dihydro-isoindol-2-yl)-ethanon, 1-[2-Brom-4-(pentafluorsulfanyl)-phenyl]-2-(5,6-diethoxy-7-fluor-1-imino-1,3-dihydro-isoindol-2-yl)-ethanon, 1-[2-Chlor-4-(pentafluorsulfanyl)-phenyl]-2-(5,6-diethoxy-7-fluor-1-imino-1,3-dihydro-isoindol-2-yl)-ethanon, 2-(5,6-Diethoxy-7-fluor-1-imino-1,3-dihydro-isoindol-2-yl)-1-[3-morpholin-4-yl-5-(pentafluorsulfanyl)-phenyl]-ethanon oder 2-(5,6-Diethoxy-7-fluor-1-imino-1,3-dihydro-isoindol-2-yl)-1-[3-(2-methoxy-ethoxy)-5-(pentafluorsulfanyl)-phenyl]-ethanon als Hydrochlorid.

6. Arzneimittel, **gekennzeichnet durch** einen wirksamen Gehalt an mindestens einer Verbindung der Formel I gemäß einem oder mehreren der Ansprüche 1 bis 5 zusammen mit einem pharmazeutisch geeigneten und physiologisch verträglichen Trägerstoff, Zusatzstoff und/oder anderen Wirk- und Hilfsstoffen.

7. Verwendung der Verbindung der Formel I gemäß einem oder mehreren der Ansprüche 1 bis 5 zur Herstellung eines Arzneimittels zur Prophylaxe, Sekundärprevention und Therapie all solcher Erkrankungen, die die mit Thrombosen, Embolien, Hyperkoagulabilität, fibrotischen Veränderungen oder entzündlichen Erkrankungen einhergehen.

8. Verwendung gemäß Anspruch 7, **dadurch gekennzeichnet, dass** es sich um Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder Eingriffen, die zu einem Kontakt des Blutes mit Fremdoberflächen führen wie bei Dialysepatienten und Patienten mit Verweilkathetern oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung und/oder Behandlung von Narbenbildung.

9. Verfahren zur Herstellung der Verbindung der Formel I gemäß einem oder mehreren der Ansprüche 1 bis 5, **dadurch gekennzeichnet, dass** man
a) eine Verbindung der Formel II, 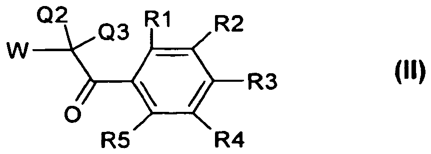 wobei R1, R2, R3, R4, R5, Q2 und Q3 wie in Formel I definiert sind und W für Chlorid, Bromid, Mesylat oder Tosylat steht, mit einer Verbindung der Formel III, 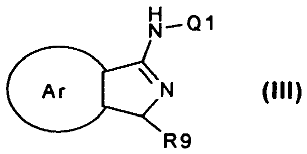 wobei Ar, R9 und Q1 wie in Formel I definiert sind, mit oder ohne Basenzu-gabe in einem Lösungsmittel zu einer Verbindung der Formel I umsetzt, oder
b) eine Verbindung der Formel VII, 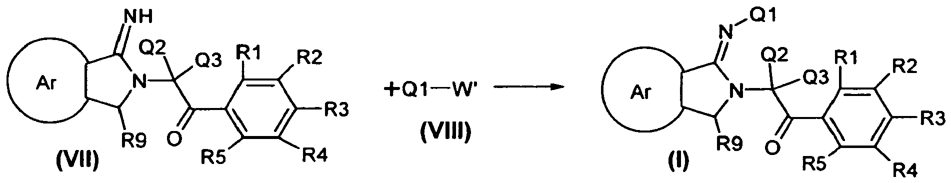 wobei R1, R2, R3, R4, R5, R9, Ar, Q1, Q2 und Q3 wie in Formel I definiert sind, mit der Verbindung Q1-W', wobei W' Chlorid, Bromid, Mesylat, Tosylat, Methylsulfat oder eine ähnliche gute Fluchtgruppe bedeutet, mit oder ohne Basenzugabe zu einer Verbindung der Formel I umsetzt, oder
c) die nach den Verfahren a) oder b) hergestellte Verbindung der Formel I entweder in freier Form isoliert oder aus physiologisch unverträglichen Salzen freisetzt oder im Falle des Vorliegens von sauren oder basischen Gruppen in physiologisch verträgliche Salze umwandelt, oder
d) eine nach den Verfahren a) oder b) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt.

10. Verfahren zur Herstellung der Verbindung der Formel 11 gemäß Anspruch 9, wobei W Br bedeutet, **dadurch gekennzeichnet, dass** man
a) eine Verbindung der Formel X, 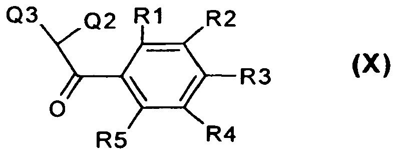 wobei R1, R2, R3, R4, R5, Q2 und Q3 wie in Formel I definiert sind, mit einem Bromierungsreagens in die Verbindung der Formel II überführt, oder
b) eine Verbindung der Formel XI oder XI', 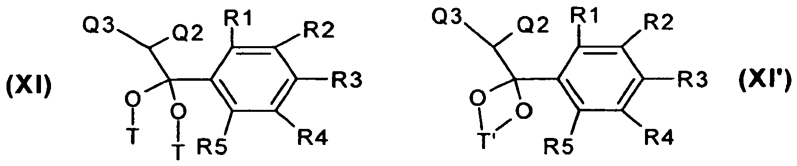 wobei R1, R2, R3, R4, R5, Q2 und Q3 wie in Formel I definiert sind, T für -(C1-C4)-Alkyl steht und T' für Ethylen, Propylen oder Butylen steht oder zusammen mit der Gruppe -O-C-O- einen 1,3- Dioxo-Ring der Ringgröße 5, 6 oder 7 bildet, mit einem Bromierungsreagenz behandelt wird und anschließend in Gegenwart einer Säure in die Verbindung der Formel II übergeführt wird.

11. Verfahren zur Herstellung der Verbindung der Formel X gemäß Anspruch 10, wobei R4 für Dimethylamin steht und R1, R2, R3, R5, Q2 und Q3 wie in Formel I definiert sind, **dadurch gekennzeichnet, dass** man
a) eine Verbindung der Formel XIVa 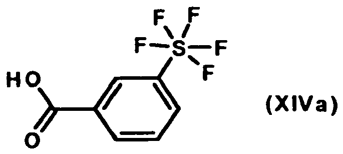 zunächst nitriert und anschließend mit Wasserstoff zum Amin der Formel Xllla reduziert, 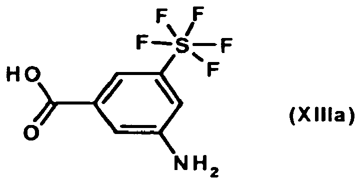
b) die erhaltene Verbindung der Formel XIIIa am Stickstoff dimethyliert und die Carbonsäure mit Thionylchlorid ins Säurechlorid überführt und anschließend mit O,N-dimethyl-hydroxylamin zu einer Verbindung der Formel XIIa umgesetzt, und 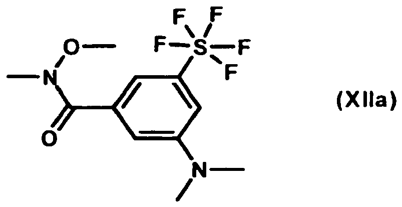
c) die erhaltene Verbindung der Formel XIIa mit Methylmagnesiumbromid in Verbindung der Formel Xa, 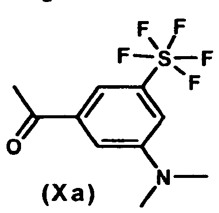 überführt.

12. Verfahren zur Herstellung der Verbindung der Formel X gemäß Anspruch 10, wobei R4 für Methoxy steht und R1, R2, R3, R5, Q2 und Q3 wie in Formel I definiert sind, **dadurch gekennzeichnet, dass** man
a) die Verbindung der Formel Xllla gemäß Anspruch 11 zunächst diazotiert und anschließend zum Phenol Xlllb verkocht, 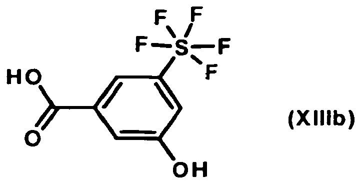
b) die erhaltene Verbindung der Formel XIIIb methyliert, ins Weinrebamid überführt und anschließend mit Methylmagnesiumbromid in die Verbindung Xb überführt.

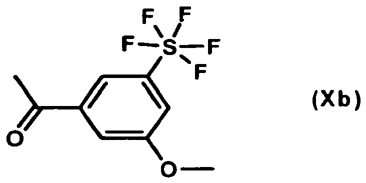

## Claims

1. A compound of the formula I 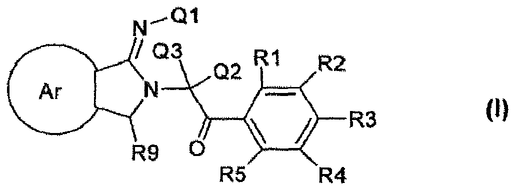 and/or any stereoisomeric or tautomeric forms of the compound of the formula I and/or mixtures of these forms in any ratio, and/or a physiologically compatible salt of the compound of the formula I, where Ar is a fused benzene, pyridine, pyrimidine, pyridazine or pyrazine ring, where the fused ring is unsubstituted or mono-, di-, tri- or tetrasubstituted independently by -(C₁-C₆)-alkyl, -(C₃-C₆)-cycloalkyl, -O-(C₁-C₈)-alkyl, -O-(C₃-C₆)-cycloalkyl, -(C₀-C₄)-alkylene-C(O)-N(R11)-R12, -(C₀-C₄)-alkylene-C(O)-O-R11, -(C₀-C₄)-alkylene-C(O)-R11, -(C₀-C₄)-alkylene-N(R11)-R12, -(C₀-C₄)-alkylene-N(R11)-C(O)-R12, halogen, OH, -CN, -NO₂, -SO₂CH₃, -SO₂CF₃, -SF₅, -Si[-C₁-C₄)-alkyl]₃, -(C₁-C₆)-alkylene-O-(C₁-C₆)-alkyl, -(C₆-C₁₄)-aryl, where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -(C₄-C₁₅)-Het where Het is unsubstituted or mono-, di- or trisubstituted independently by -(C₁-C₄)-alkyl, -(C₃-C₆)-cydoalkyl, OH, -O-(C₁-C₆)-alkyl or -O-(C₃-C₆)-cycloalkyl, or -O-(C₁-C₆)-alkylene-O-(C₁-C₆)-alkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl,
-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl,
where all or some of the hydrogen atoms in alkyl, alkylene or cycloalkyl may be replaced by fluorine,
Q1 is a hydrogen atom, -(C₁-C₆)-alkyl, -(C₃-C₆)-cycloalkyl, -OH, -O-(C₁-C₆)-alkyl or -O-(C₃-C₆)-cycloalkyl, where alkyl and cycloalkyl are each unsubstituted or mono-, di- or trisubstituted independently by -(C₁-C₄)-alkyl, -(C₃-C₆)-cycloalkyl, OH, -O-(C₁-C₆)-alkyl or -O-(C₃-C₆)-cycloalkyl, where all or some of the hydrogen atoms in alkyl or cycloalkyl may be replaced by fluorine,
Q2 and Q3 are the same or different and each independently a hydrogen atom, -(C₁-C₆)-alkyl or -(C₃-C₆)-cycloalkyl, where all or some of the hydrogen atoms in alkyl or cycloalkyl may be replaced by fluorine,
R11 and R12 are each independently a hydrogen atom, -(C₁-C₆)-alkyl, -(C₃-C₆)-cycloalkyl, -(C₀-C₄)-alkylene-(C₆-C₁₄)-aryl, -(C₀-C₄)-alkylene-(C₄-C₁₅)-Het, -SO₂CH₃ or -SO₂CF₃,
where all or some of the hydrogen atoms in alkyl, alkylene or cycloalkyl may be replaced by fluorine, or
R11 and R12 in the "N(R11)-R12" and "N(R11)-C(O)-R12" fragments are a 5- to 8-membered ring which is formed together with the nitrogen atom "N" or the "N-C(O)" group to form cyclic amines, imides or lactams which contain up to 2 further heteroatoms from the group of N, O and S, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where all or some of the hydrogen atoms in alkyl or cycloalkyl may be replaced by fluorine,
R1, R2, R3, R4 and R5 are the same or different and are each independently a hydrogen atom, -(C₁-C₆)-alkyl, -(C₃-C6)-cycloalkyl, OH, -CN, -NO₂, -O-(C₁-C₆)-alkyl, -O-(C₃-C₆)-cycloalkyl, -(C₀-C₄)-alkylene-(CO)-N(R21)-R22, -SO₂CH₃, -SO₂CF₃, -(C₀-C₄)-alkylene-C(O)-O-R21, halogen, -SF₅, -(C₀-C₄)-alkylene-C(O)-R21, -(C₀-C₄)-alkylene-N(R21)-R22, -(C₀-C₄)-alkylene-N(R21)-C(O)-R22, -(C₁-C₆)-alkylene-O-(C₁-C₆)-alkyl, -O-(C₁-C₆)-alkylene-O-(C₁-C₆)-alkyl, -(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -Si[(C₁-C₄)-alkyl]₃ or -(C₄-C₁₅)-Het where Het is unsubstituted or mono-, di- or trisubstituted independently by -(C₁-C₄)-alkyl, -(C₃-C₆)-cycloalkyl, OH, -O-(C₁-C₆)-alkyl or -O-(C₃-C₆)-cycloalkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆)-cycloalkyl, OH, - O-(C₁-C₆)-alkyl or -O-(C₃-C₆)-cycloalkyl,
-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl,
where all or some of the hydrogen atoms in alkyl, alkylene or cycloalkyl may be replaced by fluorine,
with the proviso that at least one R1, R2, R3, R4 or R5 is -SF₅,
R9 is a hydrogen atom, -(C₁-C₆)-alkyl, -(C₃-C₆)-cycloalkyl, -(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl,
-(C₀-C₄)-alkylene-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, and where all or some of the hydrogen atoms in alkyl, alkylene or cycloalkyl may be replaced by fluorine,
R21 and R22 are each independently a hydrogen atom, -(C₁-C₆)-alkyl, -(C₃-C₆)-cycloalkyl, -(C₀-C₄)-alkylene-(C₆-C₁₄)-aryl, -(C₀-C₄)-alkylene-(C₄-C₁₅)-Het, -SO₂CH₃ or -SO₂CF₃,
where some or all of the hydrogen atoms in alkyl, alkylene or cycloalkyl may be replaced by fluorine, or
R21 and R22 in the "N(R21)-R22" and "N(R21)-C(O)-R22" fragments are a 5- to 8-membered ring which is formed together with the nitrogen atom "N" or the "N-C(O)" group to form cyclic amines, imides or lactams which contain up to two further heteroatoms from the group of N, O and S, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine,
where the radical "-(C₄-C₁₅)-Het" is a ring system which has 4 to 15 carbon atoms and is present in one, two or three ring systems joined to one another and which, according to ring size, contains one, two,
three or four identical or different heteroatoms from the group of oxygen, nitrogen and sulfur.

2. A compound of the formula I as claimed in claim 1, where
Ar is a fused benzene or pyridine ring,
where the fused ring is unsubstituted or mono-, di-, tri- or tetrasubstituted independently by -(C₁-C₆)-alkyl, -(C₃-C₆)-cycloalkyl, OH, -CN, -O-(C₁-C₈)-alkyl, -O-(C₃-C₆)-cycloalkyl, -(C₀-C₄)-alkylene-C(O)-N(R11)-R12, -(C₀-C₄)-alkylene-C(O)-O-R11, -(C₀-C₄)-alkylene-C(O)-R11, SO₂CH₃, -(C₀-C₄)-alkylene-N(R11)-R12, -(C₀-C₄)-alkylene-N(R11)-C(O)-R12, halogen, -SO₂CF₃, -Si[(C₁-C₄)-alkyl]₃, - (C₁-C₆)-alkylene-O-(C₁-C₆)-alkyl, -(C₆-C₁₄)-aryl, where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -(C₄-C₁₅)-Het where Het is unsubstituted or mono-, di- or trisubstituted independently by -(C₁-C₄)-alkyl, -(C₃-C₆)-cycloalkyl, OH, -O-(C₁-C₆)-alkyl or -O-(C₃-C₆)-cycloalkyl, or -O-(C₁-C₆)-alkylene-O-(C₁-C₆)-alkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl,
-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl,
where all or some of the hydrogen atoms in alkyl, alkylene or cycloalkyl may be replaced by fluorine,
Q1, Q2 and Q3 are the same or different and each independently a hydrogen atom, -(C₁-C₆)-alkyl or -(C₃-C₆)-cycloalkyl, where all or some of the hydrogen atoms in alkyl or cycloalkyl may be replaced by fluorine,
R11 and R12 are each independently a hydrogen atom, -(C₁-C₆)-alkyl, -(C₃-C₆)-cycloalkyl, -SO₂CH₃ or -SO₂CF₃,
where all or some of the hydrogen atoms in alkyl, alkylene or cycloalkyl may be replaced by fluorine, or
R11 and R12 in the "N(R11)-R12" and "N(R11)-C(O)-R12" fragments are a 5- to 8-membered ring which is formed together with the nitrogen atom "N" or the "N-C(O)" group to form cyclic amines, imides or lactams which contain up to 2 further heteroatoms from the group of N, O and S, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where all or some of the hydrogen atoms in alkyl or cycloalkyl may be replaced by fluorine,
R1, R2, R3, R4 and R5 are the same or different and are each independently a hydrogen atom, -(C₁-C₆)-alkyl, -(C₃-C₆)-cycloalkyl, OH, -CN, -NO₂, -O-(C₁-C₆)-alkyl, -O-(C₃-C₆)-cycloalkyl, -(C₀-C₄)-alkylene-(CO)-N(R21)-R22, -SO₂CH₃, -SO₂CF₃, -(C₀-C₄)-alkylene-C(O)-O-R21, halogen, -SF₅, -(C₀-C₄)-alkylene-C(O)-R21, -(C₀-C₄)-alkylene-N(R21)-R22, -(C₀-C₄)-alkylene-N(R21)-C(O)-R22, -(C₁-C₆)-alkylene-O-(C₁-C₆)-alkyl, -O-(C₁-C₆)-alkylene-O-(C₁-C₆)-alkyl, -(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, -Si[(C₁-C₄)-alkyl]₃ or -(C₄-C₁₅)-Het where Het is unsubstituted or mono-, di- or trisubstituted independently by -(C₁-C₄)-alkyl, -(C₃-C₆)-cycloalkyl, OH, -O-(C₁-C₆)-alkyl or -O-(C₃-C₆)-cycloalkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C1-C₄)-alkyl, -(C₃-C₆)-cycloalkyl, OH, - O-(C₁-C₆)-alkyl or -O-(C₃-C₆)-cycloalkyl,
-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl,
where all or some of the hydrogen atoms in alkyl, alkylene or cycloalkyl may be replaced by fluorine,
with the proviso that at least one R1, R2, R3, R4 or R5 is -SF₅,
R9 is a hydrogen atom, -(C₁-C₆)-alkyl, -(C₃-C₆)-cycloalkyl, -(C₀-C₄)-alkylene-(C₆-C₁₄)-aryl where aryl is unsubstituted or mono-, di- or trisubstituted independently by -O-(C₁-C₆)-alkyl, -(C₁-C₄)-alkyl, OH, -(C₃-C₆)-cycloalkyl or -O-(C₃-C₆)-cycloalkyl, or -(C_{O}-C₄)-alkylene-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, and where all or some of the hydrogen atoms in alkyl, alkylene or cycloalkyl may be replaced by fluorine,
R21 and R22 are each independently a hydrogen atom, -(C₁-C₆)-alkyl, -(C₃-C₆)-cycloalkyl, -(C₀-C₄)-alkylene-(C₆-C₁₄)-aryl, -(C₀-C₄)-alkylene-(C₄-C₁₅)-Het, -SO₂CH₃ or -SO₂CF₃,
where some or all of the hydrogen atoms in alkyl, alkylene or cycloalkyl may be replaced by fluorine, or
R21 and R22 in the "N(R21)-R22" and "N(R21)-C(O)-R22" fragments are a 5- to 8-membered ring which is formed together with the nitrogen atom "N" or the "N-C(O)" group to form cyclic amines, imides or lactams which contain up to two further heteroatoms from the group of N, O and S, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine,
where the radical "-(C₄-C₁₅)-Het" is a ring system which has 4 to 15 carbon atoms and is present in one, two or three ring systems joined to one another and which, according to ring size, contains one, two, three or four identical or different heteroatoms from the group of oxygen, nitrogen and sulfur.

3. A compound of the formula I as claimed in claim 1 or 2, where Ar is a fused benzene or pyridine ring,
where the fused ring is unsubstituted or mono-, di-, tri- or tetrasubstituted independently by -(C₁-C₆)-alkyl, -(C₃-C₆)-cycloalkyl, OH, -CN, -O-(C₁-C₈)-alkyl, -O-(C₃-C₆)-cycloalkyl, -(C₀-C₄)-alkylene-C(O)-N(R11)-R12, -(C₀-C₄)-alkylene-C(O)-O-R11, -(C₀-C₄)-alkylene-C(O)-R11, SO₂CH₃, -(C₀-C₄)-alkylene-N(R11)-R12, -(C₀-C₄)-alkylene-N(R11)-C(O)-R12, halogen, -Si[(C₁-C₄)-alkyl]₃, -(C₁-C₆)-alkylene-O-(C₁-C₆)-alkyl or -O-(C₁-C₆)-alkylene-O-(C₁-C₆)-alkyl,
where alkyl, alkylene and cycloalkyl are each unsubstituted or mono-, di- or trisubstituted independently by -(C₁-C₄)-alkyl, -(C₃-C₆)-cycloalkyl, OH, -O-(C₁-C₆)-alkyl, -(C₆-C₁₄)-aryl where aryl is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl,
-(C₄-C₁₅)-Het where Het is unsubstituted or mono-, di-, tri-, tetra- or pentasubstituted independently by halogen, -(C₁-C₄)-alkyl, -(C₃-C₆)-cycloalkyl, OH, -O-(C₁-C₆)-alkyl or -O-(C₃-C₆)-cycloalkyl, or -O-(C₃-C₆)-cycloalkyl,
where all or some of the hydrogen atoms in alkyl, alkylene or cycloalkyl may be replaced by fluorine,
Q1, Q2 and Q3 are the same or different and each independently a hydrogen atom, -(C₁-C₆)-alkyl or -(C₃-C₆)-cycloalkyl, where all or some of the hydrogen atoms in alkyl or cycloalkyl may be replaced by fluorine,
R11 and R12 are each independently a hydrogen atom, -(C₁-C₆)-alkyl, -(C₃-C₆)-cycloalkyl or -SO₂CH₃,
where all or some of the hydrogen atoms in alkyl, alkylene or cycloalkyl may be replaced by fluorine, or
R11 and R12 in the "N(R11)-R12" and "N(R11)-C(O)-R12" fragments are a 5- to 8-membered ring which is selected from the group of azetidinyl, pyrrolidinyl, piperidinyl, piperazinyl, azepinyl, morpholinyl, thiomorpholinyl, pyrrolidine-2,5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl, where the ring is unsubstituted or mono- or disubstituted independently by - (C₁-C₄)-alkyl, -(C₃-C₆)-cycloalkyl, OH, -O-(C₁-C₆)-alkyl or -O-(C₃-C₆)-cycloalkyl, where all or some of the hydrogen atoms in alkyl or cycloalkyl may be replaced by fluorine,
R1, R2, R3, R4 and R5 are the same or different and are each independently a hydrogen atom, -(C₁-C₆)-alkyl, -(C₃-C₆)-cycloalkyl, OH, -CN, -NO₂, -O-(C₁-C₆)-alkyl, -O-(C₃-C₆)-cycloalkyl, -(C₀-C₄)-alkylene-(CO)-N(R21)-R22, -SO₂CH₃, -(C₀-C₄)-alkylene-C(O)-O-R21, halogen, -SF₅, -(C₀-C₄)-alkylene-C(O)-R21, -(C₀-C₄)-alkylene-N(R21)-R22, -(C₀-C₄)-alkylene-N(R21)-C(O)-R22, -(C₁-C₆)-alkylene-O-(C₁-C₆)-alkyl, -O-(C₁-C₆)-alkylene-O-(C₁-C₆)-alkyl, -Si[(C₁-C₄)-alkyl]₃ or -(C₄-C₁₅)-Het,
where alkyl, alkylene and cycloalkyl are each unsubstituted or mono-, di- or trisubstituted independently by -(C₁-C₄)-alkyl, OH,-(C₃-C₆)-cycloalkyl, -O-(C₁-C₆)-alkyl or -O-(C₃-C₆)-cycloalkyl, where all or some of the hydrogen atoms in alkyl or cycloalkyl may be replaced by fluorine,
with the proviso that one R1, R2, R3, R4 or R5 is -SF₅,
R9 is a hydrogen atom, -(C₁-C₆)-alkyl or -(C₃-C₆)-cycloalkyl, where all or some of the hydrogen atoms in alkyl or cycloalkyl may be replaced by fluorine,
R21 and R22 are each independently a hydrogen atom, -(C₁-C₆)-alkyl, -(C₃-C₆)-cycloalkyl, -(C₀-C₄)-alkylene-(C₆-C₁₄)-aryl, -(C₀-C₄)-alkylene-(C₄-C₁₅)-Het, -SO₂CH₃ or -SO₂CF₃,
where some or all of the hydrogen atoms in alkyl, alkylene or cycloalkyl may be replaced by fluorine, or
R21 and R22 in the "N(R21)-R22" and "N(R21)-C(O)-R22" fragments are a 5- to 8-membered ring selected from the group of azetidinyl, pyrrolidinyl, piperidinyl, piperazinyl, azepinyl, morpholinyl, thiomorpholinyl, pyrrolidine-2,5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl, where the ring is unsubstituted or mono- or disubstituted independently by -(C₁-C₄)-alkyl, -(C₃-C₆)-cycloalkyl, OH, -O-(C₁-C₆)-alkyl or -O-(C₃-C₆)-cycloalkyl, where some or all of the hydrogen atoms in alkyl or cycloalkyl may be replaced by fluorine,
where the radical "-(C₄-C₁₅)-Het" is a ring system which has 4 to 15 carbon atoms and is present in one, two or three ring systems joined to one another and which, according to ring size, contains one, two, three or four identical or different heteroatoms from the group of oxygen, nitrogen and sulfur.

4. A compound of the formula I as claimed in any of claims 1 to 3, where
Ar is a fused benzene ring,
where the fused ring is unsubstituted or mono- or disubstituted independently by -O-(C₁-C₆)-alkyl, -C(O)-N(R11)-R12 or halogen, Q1, Q2 and Q3 are the same and are each a hydrogen atom,
R11 and R12 are each independently a hydrogen atom or -(C₁-C₆)-alkyl,
R1, R2, R3, R4 and R5 are the same or different and are each independently a hydrogen atom, -(C₁-C₆)-alkyl, -O-(C₁-C₆)-alkyl, halogen, -SF₅, -(CO-C₄)-alkylene-N(R21)-R22, -O-(C₁-C₆)-alkylene-O-(C₁-C₆)-alkyl or -(C₀-C₄)-alkylene-N(R21)-C(O)-R22,
with the proviso that one R1, R2, R3, R4 or R5 is -SF₅,
R9 is a hydrogen atom,
R21 and R22 are each independently a hydrogen atom, -(C₁-C₆)-alkyl, -(C₃-C₆)-cycloalkyl, CF₃ or -SO₂CH₃,
where some or all of the hydrogen atoms in alkyl, alkylene or cycloalkyl may be replaced by fluorine, or
R21 and R22 in the "N(R21)-R22" fragment are a ring selected from the group of azetidinyl, pyrrolidinyl, piperidinyl, piperazinyl, azepinyl, morpholinyl, thiomorpholinyl, pyrrolidine-2,5-dionyl, piperidine-2,6-dionyl, piperazine-2,6-dionyl, morpholine-3,5-dionyl, pyrrolidin-2-onyl, piperidin-2-onyl, piperazin-2-onyl and morpholin-3-onyl.

5. A compound of the formula I as claimed in any of claims 1 to 4, where the compound of the formula I is selected from the group of 2-(1-imino-1,3-dihydroisoindol-2-yl)-1-(3-pentafluorosulfanylphenyl)ethanone as the hydrobromide, N-methyl-2-[2-(3-dimethylamino-5-pentafluorosulfanylphenyl)-2-oxoethyl]-6-ethoxy-3-imino-2,3-dihydro-1H-isoindole-5-carboxamide, N-methyl-6-ethoxy-3-imino-2-[2-(3-methylamino-5-pentafluorosulfanylphenyl)-2-oxoethyl]-2,3-dihydro-1H-isoindole-5-carboxamide, N-methyl-6-ethoxy-3-imino-2-{2-[3-pentafluorosulfanyl-5-(2,2,2-trifluoroacetylamino)phenyl]-2-oxoethyl}-2,3-dihydro-1H-isoindole-5-carboxamide, N-methyl-6-ethoxy-2-(2-{3-[ethyl-(2,2,2-trifluoroacetyl)amino]-5-pentafluorosulfanylphenyl}-2-oxoethyl)-3-imino-2,3-dihydro-1H-isoindole-5-carboxamide, N-methyl-6-ethoxy-2-[2-(3-ethylamino-5-pentafluorosulfanylphenyl)-2-oxoethyl]-3-imino-2,3-dihydro-1H-isoindole-5-carboxamide as the hydrochoride, N-methyl-2-[2-(3-amino-5-pentafluorosulfanylphenyl)-2-oxoethyl]-6-ethoxy-3-imino-2,3-dihydro-1H-isoindole-5-carboxamide, N-methyl-2-[2-(3-acetylamino-5-pentafluorosulfanylphenyl)-2-oxoethyl)-6-ethoxy-3-imino-2,3-dihydro-1H-isoindole-5-carboxamide, 2-(5,6-diethoxy-7-fluoro-1-imino-1,3-dihydroisoindol-2-yl)-1-[3-methylamino-5-(pentamethylsulfanyl)phenyl]ethanone, N-methyl-6-ethoxy-3-imino-2-{2-[3-dimethanesulfonylamino-5-(pentafluorosulfanyl)phenyl]-2-oxoethyl)-2,3-dihydro-1H-isoindole-5-carboxamide, N-methyl-2-{2-[3-(acetylmethylamino)-5-(pentafluorosulfanyl)phenyl]-2-oxoethyl}-6-ethoxy-3-imino-2,3-dihydro-1H-isoindole-5-carboxamide, N-[3-[2-(5,6-diethoxy-7-fluoro-1-imino-1,3-dihydroisoindol-2-yl)acetyl]-5-(pentafluorosulfanyl)phenyl]-N-methylacetamide, N-[3-[2-(5,6-diethoxy-7-fluoro-1-imino-1,3-dihydroisoindol-2-yl)acetyl]-5-(pentafluorosulfanyl)phenyl]isobutyramide, N-[3-[2-(5,6-diethoxy-7-fluoro-1-imino-1,3-dihydroisoindol-2-yl)acetyl]-5-(pentafluoro-sulfanyl)phenyl]acetamide as the hydrobromide, N-[5-[2-(5,6-diethoxy-7-fluoro-1-imino-1,3-dihydroisoindol-2-yl)acetyl]-4-methyl-2-(pentafluorosulfanyl)phenyl]acetamide, 1-[5-amino-2-methyl-4-(pentafluorosulfanyl)phenyl]-2-(5,6-diethoxy-7-fluoro-1-imino-1,3-dihydroisoindol-2-yl)ethanone, N-{3-[2-(5,6-diethoxy-7-fluoro-1-imino-1,3-dihydroisoindol-2-yl)acetyl]-5-(pentafluorosulfanylphenyl}-3,3-dimethylbutyramide, 1-{3-[2-(5,6-diethoxy-7-fluoro-1-imino-1,3-dihydroisoindol-2-yl)acetyl]-5-pentafluorosulfanylphenyl}pyrrolidine-2,5-dione, 1-[2-amino-4-(pentafluorosulfanyl)phenyl]-2-(5,6-diethoxy-7-fluoro-1-imino-1,3-dihydroisoindo-2-yl)ethanone, 1-[2-amino-5-bromo-4-(pentafluorosulfanyl)phenyl]-2-(5,6-diethoxy-7-fluoro-1-imino-1,3-dihydroisoindol-2-yl)ethanone, 2-(5,6-diethoxy-7-fluoro-1-imino-1,3-dihydroisoindol-2-yl)-1-[3-methoxy-5-(pentafluorosulfanyl)-phenyl]ethanone, N-[3-[2-(5,6-diethoxy-7-fluoro-1-imino-1,3-dihydroisoindol-2-yl)acetyl]-5-(pentafluorosulfanyl)phenyl]cyclo-butanecarboxamide as the hydrochloride, N-[3-[2-(5,6-diethoxy-7-fluoro-1-imino-1,3-dihydroisoindol-2-yl)acetyl]-5-(pentafluorosulfanyl)phenyl]cyclopropanecarboxamide as the hydrochloride, 2-(5,6-diethoxy-7-fluoro-1-imino-1,3-dihydroisoindol-2-yl)-1-[3-ethoxy-5-(pentafluorosulfanyl)phenyl]ethanone, N-methyl-6-ethoxy-3-imino-2-{2-[3-methoxy-5-(pentafluorosulfanyl)phenyl]-2-oxoethyl}-2,3-dihydro-1H-isoindole-5-carboxamide, 1-[3-bromo-5-(pentafluorosulfanyl)phenyl]-2-(5,6-diethoxy-7-fluoro-1-imino-1,3-dihydroisoindol-2-yl)ethanone, 1-[3-chloro-5-(pentafluorosulfanyl)phenyl]-2-(5,6-diethoxy-7-fluoro-1-imino-1,3-dihydroisoindol-2-yl)ethanone, 1-[2-bromo-4-(pentafluorosulfanyl)phenyl]-2-(5,6-diethoxy-7-fluoro-1-imino-1,3-dihydroisoindol-2-yl)ethanone, 1-[2-chloro-4-(pentafluorosulfanyl)phenyl]-2-(5,6-diethoxy-7-fluoro-1-imino-1,3-dihydroisoindol-2-yl)ethanone, 2-(5,6-diethoxy-7-fluoro-1-imino-1,3-dihydroisoindol-2-yl)-1-[3-morpholin-4-yl-5-(pentafluorosulfanyl)phenyl]ethanone or 2-(5,6-diethoxy-7-fluoro-1-imino-1,3-dihydroisoindol-2-yl)-1-[3-(2-methoxyethoxy)-5-(pentafluorosulfanyl)phenyl]ethanone as the hydrochloride.

6. A medicament, **characterized by** an effective content of at least one compound of the formula I as claimed in one or more of claims 1 to 5 together with a pharmaceutically suitable and physiologically compatible carrier, additive and/or other active ingredients and excipients.

7. The use of the compound of the formula I as claimed in one or more of claims 1 to 5 for producion of a medicament for prophylaxis, secondary prevention and therapy of all those disorders associated with thromboses, embolisms, hypercoagulability, fibrotic changes or inflammatory disorders.

8. The use as claimed in claim 7, which is applied to myocardial infarction, angina pectoris and other types of acute coronary syndrome, stroke, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, or reduction of the risk of thrombosis following surgical procedures such as knee and hip joint operations or procedures leading to contact of blood with foreign surfaces, such as for dialysis patients and patients with indwelling catheters or disseminated intravascular coagulation, sepsis and other intravascular events associated with inflammation, atherosclerosis, diabetes and metabolic syndrome and the sequelae thereof, tumor growth and tumor metastasis, inflammatory and degenerative articular disorders such as rheumatoid arthritis and arthrosis, impairments of the hemostatic system such as fibrin deposits, fibrotic changes in the lung such as chronic obstructive pulmonary disease, adult respiratory distress syndrome or fibrin deposits in the eye following eye operations or prevention and/or treatment of scarring.

9. A process for preparing the compound of the formula I as claimed in one or more of claims 1 to 5, which comprises
a) reacting a compound of the formula II 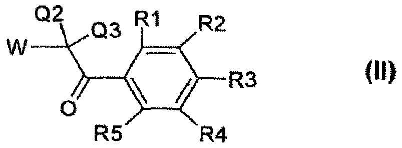 wherein R1, R2, R3, R4, R5, Q2 and Q3 are each as defined in formula I and W is chloride, bromide, mesylate or tosylate with a compound of the formula III 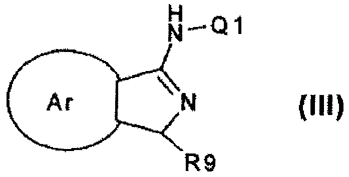 where Ar, R9 and Q1 are each as defined in formula I, with or without addition of base, in a solvent, to give a compound of the formula I, or
b) reacting a compound of the formula VII 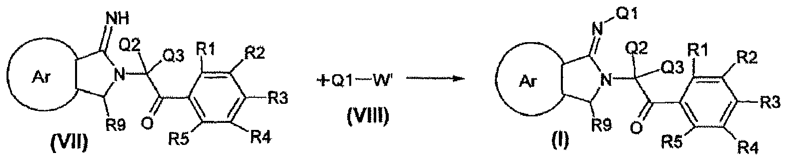 where R1, R2, R3, R4, R5, R9, Ar, Q1, Q2 and Q3 are each as defined in formula I with the compound Q1-W' where W' is chloride, bromide, mesylate, tosylate, methylsulfate or a similarly good leaving group, with or without addition of base, to give a compound of the formula I, or
c) either isolating the compound of the formula I prepared by process a) or b) in free form or releasing it from physiologically unacceptable salts or, in the case of presence of acidic or basic groups, converting them to physiologically acceptable salts, or
d) separating a compound of the formula I prepared by process a) or b), or a suitable precursor of the formula I, which, owing to its chemical structure, occurs in enantiomeric or diastereomeric forms into the pure enantiomers or diastereomers by salt formation with enantiomerically pure acids or bases, chromatography on chiral stationary phases or derivatization by means of chiral enantiomerically pure compounds such as amino acids, separation of the diastereomers thus obtained, and detachment of the chiral auxiliary groups.

10. A process for preparing the compound of the formula II as claimed in claim 9, where W is Br, which comprises
a) converting a compound of the formula X 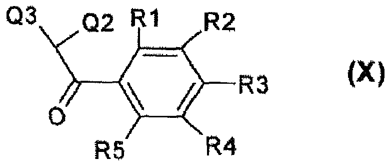 where R1, R2, R3, R4, R5, Q2 and Q3 are each as defined in formula I with a brominating reagent to the compound of the formula II, or
b) treating a compound of the formula XI or XI' 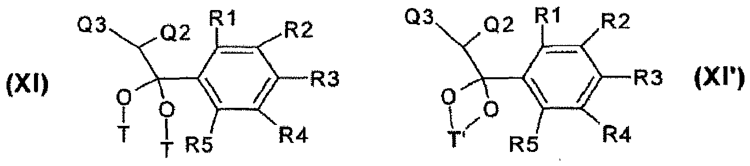 where R1, R2, R3, R4, R5, Q2 and Q3 are each as defined in formula I, T is -(C₁-C₄)-alkyl and T' is ethylene, propylene or butylene or, together with the -O-C-O- group, forms a 1,3-dioxo ring of ring size 5, 6 or 7, with a brominating reagent and then converting it to the compound of the formula II in the presence of an acid.

11. A process for preparing the compound of the formula X as claimed in claim 10, where R4 is dimethylamine and R1, R2, R3, R5, Q2 and Q3 are each as defined in formula I, which comprises
a) first nitrating a compound of the formula XIVa 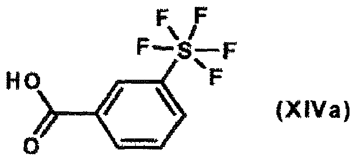 and then reducing it with hydrogen to give the amine of the formula XIIIa 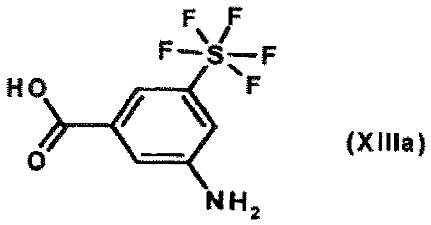
b) dimethylating the compound of the formula XIIIa obtained on the nitrogen and converting the carboxylic acid with thionyl chloride to the acid chloride, and then reacting it with O,N-dimethylhydroxylamine to give a compound of the formula XIIa 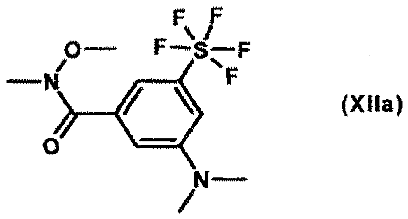 and
c) converting the compound of the formula XIIa obtained with methylmagnesium bromide to the compound of the formula Xa

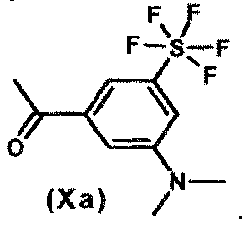

12. A process for preparing the compound of the formula X as claimed in claim 10, where R4 is methoxy and R1, R2, R3, R5, Q2 and Q3 are each as defined in formula I, which comprises
a) first diazotizing the compound of the formula XIIIa as claimed in claim 11 and then hydrolyzing it to the phenol XIIIb, 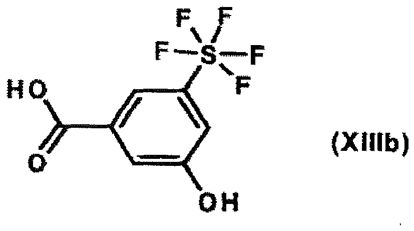
b) methylating the compound of the formula XIIIb obtained, converting it to the Weinreb amide and then converting the latter with methylmagnesium bromide to the compound Xb

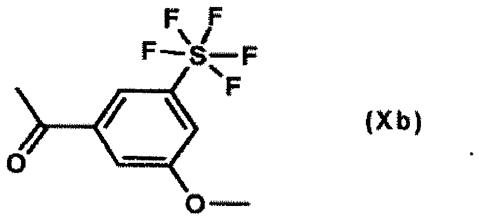

## Revendications

1. Composé de formule I 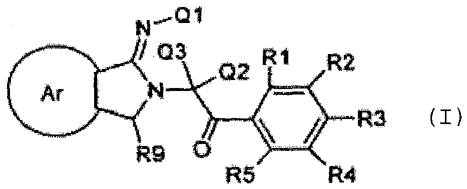 et/ou toutes formes tautomères ou stéréoisomères du composé de formule I et/ou mélanges de ces formes et tout rapport, et/ou se1 physiologiquement acceptable du composé de formule I, dans laquelle
Ar représente un cycle benzénique, pyridine, pyrimidine, pyridazine ou pyrazine soudé, le cycle soudé étant non substitué ou une, deux, trois ou quatre fois substitué chaque fois indépendamment par -alkyle en C₁-C₆, -cycloalkyle en -C₃-C₆, -O-alkyle(C₁-C₈), -O-cycloalkyle(C₃-C₆), -alkylène(C₀-C₄)-C(O)-N(R11)-R12, -alkylène (C₀-C₄)-C(O)-O-R11, -alkylène(C₀-C₄)-C(O)-R11, -alkylène(C₀-C₄)-N(R11)-R12, -alkylène(C₀-C₄)-N(R11)-C(O)-R12, halogène, OH, -CN, -NO₂, -SO₂CH₃-, -SO₂CF₃, -SF₅, -Si[alkyle(C₁-C₄)]₃, -alkylène(C₁-C₆)-O-alkyle(C₁-C₆), -aryle en C₆-C₁₄, le radical aryle étant non substitué ou portant un, deux ou trois substituants, indépendamment les uns des autres, -O-alkyle(C₁-C₆), -alkyle en C₁-C₄, OH, -cycloalkyle en C₃-C₆ ou -O-cycloalkyle(C₃-C₆), par -Het(C₄-C₁₅), Het étant non substitué ou portant un, deux ou trois substituants, indépendamment les uns des autres, -alkyle en C₁-C₄, -cycloalkyle en C₃-C₆, OH, -O-alkyle(C₁-C₆) ou -O-cycloalkyle(C₃-C₆), ou par -O-alkylène(C₁-C₆)-O-alkyle(C₁-C₆), les groupes alkyle, alkylène et cycloalkyle étant chacun non substitué ou une, deux ou trois fois substitué, indépendamment les uns des autres, par -alkyle en C₁-C₄, -cycloalkyle en C₃-C₆, OH, -O-alkyle(C₁-C₆), -aryle en C₆-C₁₄, le radical aryle étant non substitué ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆),
par -Het(C₄-C₁₅), Het étant non substitué ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆), ou par -O-cycloalkyle(C₃-C₆), dans les radicaux alkyle, alkylène ou cycloalkyle les atomes d'hydrogène pouvant être en partie ou en totalité remplacés par le fluor,
Q1 représente un atome d'hydrogène, un groupe -alkyle en C₁-C₆, -cycloalkyle en C₃-C₆, -OH, -O-alkyle(C₁-C₆) ou -O-cycloalkyle(C₃-C₆), les radicaux alkyle et cycloalkyle étant chacun non substitué ou une, deux ou trois fois substitué, indépendamment les uns des autres, par -alkyle en C₁-C₄, -cycloalkyle en C₃-C₆, OH, -O-alkyle(C₁-C₆) ou -O-cycloalkyle(C₃-C₆), dans les radicaux alkyle ou cycloalkyle les atomes hydrogène pouvant être en partie ou en totalité remplacés par le fluor, Q2 et Q3 sont identiques ou différents et représentent indépendamment l'un de l'autre un atome d'hydrogène, un groupe -alkyle en C₁-C₆ ou -cycloalkyle en C₃-C_{6,} dans les radicaux alkyle ou cycloalkyle les atomes d'hydrogène pouvant être en partie ou en totalité remplacés par le fluor, R11 et R12 représentent indépendamment l'un de l'autre un atome d'hydrogène, un groupe -alkyle en C₁-C₆, -cycloalkyle en C₃-C₆, -alkylène(C₀-C₄)-aryle(C₆-C₁₄), -alkylène(C₀-C₄)-Het(C₄-C₁₅), -SO₂CH₃ ou -SO₂CF₃,
dans les radicaux alkyle, alkylène ou cycloalkyle les atomes d'hydrogène pouvant être en partie ou en totalité remplacés par le fluor, ou R11 et R12 dans les fragments « N(R11)-R12 » et « N(R11)-C(O)-R12 » représentent un cycle à 5-8 chaînons, qui est formé conjointement avec l'atome d'azote « N » ou le groupe « N-C(O) », de sorte que sont formés des amines, imides ou lactames cycliques, qui contiennent jusqu'à 2 autres hétéroatomes choisis dans l'ensemble constitué par N, O ou S, le cycle étant non substitué ou portant un ou deux substituants, indépendamment l'un de l'autre, -alkyle en C₁-C₄, -cycloalkyle en C₃-C₆, OH, -O-alkyle(C₁-C₆) ou -O-cycloalkyle(C₃-C₆), dans les radicaux alkyle ou cycloalkyle les atomes d'hydrogène pouvant être en partie ou en totalité remplacés par le fluor,
R1, R2, R3, R4 et R5 sont identiques ou différents et représentent indépendamment les uns des autres un atome d'hydrogène, un groupe -alkyle en C₁-C₆, -cycloalkyle en C₃-C₆, OH, -CN, -NO₂, -O-alkyle(C₁-C₆) ou -O-cycloalkyle(C₃-C₆), -alkylène(C₀-C₄)-(CO)-N(R21)-R22, -SO₂CH₃, -SO₂CF₃, -alkylène(C₀-C₄)-C(O)-O-R21, un atome d'halogène, un groupe -SF₅, -alkylène(C₀-C₄)-C(O)-R21, -alkylène(C₀-C₄)-N(R21)-R22, -alkylène(C₀-C₄)-N(R21)-C(O)-R22, -alkylène(C₁-C₆)-O-alkyle(C₁-C₆), -O-alkylène(C₁-C₆)-O-alkyle(C₁-C₆), -aryle en C₆-C₁₄, le radical aryle étant non substitué ou portant un, deux ou trois substituants, indépendamment les uns des autres, -O-alkyle(C₁-C₆), -alkyle en C₁-C₄, OH, -cycloalkyle en C₃-C₆ ou -O-cycloalkyle(C₃-C₆), un groupe -Si[alkyle(C₁-C₄)]₃ ou -Het(C₄-C₁₅), Het étant non substitué ou portant un, deux ou trois substituant, indépendamment les uns des autres, -alkyle en C₁-C₄, -cycloalkyle en C₃-C₆, OH, -O-alkyle(C₁-C₆) ou -O-cycloalkyle(C₃-C₆),
les radicaux alkyle, alkylène et cycloalkyle étant chacun non substitué ou une, deux ou trois fois substitué, indépendamment les uns des autres, par -alkyle en C₁-C₄, -cycloalkyle en C₃-C₆, OH, -O-alkyle(C₁-C₆), -aryle en C₆-C₁₄, les radicaux aryle étant non substitués ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆),
par -Het(C₄-C₁₅), Het étant non substitué ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆), ou par O-cycloalkyle(C₃-C₆), dans les radicaux alkyle, alkylène ou cycloalkyle les atomes d'hydrogène pouvant être en partie ou en totalité remplacés par le fluor,
étant entendu qu'au moins un symbole R1, R2, R3, R4 ou R5 représente -SF₅, R9 représente un atome d'hydrogène, un groupe -alkyle en C₁-C₆, -cycloalkyle en C₃-C₆, -alkylène(C₀-C₄)-aryle(C₆-C₁₄), le radical aryle étant non substitué ou portant un, deux ou trois substituants, indépendamment les uns des autres, -O-alkyle(C₁-C₆), -alkyle en C₁-C₄, OH, -cycloalkyle en C₃-C₆ ou -O-cycloalkyle(C₃-C₆), un groupe -alkylène(C₀-C₄)-Het(C₄-C₁₅), Het étant non substitué ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆), et dans les radicaux alkyle, alkylène ou cycloalkyle les atomes d'hydrogène pouvant être en partie ou en totalité remplacés par le fluor, R21 et R22 représentent indépendamment l'un de l'autre un atome d'hydrogène, un groupe -alkyle en C₁-C₆, -cycloalkyle en C₃-C₆, -alkylène(C₀-C₄)-aryle(C₆-C₁₄), -alkylène(C₀-C₄)-Het(C₄-C₁₅), -SO₂CH₃ ou -SO₂CF₃,
dans les radicaux alkyle, alkylène ou cycloalkyle les atomes d'hydrogène pouvant être en partie ou en totalité remplacés par le fluor, ou R21 et R22 dans les fragments « N(R21)-R22 » et « N(R21)-C(O)-R22 » représentent un cycle à 5-8 chaînons, qui est formé conjointement avec l'atome d'azote « N » ou le groupe « N-C(O) », de sorte que sont formés des amines, imides ou lactames cycliques, qui contiennent jusqu'à 2 autres hétéroatomes choisis dans l'ensemble constitué par N, O ou S, le cycle étant non substitué ou portant un ou deux substituants, indépendamment l'un de l'autre, -alkyle en C₁-C₄, -cycloalkyle en C₃-C₆, OH, -O-alkyle(C₁-C₆) ou -O-cycloalkyle(C₃-C₆), dans les radicaux alkyle ou cycloalkyle les atomes d'hydrogène pouvant être en partie ou en totalité remplacés par le fluor,
le radical -Het(C₄-C₁₅) représentant un système cyclique ayant de 4 à 15 atomes de carbone, qui est présent dans un, deux ou trois systèmes cycliques liés entre eux et qui selon la taille du cycle contient un, deux, trois ou quatre hétéroatomes identiques ou différents, choisis dans l'ensemble constitué par les atomes d'oxygène, d'azote et de soufre.

2. Composé de formule I selon la revendication 1, dans lequel Ar représente un cycle benzénique ou pyridine soudé, le cycle soudé étant non substitué ou une, deux, trois ou quatre fois substitué chaque fois indépendamment par -alkyle en C₁-C₆, -cycloalkyle en - C₃-C₆, OH, -CN, -O-alkyle(C₁-C₈), -O-cycloalkyle(C₃-C₆), -alkylène(C₀-C₄) -C (0) -N(R11)-R12, -alkylène(C₀-C₄)-C(O)-O-R11, -alkylène(C₀-C₄)-C(O)-R11, -SO₂CH₃-, -alkylène(C₀-C₄)-N(R11)-R12, -alkylène(C₀-C₄)-N(R11)-C(O)-R12, halogène, -SO₂CF₃, -Si[alkyle(C₁-C₄)]₃, -alkylène(C₁-C₆)-O-alkyle(C₁-C₆), -aryle en C₆-C₁₄, le radical aryle étant non substitué ou portant indépendamment les uns des autres un, deux ou trois substituants -O-alkyle(C₁-C₆), -alkyle en C₁-C₄, OH, -cycloalkyle en C₃-C₆ ou -O-cycloalkyle(C₃-C₆),
par -Het(C₄-C₁₅), Het étant non substitué ou portant indépendamment les uns des autres un, deux ou trois substituants -alkyle en C₁-C₄, -cycloalkyle en C₃-C₆, OH, -o-alkyle(C₁-C₆) ou -O-cycloalkyle(C₃-C₆), ou par -O-alkylène(C₁-C₆)-O-alkyle(C₁-C₆), les groupes alkyle, alkylène et cycloalkyle étant chacun non substitué ou une, deux ou trois fois substitué, indépendamment les uns des autres, par -alkyle en C₁-C₄, -cycloalkyle en C₃-C₆, OH, -O-alkyle(C₁-C₆), -aryle en C₆-C₁₄, le radical aryle étant non substitué ou portant indépendamment les uns des autres un, deux, trois, quatre ou cinq substituants halogène, -alkyle en C₁-C₄, -cycloalkyle en C₃-C₆, OH, -O-alkyle(C₁-C₆) ou -O-cycloalkyle(C₃-C₆),
par -Het(C₄-C₁₅), Het étant non substitué ou portant indépendamment les uns des autres un, deux, trois, quatre ou cinq substituants halogène, -alkyle en C₁-C₄, -cycloalkyle en C₃-C₆, OH, -O-alkyle(C₁-C₆) ou -O-cycloalkyle(C₃-C₆), ou par -O-cycloalkyle(C₃-C₆),
dans les radicaux alkyle, alkylène ou cycloalkyle les atomes d'hydrogène pouvant être en partie ou en totalité remplacés par le fluor,
Q1, Q2 et Q3 sont identiques ou différents et représentent chacun indépendamment un atome d'hydrogène, un groupe -alkyle en C₁-C₆ ou -cycloalkyle en C₃-C₆, dans les radicaux alkyle ou cycloalkyle les atomes d'hydrogène pouvant être en partie ou en totalité remplacés par le fluor,
R11 et R12 représentent indépendamment l'un de l'autre un atome d'hydrogène, un groupe -alkyle en C₁-C₆, -cycloalkyle en C₃-C₆, -SO₂CH₃ ou -SO₂CF₃,
dans les radicaux alkyle, alkylène ou cycloalkyle les atomes d'hydrogène pouvant être en partie ou en totalité remplacés par le fluor, ou
R11 et R12 dans les fragments « N(R11)-R12 » et « N(R11)-C(O)-R12 » représentent un cycle à 5-8 chaînons, qui est formé conjointement avec l'atome d'azote « N » ou le groupe « N-C(O) », de sorte que sont formés des amines, imides ou lactames cycliques, qui contiennent jusqu'à 2 autres hétéroatomes choisis dans l'ensemble constitué par N, O ou S, le cycle étant non substitué ou portant un ou indépendamment l'un de l'autre deux substituants -alkyle en C₁-C₄, -cycloalkyle en C₃-C₆, OH, -o-alkyle(C₁-C₆) ou -O-cycloalkyle(C₃-C₆), dans les radicaux alkyle ou cycloalkyle les atomes d'hydrogène pouvant être en partie ou en totalité remplacés par le fluor, R1, R2, R3, R4 et R5 sont identiques ou différents et représentent indépendamment les uns des autres un atome d'hydrogène, un groupe -alkyle en C₁-C₆, -cycloalkyle en C₃-C₆, OH, -CN, -NO₂, -O-alkyle(C₁-C₆), -O-cycloalkyle(C₃-C₆), -alkylène(C₀-C₄)-(CO)-N(R21)-R22, -SO₂CH₃, -SO₂CF₃, -alkylène(C₀-C₄)-C(O)-O-R21, un atome d'halogène, un groupe -SF₅, -alkylène(C₀-C₄)-C(O)-R21, -alkylène(C₀-C₄)-N(R21)-R22, -alkylène(C₀-C₄)-N(R21)-C(O)-R22, -alkylène(C₁-C₆)-O-alkyle(C₁-C₆), -O-alkylène(C₁-C₆)-O-alkyle(C₁-C₆), -aryle en C₆-C₁₄, le radical aryle étant non substitué ou portant un, deux ou trois substituants, indépendamment les uns des autres, -O-alkyle(C₁-C₆), -alkyle en C₁-C₄, OH, -cycloalkyle en C₃-C₆ ou -O-cycloalkyle(C₃-C₆), un groupe -Si[alkyle(C₁-C₄)]₃ ou -Het(C₄-C₁₅), Het étant non substitué ou portant un, deux ou trois substituants, indépendamment les uns des autres, -alkyle en C₁-C₄, -cycloalkyle en C₃-C₆, OH, -O-alkyle(C₁-C₆) ou -O-cycloalkyle(C₃-C₆), les radicaux alkyle, alkylène et cycloalkyle étant chacun non substitué ou une, deux ou trois fois substitué, indépendamment les uns des autres, par -alkyle en C₁-C₄, -cycloalkyle en C₃-C₆, OH, -O-alkyle(C₁-C₆), -aryle en C₆-C₁₄, les radicaux aryle étant non substitués ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆),
par -Het(C₄-C₁₅), Het étant non substitué ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆), ou par O-cycloalkyle(C₃-C₆), dans les radicaux alkyle, alkylène ou cycloalkyle les atomes d'hydrogène pouvant être en partie ou en totalité remplacés par le fluor, étant entendu qu'au moins un symbole R1, R2, R3, R4 ou R5 représente -SF₅, R9 représente un atome d'hydrogène, un groupe -alkyle en C₁-C₆, -cycloalkyle en C₃-C₆, -alkylène(C₀-C₄)-aryle(C₆-C₁₄), le radical aryle étant non substitué ou portant un, deux ou trois substituants, indépendamment les uns des autres, -O-alkyle(C₁-C₆), -alkyle en C₁-C₄, OH, -cycloalkyle en C₃-C₆ ou -O-cycloalkyle(C₃-C₆), ou
-alkylène(C₀-C₄)-Het(C₄-C₁₅), Het étant non substitué ou portant un, deux, trois, quatre ou cinq substituants, indépendamment les uns des autres, halogène, -alkyle en C₁-C₄, -cycloalkyle en C₃-C₆, OH, -O-alkyle(C₁-C₆) ou -O-cycloalkyle(C₃-C₆), et dans les radicaux alkyle, alkylène ou cycloalkyle les atomes d'hydrogène pouvant être en partie ou en totalité remplacés par le fluor, R21 et R22 représentent indépendamment l'un de l'autre un atome d'hydrogène, un groupe -alkyle en C₁-C₆, -cycloalkyle en C₃-C₆, -alkylène(C₀-C₄)-aryle(C₆-C₁₄), -alkylène(C₀-C₄)-Het(C₄-C₁₅), -SO₂CH₃ ou -SO₂CF₃,
dans les radicaux alkyle, alkylène ou cycloalkyle les atomes d'hydrogène pouvant être en partie ou en totalité remplacés par le fluor, ou
R21 et R22 dans les fragments « N(R21)-R22 » et « N(R21)-C(O)-R22 » représentent un cycle à 5-8 chaînons, qui est formé conjointement avec l'atome d'azote « N » ou le groupe « N-C(O) », de sorte que sont formés des amines, imides ou lactames cycliques, qui contiennent jusqu'à 2 autres hétéroatomes choisis dans l'ensemble constitué par N, 0 ou S, le cycle étant non substitué ou portant indépendamment l'un de l'autre un ou deux substituants -alkyle en C₁-C₄, -cycloalkyle en C₃-C₆, OH, -O-alkyle(C₁-C₆) ou -O-cycloalkyle(C₃-C₆), dans les radicaux alkyle ou cycloalkyle les atomes d'hydrogène pouvant être en partie ou en totalité remplacés par le fluor, le radical -Het(C₄-C₁₅) représentant un système cyclique ayant de 4 à 15 atomes de carbone, qui est présent dans un, deux ou trois systèmes cycliques liés entre eux et qui selon la taille du cycle contient un, deux, trois ou quatre hétéroatomes identiques ou différents, choisis dans l'ensemble constitué par les atomes d'oxygène, d'azote et de soufre.

3. Composé de formule I selon la revendication 1 ou 2, dans lequel Ar représente un cycle benzénique ou pyridine soudé, le cycle soudé étant non substitué ou une, deux, trois ou quatre fois substitué chaque fois indépendamment par -alkyle en C₁-C₆, -cycloalkyle en - C₃-C₆, OH, -CN, -O-alkyle(C₁-C₈), -O-cycloalkyle(C₃-C₆), -alkylène(C₀-C₄)-C(O)-N(R11)-R12, -alkylène(C₀-C₄)-C(O)-O-R11, -alkylène(C₀-C₄)-C(O)-R11, SO₂CH₃, -alkylène(C₀-C₄)-N(R11)-R12, -alkylène(C₀-C₄)-N(R11)-C(O)-R12, halogène, -Si[alkyle(C₁-C₄)]₃, -alkylène(C₁-C₆)-O-alkyle(C₁-C₆) ou -O-alkylène(C₁-C₆)-O-alkyle(C₁-C₆), les groupes alkyle, alkylène et cycloalkyle étant chacun non substitué ou une, deux ou trois fois substitué, indépendamment les uns des autres, par -alkyle en C₂-C₄, -cycloalkyle en C₃-C₆, OH, -O-alkyle(C₁-C₆), -aryle en C₆-C₁₄, le radical aryle étant non substitué ou portant indépendamment les uns des autres un, deux, trois, quatre ou cinq substituants halogène, -alkyle en C₁-C₄, -cycloalkyle en C₃-C₆, OH, -O-alkyle(C₁-C₆) ou -O-cycloalkyle(C₃-C₆),
par -Het(C₄-C₁₅), Het étant non substitué ou portant indépendamment les uns des autres un, deux, trois, quatre ou cinq substituants halogène, -alkyle en C₁-C₄, -cycloalkyle en C₃-C₆, OH, -O-alkyle(C₁-C₆) ou -O-cycloalkyle(C₃-C₆), ou par -O-cycloalkyle(C₃-C₆), dans les radicaux alkyle, alkylène ou cycloalkyle les atomes d'hydrogène pouvant être en partie ou en totalité remplacés par le fluor, Q1, Q2 et Q3 sont identiques ou différents et représentent chacun indépendamment un atome d'hydrogène, un groupe -alkyle en C₁-C₆ ou -cycloalkyle en C₃-C₆, dans les radicaux alkyle ou cycloalkyle les atomes d'hydrogène pouvant être en partie ou en totalité remplacés par le fluor, R11 et R12 représentent indépendamment l'un de l'autre un atome d'hydrogène, un groupe -alkyle en C₁-C₆, -cycloalkyle en C₃-C₆ ou -SO₂CH₃,
dans les radicaux alkyle, alkylène ou cycloalkyle les atomes d'hydrogène pouvant être en partie ou en totalité remplacés par le fluor, ou
R11 et R12 dans les fragments « N(R11)-R12 » et « N(R11)-C(O)-R12 » représentent un cycle à 5-8 chaînons, choisi dans l'ensemble constitué par les cycles azétidinyle, pyrrolidinyle, pipéridinyle, pipérazinyle, azépinyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle, le cycle étant non substitué ou portant un ou indépendamment l'un de l'autre deux substituants -alkyle en C₁-C₄, -cycloalkyle en C₃-C₆, OH, -O-alkyle(C₁-C₆) ou -O-cycloalkyle(C₃-C₆), dans les radicaux alkyle ou cycloalkyle les atomes d'hydrogène pouvant être en partie ou en totalité remplacés par le fluor, R1, R2, R3, R4 et R5 sont identiques ou différents et représentent indépendamment les uns des autres un atome d'hydrogène, un groupe -alkyle en C₁-C₆, -cycloalkyle en C₃-C₆, OH, -CN, -NO₂, -O-alkyle(C₁-C₆), -O-cycloalkyle(C₃-C₆), -alkylène(C₀-C₄)-(CO)-N(R21)-R22, -SO₂CH₃, -alkylène(C₀-C₄)-(CO)-O-R21, un atome d'halogène, un groupe -SF₅, -alkylène(C₀-C₄)-C(O)-R21, -alkylène(C₀-C₄)-N(R21)-R22, -alkylène(C₀-C₄)-N(R21)-C(O)-R22, -alkylène(C₁-C₆)-O-alkyle(C₁-C₆), -O-alkylène(C₁-C₆)-O-alkyle(C₁-C₆), -Si [alkyle(C₁-C₄)]₃ ou -Het(C₄-C₁₅), les radicaux alkyle, alkylène et cycloalkyle étant chacun non substitué ou une, deux ou trois fois substitué, indépendamment les uns des autres, par -alkyle en C₁-C₄, OH, -cycloalkyle en C₃-C₆, -O-alkyle(C₁-C₆) ou -O-cycloalkyle(C₃-C₆),
dans les radicaux alkyle ou cycloalkyle les atomes d'hydrogène pouvant être en partie ou en totalité remplacés par le fluor, étant entendu qu'un symbole R1, R2, R3, R4 ou R5 représente -SF₅, R9 représente un atome d'hydrogène, un groupe -alkyle en C₁-C₆ ou -cycloalkyle en C₃-C₆, dans les radicaux alkyle ou cycloalkyle les atomes d'hydrogène pouvant être en partie ou en totalité remplacés par le fluor,
R21 et R22 représentent indépendamment l'un de l'autre un atome d'hydrogène, un groupe -alkyle en C₁-C₆, -cycloalkyle en C₃-C₆, -alkylène(C₀-C₄)-aryle(C₆-C₁₄), -alkylène(C₀-C₄)-Het(C₄-C₁₅), -SO₂CH₃ ou -SO₂CF₃,
dans les radicaux alkyle, alkylène ou cycloalkyle les atomes d'hydrogène pouvant être en partie ou en totalité remplacés par le fluor, ou R21 et R22 dans les fragments « N(R21)-R22 » et « N(R21)-C(O)-R22 » représentent un cycle à 5-8 chaînons, choisi dans l'ensemble constitué par les cycles azétidinyle, pyrrolidinyle, pipéridinyle, pipérazinyle, azépinyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle, le cycle étant non substitué ou portant un ou indépendamment l'un de l'autre deux substituants -alkyle en C₁-C₄, -cycloalkyle en C₃-C₆, OH, -O-alkyle(C₁-C₆) ou -O-cycloalkyle(C₃-C₆), dans les radicaux alkyle ou cycloalkyle les atomes d'hydrogène pouvant être en partie ou en totalité remplacés par le fluor, le radical -Het(C₄-C₁₅) représentant un système cyclique ayant de 4 à 15 atomes de carbone, qui est présent dans un, deux ou trois systèmes cycliques liés entre eux et qui selon la taille du cycle contient un, deux, trois ou quatre hétéroatomes identiques ou différents, choisis dans l'ensemble constitué par les atomes d'oxygène, d'azote et de soufre.

4. Composé de formule I selon les revendications 1 à 3, dans lequel Ar représente un cycle benzénique soudé, le cycle soudé étant non substitué ou une ou deux fois substitué chaque fois indépendamment par -O-alkyle(C₁-C₆), -C(O)-N(R11)-R12 ou halogène, Q1, Q2 et Q3 sont identiques et représentent chacun un atome d'hydrogène, R11 et R12 représentent indépendamment l'un de l'autre un atome d'hydrogène ou un groupe -alkyle en C₁-C₆,
R1, R2, R3, R4 et R5 sont identiques ou différents et représentent indépendamment les uns des autres un atome d'hydrogène, un groupe -alkyle en C₁-C₆, -O-alkyle(C₁-C₆), un atome d'halogène, un groupe -SF₅, -alkylène(C₀-C₄)-N(R21)-R22, -O-alkylène(C₁-C₆)-O-alkyle(C₁-C₆) ou -alkylène(C₀-C₄)-N(R21)-C(O)-R22, étant entendu qu'un symbole R1, R2, R3, R4 ou R5 représente -SF₅ R9 représente un atome d'hydrogène,
R21 et R22 représentent indépendamment l'un de l'autre un atome d'hydrogène, un groupe -alkyle en C₁-C₆, -cycloalkyle en C₃-C₆, CF₃ ou -SO₂CH₃,
dans les radicaux alkyle, alkylène ou cycloalkyle les atomes d'hydrogène pouvant être en partie ou en totalité remplacés par le fluor, ou R21 et R22 dans les fragments « N(R21)-R22 » représentent un cycle, choisi dans l'ensemble constitué par les cycles azétidinyle, pyrrolidinyle, pipéridinyle, pipérazinyle, azépinyle, morpholinyle, thiomorpholinyle, pyrrolidine-2,5-dionyle, pipéridine-2,6-dionyle, pipérazine-2,6-dionyle, morpholine-3,5-dionyle, pyrrolidin-2-onyle, pipéridin-2-onyle, pipérazin-2-onyle et morpholin-3-onyle.

5. Composé de formule I selon les revendications 1 à 4, le composé de formule I étant choisi dans le groupe constitué par la 2-(1-imino-1,3-dihydro-iso-indol-2-yl)-1-(3-pentafluorosulfanyl-phényl)-éthanone sous forme de bromhydrate, le méthylamide d'acide 2-[2-(3-diméthylamino-5-pentafluorosulfanyl-phényl)-2-oxo-éthyl]-6-éthoxy-3-imino-2,3-dihydro-1H-iso-indole-5-carboxylique, le méthylamide d'acide 6-éthoxy-3-imino-2-[2-(3-méthylamino-5-pentafluorosulfanyl-phényl)-2-oxo-éthyl]-2,3-dihydro-1H-iso-indole-5-carboxylique, le méthylamide d'acide 6-éthoxy-3-imino-2-{2-[3-pentafluorosulfanyl-5-(2,2,2-trifluoro-acétylamino)-phényl]-2-oxo-éthyl}-2,3-dihydro-1H-iso-indole-5-carboxylique, le méthylamide d'acide 6-éthoxy-2-(2-{3-[éthyl-(2,2,2-trifluoro-acétyl)-amino]-5-pentafluorosulfanyl-phényl}-2-oxo-éthyl)-3-imino-2,3-dihydro-1H-iso-indole-5-carboxylique, le méthylamide d'acide 6-éthoxy-2-[2-(3-éthylamino-5-pentafluorosulfanylphényl)-2-oxo-éthyl]-3-imino-2,3-dihydro-1H-iso-indole-5-carboxylique sous forme de chlorhydrate, le méthylamide d'acide 2-[2-(3-amino-5-pentafluorosulfanyl-phényl)-2-oxo-éthyl]-6-éthoxy-3-imino-2,3-dihydro-1H-iso-indole-5-carboxylique, le méthylamide d'acide 2-[2-(3-acétylamino-5-pentafluorosulfanylphényl)-2-oxo-éthyl]-6-éthoxy-3-imino-2,3-dihydro-1H-iso-indole-5-carboxylique, la 2-(5,6-diéthoxy-7-fluoro-1-imino-1,3-dihydro-iso-indol-2-yl)-1-[3-méthylamino-5-(pentaméthyl-sulfanyl)-phényl]éthanone, le méthylamide d'acide 6-éthoxy-3-imino-2-{2-[3-diméthanesulfonylamino-5-(pentafluorosulfanyl)-phényl]-2-oxo-éthyl}-2,3-dihydro-1H-iso-indole-5-carboxylique, le méthylamide d'acide 2-{2-[3-(acétyl-méthylamino)-5-(pentafluorosulfanyl)-phényl]-2-oxo-éthyl}-6-éthoxy-3-imino-2,3-dihydro-1H-iso-indole-5-carboxylique, le N-[3-[2-(5,6-diéthoxy-7-fluoro-1-imino-1,3-dihydro-iso-indol-2-yl)-acétyl)-5-(pentafluorosulfanyl)-phényl)-N-méthyl-acétamide, le N-[3-[2-(5,6-diéthoxy-7-fluoro-1-imino-1,3-dihydro-iso-indol-2-yl)-acétyl]-5-(pentafluorosulfanyl)-phényl]-isobutyramide, le N-[3-[2-(5,6-diéthoxy-7-fluoro-1-imino-1,3-dihydro-iso-indol-2-yl)-acétyl]-5-pentafluorosulfanyl)-phényl]-acétamide sous forme de bromhydrate, le N-[5-[2-(5,6-diéthoxy-7-fluoro-1-imino-1,3-dihydro-iso-indol-2-yl)-acétyl]-4-méthyl-2-(pentafluorosulfanyl)-phényl]-acétamide, la 1-[5-amino-2-méthyl-4-(pentafluorosulfanyl)-phényl]-2-(5,6-diéthoxy-7-fluoro-1-imino-1,3-dihydro-iso-indol-2-yl)-éthanone, le N-{3-[2-(5,6-diéthoxy-7-fluoro-1-imino-1,3-dihydro-iso-indol-2-yl)-acétyl]-5-pentafluorosulfanyl-phényl}-3,3-diméthylbutyramide, la 1-{3-[2-(5,6-diéthoxy-7-fluoro-1-imino-1,3-dihydro-iso-indol-2-yl)-acétyl]-5-pentafluorosulfanyl-phényl}-pyrrolidine-2,5-dione, la 1-[2-amino-4-(pentafluorosulfanyl)-phényl]-2-(5,6-diéthoxy-7-fluoro-1-imino-1,3-dihydro-iso-indol-2-yl)-éthanone, la 1-[2-amino-5-bromo-4-(pentafluorosulfanyl)-phényl]-2-(5,6-diéthoxy-7-fluoro-1-imino-1,3-dihydro-iso-indol-2-yl)-éthanone, la 2-(5,6-diéthoxy-7-fluoro-1-imino-1,3-dihydro-iso-indol-2-yl)-1-[3-méthoxy-5-(pentafluorosulfanyl)-phényl]-éthanone, le [3-[2-(5,6-diéthoxy-7-fluoro-1-imino-1,3-dihydro-iso-indol-2-yl)-acétyl]-5-(pentafluorosulfanyl)-phényl]-amide d'acide cyclobutanecarboxylique sous forme de chlorhydrate, le [3-[2-(5,6-diéthoxy-7-fluoro-1-imino-1,3-dihydro-iso-indol-2-yl)-acétyl]-5-(pentafluorosulfanyl)-phényl]-amide d'acide cyclopropanecarboxylique sous forme de chlorhydrate, la 2-(5,6-diéthoxy-7-fluoro-1-imino-1,3-dihydro-iso-indol-2-yl)-1-[3-éthoxy-5-(pentafluoro-sulfanyl)-phényl]-éthanone, le méthylamide d'acide 6-éthoxy-3-imino-2-{2-[3-méthoxy-5-(pentafluoro-sulfanyl)-phényl]-2-oxo-éthyl}-2,3-dihydro-1H-iso-indole-5-carboxylique, la 1-[3-bromo-5-(pentafluorosulfanyl)-phényl]-2-(5,6-diéthoxy-7-fluoro-1-imino-1,3-dihydro-iso-indol-2-yl)-éthanone, la 1-[3-chloro-5-(pentafluorosulfanyl)-phényl]-2-(5,6-diéthoxy-7-fluoro-1-imino-1,3-dihydro-iso-indol-2-yl)-éthanone, la 1-[2-bromo-4-(pentafluorosulfanyl)-phényl]-2-(5,6-diéthoxy-7-fluoro-1-imino-1,3-dihydro-iso-indol-2-yl)-éthanone, la 1-[2-chloro-4-(pentafluorosulfanyl)-phényl]-2-(5,6-diéthoxy-7-fluoro-1-imino-1,3-dihydro-iso-indol-2-yl)-éthanone, la 2-(5,6-diéthoxy-7-fluoro-1-imino-1,3-dihydro-iso-indol-2-yl)-1-[3-morpholin-4-yl-5-(pentafluorosulfanyl)-phényl]-éthanone ou la 2-(5,6-diéthoxy-7-fluoro-1-imino-1,3-dihydro-iso-indol-2-yl)-1-[3-(2-méthoxyéthoxy)-5-(pentafluorosulfanyl)-phényl]-éthanone sous forme de chlorhydrate.

6. Médicament, **caractérisé par** une teneur efficace en au moins un composé de formule I selon une ou plusieurs des revendications 1 à 5, conjointement avec un véhicule, additif et/ou autres principes actifs et adjuvants, pharmaceutiquement appropriés et physiologiquement acceptables.

7. Utilisation du composé de formule I selon une ou plusieurs des revendications 1 à 5, pour la fabrication d'un médicament destiné à la prophylaxie, la prévention secondaire et la thérapie de toutes les maladies qui s'accompagnent de thromboses, d'embolies, d'hypercoagulabilité, d'altérations fibreuses, ou d'affections inflammatoires.

8. Utilisation selon la revendication 7, **caractérisée en ce qu'**il s'agit d'infarctus du myocarde, d'angine de poitrine et d'autres formes du syndrome coronarien aigu, de l'accident vasculaire cérébral, des maladies vasculaires périphériques, de la thrombose des veines profondes, de l'embolie pulmonaire, d'incidents emboliques ou thrombotiques dus à des arythmies cardiales, d'incidents cardiovasculaires tels que la resténose après revascularisation et angioplastie et interventions similaires telles qu'implantation de stents et opérations de pontage, ou pour la réduction du risque de thrombose après des interventions chirurgicales comme dans des opérations des articulations du genou et de la hanche, ou des interventions qui conduisent à un contact du sang avec des surfaces étrangères comme chez des patients en dialyse et des patients à cathéter permanent ou **en ce qu'**il s'agit de la coagulation intravasculaire disséminée, du sepsis, et d'autres incidents intravasculaires qui s'accompagnent d'une inflammation, de l'athérosclérose, du diabète et du syndrome métabolique et de leurs suites, de la croissance tumorales et de la dissémination de métastases tumorales, d'arthropathies inflammatoires et dégénératives telles que la polyarthrite rhumatoïde et l'arthrose, de troubles du système hémostatique tels que des dépôts de fibrine, des altérations fibreuses du poumon telles que la pneumopathie obstructive chronique, le syndrome de détresse respiratoire de l'adulte ou des dépôts de fibrine dans l'oeil après des opérations des yeux ou pour éviter et/ou traiter la cicatrisation.

9. Procédé pour la préparation du composé de formule I selon une ou plusieurs des revendications 1 à 5, **caractérisé en ce que**
a) on fait réagir un composé de formule II, 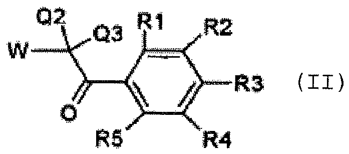 dans laquelle R1, R2, R3, R4, R5, Q2 et Q3 sont tels que définis dans la formule I et W représente l'ion chlorure, bromure, mésylate ou tosylate, avec un composé de formule III, 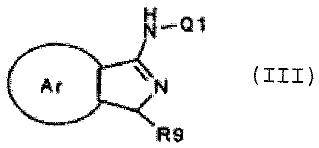 dans laquelle Ar, R9 et Q1 sont tels que définis dans la formule I, avec ou sans addition d'une base, dans un solvant, pour obtenir un composé de formule I, ou
b) on fait réagir un composé de formule VII, 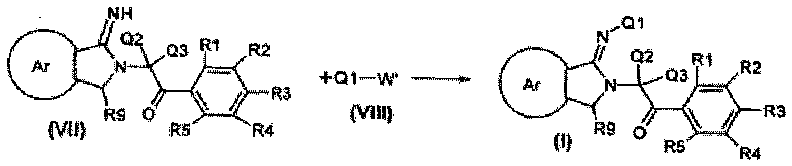 où R1, R2, R3, R4, R5, R9, Ar, Q1, Q2 et Q3 sont tels que définis dans la formule I, avec le composé Q1-W', W' représentant l'ion chlorure, bromure, mésylate, tosylate, méthylsulfate ou un bon groupe partant similaire, avec ou sans addition d'une base, pour obtenir un composé de formule I, ou
c) soit on isole sous forme libre le composé de formule I préparé selon le procédé a) ou b), soit on le libère à partir de sels physiologiquement acceptables ou, dans le cas de la présence de groupes acides ou basiques on le convertit en sels physiologiquement acceptables, ou
d) on résout en les énantiomères ou diastéréoisomères purs un composé de formule I préparé selon le procédé a) ou b), ou un précurseur approprié de formule I, qui en raison de sa structure chimique apparaît sous des formes énantiomères ou diastéréoisomères, par salification avec des acides ou des bases sous forme d'énantiomères purs, chromatographie sur des phases stationnaires chirales ou transformation en dérivé au moyen de composés chiraux sous forme d'énantiomères purs, tels que des acides aminés, séparation des diastéréoisomères ainsi obtenus, et élimination des groupes auxiliaires chiraux.

10. Procédé pour la préparation du composé de formule II selon la revendication 9, dans lequel W représente Br, **caractérisée en ce que**
a) on convertit un composé de formule X, 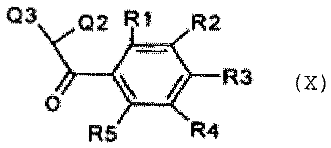 dans laquelle R1, R2, R3, R4, R5, Q2 et Q3 sont tels que définis dans la formule I, à l'aide d'un réactif de bromation, en le composé de formule II, ou
b) on traite par un réactif de bromation un composé de formule XI ou XI', 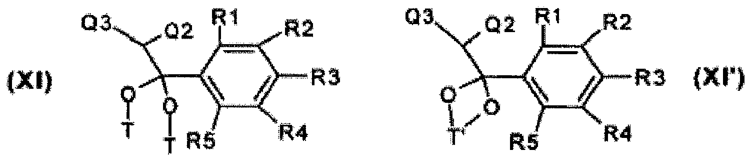 dans laquelle R1, R2, R3, R4, R5, Q2 et Q3 sont tels que définis dans la formule I, T représente un groupe -alkyle en C₁-C₄ et T' représente le groupe éthylène, propylène ou butylène ou forme conjointement avec le groupe -O-C-O- un cycle 1,3-dioxo ayant une taille de cycle de 5, 6 ou 7, et ensuite en présence d'un acide on le convertit en le composé de formule II.

11. Procédé pour la préparation du composé de formule X selon la revendication 10, dans lequel R4 représente le groupe diméthylamino et R1, R2, R3, R5, Q2 et Q3 sont tels que définis dans la formule I, **caractérisé en ce que**
a) d'abord on soumet à une nitration un composé de formule XIVa 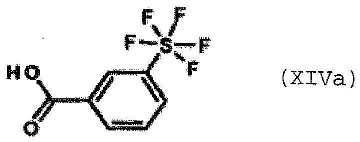 et ensuite on le réduit avec de l'hydrogène en l'amine de formule XIIIa 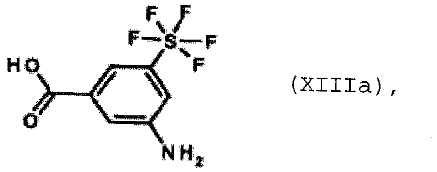
b) on soumet à une diméthylation sur l'azote le composé de formule XIIIa obtenu et on convertit à l'aide de chlorure de thionyle l'acide carboxylique en le chlorure d'acide et ensuite on le fait réagir avec de l'O,N-diméthyl-hydroxylamine pour obtenir un composé de formule XIIa, 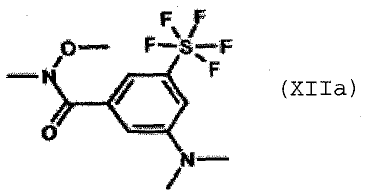
c) on convertit le composé de formule XIIa obtenu, à l'aide de bromure de méthylmagnésium, en composé de formule Xa,

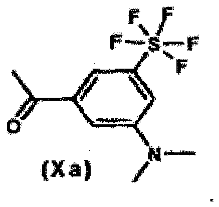

12. Procédé pour la préparation du composé de formule X selon la revendication 10, dans lequel R4 représente le groupe méthoxy et R1, R2, R3, R5, Q2 et Q3 sont tels que définis dans la formule I, **caractérisé en ce que**
a) d'abord on soumet à une diazotation le composé de formule XIIIa selon la revendication 11 et ensuite on le convertit par ébullition en le phénol XIIIb 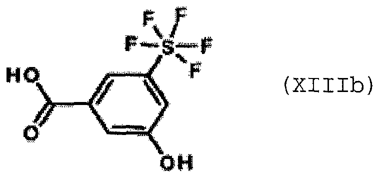
b) on soumet à une méthylation le composé de formule XIIIb obtenu, on le convertit en l'amide de Weinreb et ensuite on convertit ce dernier, à l'aide de bromure de méthylmagnésium, en le composé Xb.
